# EUROPEAN PATENT SPECIFICATION

(11) **EP 0 837 870 B1**
(45) Date of publication and mention of the grant of the patent: **24.07.2002**
(21) Application number: 96919423.2
(22) Date of filing: 13.06.1996
(51) Int. Cl.: C07H 17/08, A01N 43/22, C07D 407/12, C07D 313/00

(54) **SYNTHETIC MODIFICATION TO SPINOSYN COMPOUNDS**
SYNTHETISCHE MODIFIZIERUNG VON SPINOSYN-VERBINDUNGEN
MODIFICATION SYNTHETIQUE DE COMPOSES DE SPINOSYNE

(30) Priority: 14.06.1995 US 201 P; 14.07.1995 US 1435 P; 21.12.1995 US 9006 P
(43) Date of publication of application: 29.04.1998
(73) Proprietor: Dow AgroSciences LLC, Indianapolis, Indiana 46268 (US)
(72) Inventor: DEAMICIS, Carl, Vincent, Indianapolis, IN 46236 (US); ANZEVENO, Peter, Biagio, Zionsville, IN 46077 (US); MARTYNOW, Jacek, G., Fishers, IN 46038 (US); MCLAREN, Kevin, L., Fishers, IN 46038 (US); GREEN, Frederick, Richard, III, Noblesville, IN 46060-1256 (US); SPARKS, Thomas, C., Greenfield, IN 46140 (US); KIRST, Herbert, A., Indianapolis, IN 46278 (US); CREEMER, Lawrence, Camillo, Greenfield, IN 46140 (US); WORDEN, Thomas, V., Pittsboro, IN 46167 (US); SCHOONOVER, Joe, Raymond, Jr., Brownsburg, IN 46112 (US); GIFFORD, James, Michael, Lebanon, IN 46052 (US); HATTON, Christopher, J., Westfield, IN 46074 (US); HEGDE, Vidyadhar, B., Indiana 46032 (US); CROUSE, Gary, D., Carmel, IN 46033 (US); THOREEN, Brian, R., Indianapolis, IN 46268 (US); RICKS, Michael, J., Indianapolis, IN 46278 (US)
(74) Representative: Raynor, John
(86) International application number: US9610327
(87) International publication number: WO9700265

(56) References cited:
- EP-A- 0 375 316
- WO-A-93/09126
- WO-A-94/20518
- US-A- 5 202 242
- JOURNAL OF ORGANIC CHEMISTRY, vol. 59, no. 6, 1994, EASTON US, pages 1548-1560, XP002018758 MARTYNOW J G ET AL: "Chemistry of A83543A Derivatives. 1. Oxidations and Reductions of A83543A Aglycon"

## Description

### Field of the Invention

This invention relates to compounds produced by chemical modifications of Spinosyn compounds produced by *Saccharopolyspora spinosa.* The compounds have insecticidal activity.

### Background of the Invention

The fermentation product identified in US Patent No. 5,362,634 as A83543 is a family of related compounds produced by *Saccharopolyspora spinosa.* These compounds have been referred to as factors or components A, B, C, D, E, F, G, H J, K, L, M, N, O, P, Q, R, S, T, U, V, W, Y and the like (also see PCT WO 93/09126 and PCT WO 94/20518) and are hereinafter referred to as Spinosyn A, B, and the like. The Spinosyn compounds are useful for the control of arachnids, nematodes and insects, in particular *Lepidoptera* and *Dipcera* species. The naturally produced Spinosyn compounds consist of a 5,6,5-tricylic ring system, fused to a 12-membered macrocyclic lactone, a neutral sugar (rhamnose) and an amino sugar (forosamine) (see Kirst et al. (1991), Tetrahedron Letters, 32:4839). If the amino sugar is not present the compounds have been referred to as the pseudoaglycone of A, D, etc. and if the neutral sugar is not present then the compounds have been referred to as the reverse pseudoaglycone of A, D, etc. A more preferred nomenclature is to refer to the pseudoaglycones as Spinosyn A 17-Psa, Spinosyn D 17-Psa, and the like. A more preferred nomenclature is to refer to the reverse pseudoaglycones as Spinosyn A 9-Psa, Spinosyn D 9-Psa, and the like. The Spinosyn compounds typically have had the following structures:

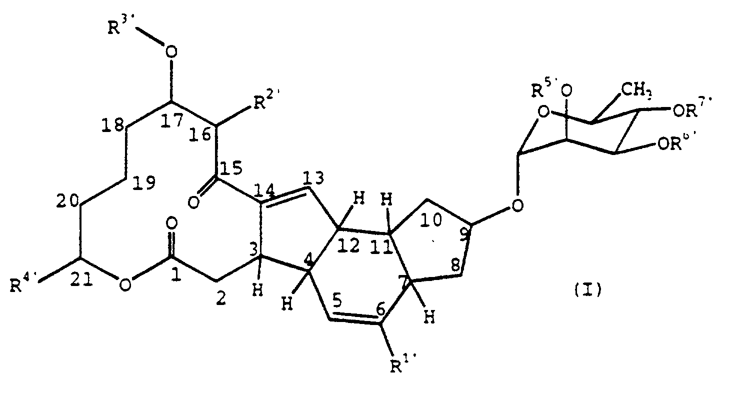

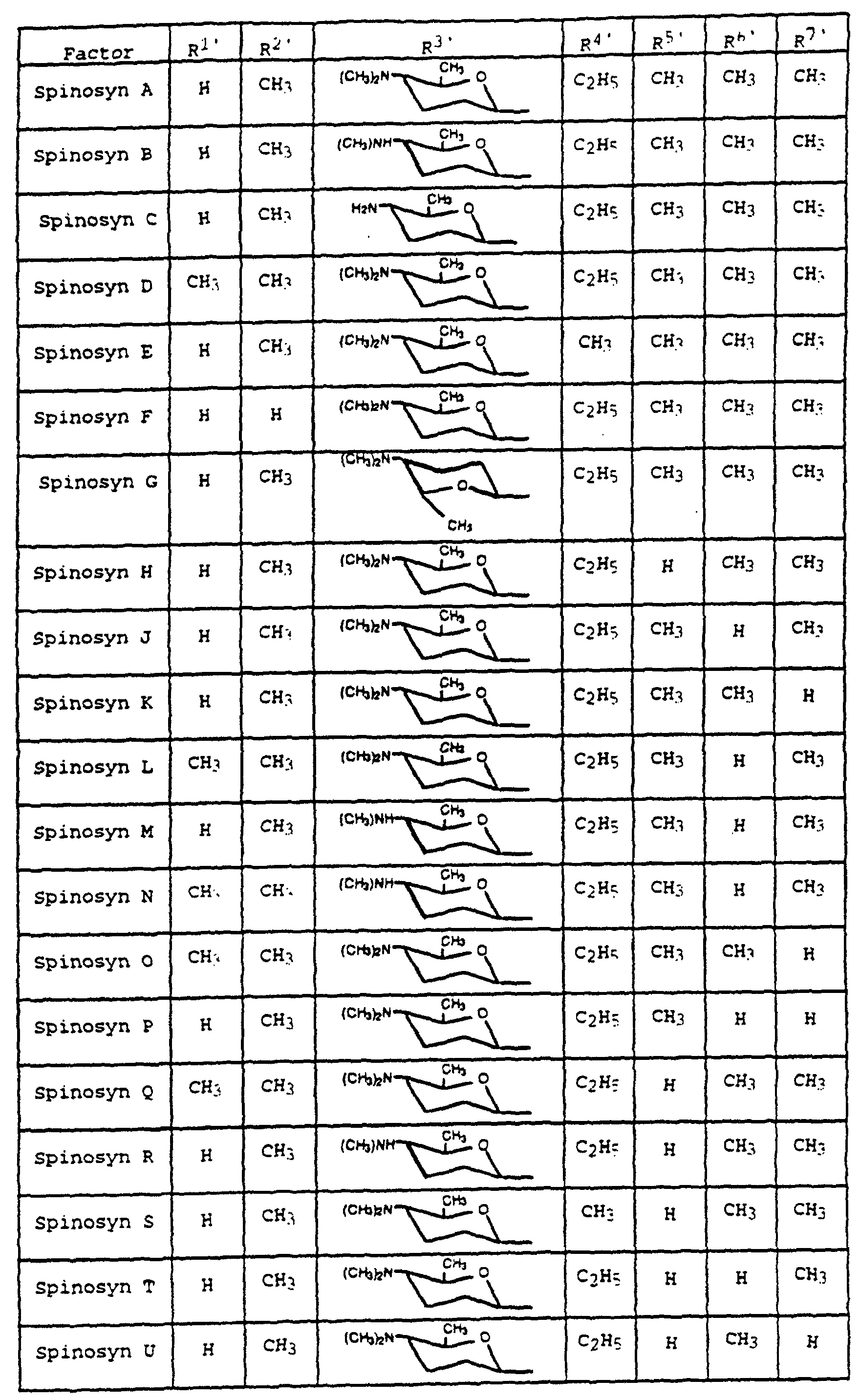

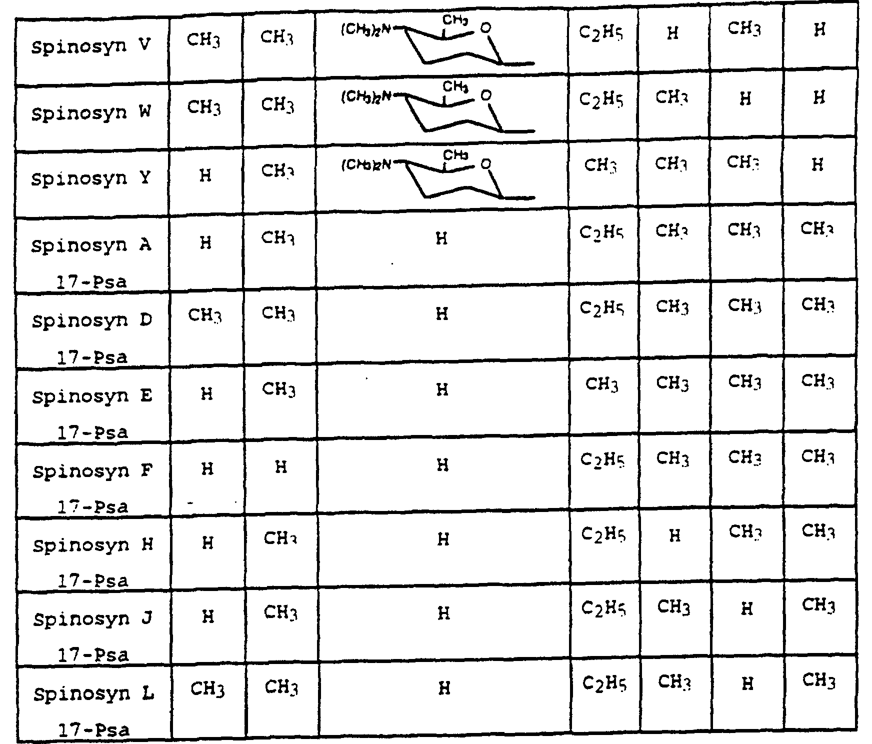

and

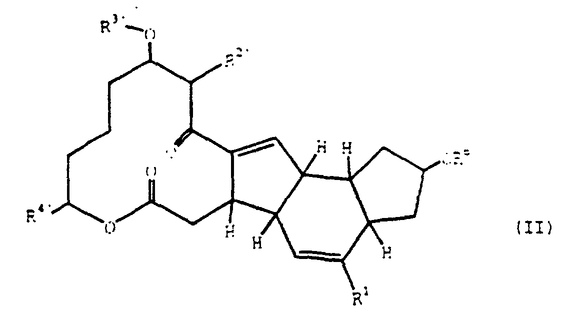

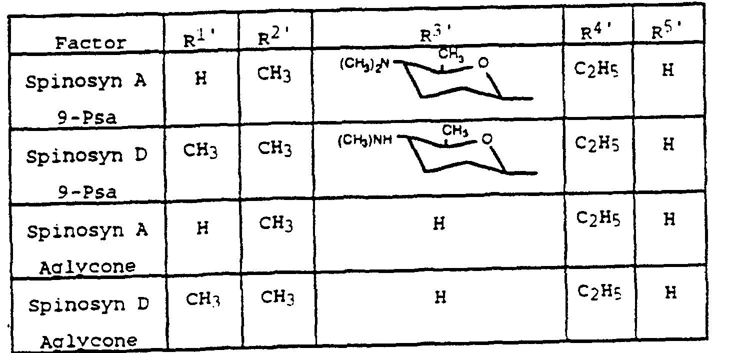

The naturally produced Spinosyn compounds may be produced via fermentation from cultures NRRL 18719, 18537, 18538, 18539, 18719, 1843 and 18823. These cultures have been deposited and made part of the stock culture collection of the Midwest Area Northern Regional Research Center, Agricultural Research Service, United States Department of Agriculture, 1815 North University Street, Peoria, IL 61604.

As previously stated, the Spinosyn compounds are particularly effective against *Lepidoptera* and *Diptera* species. The Spinosyn compounds are quite environmentally friendly and have an appealing toxicological profile. However, in some applications it would be most desirable to have a longer residual effect than provided by spinosyns disclosed in previous patents and publications. A Spinosyn analog that has a longer residual effect could be used to control mites on fruits and nuts or to control codling moth, which effects pome fruit.

### Summary of the Invention

The invention disclosed herein is to the synthetic modification of products produced by *Saccharopolyspora spinosa* to thereby produce intermediates and/or insecticidal products for use in the agricultural and animal health markets. The numerous synthetic modifications were made to the rhamnose sugar, forosamine sugar, to the molecule via hydrogenation, epoxidation, reduction, halogenation, oxidation, adding alkyl groups, adding nitrogen groups, and the addition and elimination of substituents on the macrocylclic lactone.

Note that all compounds of the present invention have a single bond or an epoxide linkage between positions 5 and 6 in formula (I) above.

### Detailed Description of the Invention

The compounds of the present invention are prepared directly or indirectly by modifying the compounds that are naturally produced from *Saccharopolyspora spinosa,* see U.S. Patent 5,362,634 to DowElanco. The compounds of the invention have been shown to have activity against insects, arachnids and nematodes. Therefore, the compounds may be used to make other compounds or the compounds themselves may be used for inhibiting or inactivating an insect or mite. An inhibitory or inactivating amount of the compound comes into contact with the locus of an insect or mite. The "locus" of the insect refers to the environment in which the insect or mite lives or where its eggs are present, including the air surrounding it, the food it eats, or objects which it contacts. Typically these compounds are applied to the foliage of a plant which an insect or mite might feed on. Unless otherwise specified herein, the phrases and terms have the following meaning: By the term "haloalkyl" it is meant an alkyl having 1 to 4 carbon atoms, where there is at least one halogen bound to a carbon atom. By the term "halogen" it is meant Cl, F, Br, or I. By the term "alkanoyl" it is meant an alkyl having 1 to 4 carbon atoms, where at least one carbonyl group is attached to an alkyl group. By the term "alkylhydroxyl amino" it is meant a substitutent having the formula 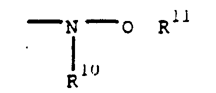 wherein R¹⁰ and R¹¹ are independently an alkyl having 1 to 4 carbon atoms or an alkanoyl having 1 to 5 carbon atoms. By the term "protected hydroxyl" it is meant substituted methyl ethers, such as but not limited to methoxy methyl, tetrahydropyran, substituted ethyl ethers such as but not limited to 1-ethoxy ethyl, substituted benzylethers such as but not limited to p-methoxybenzyl, silylethers such as but not limited to silylethers having 1 to 2 carbon atoms, typically trimethylsilyl, esters, typically esters having 1 to 2 carbon atoms, more specifically formate or acetate, carbonates typically carbonates having 1 to 2 carbon atoms, more specifically methyl carbonate, and sulfonates, typically having 1 to 2 sulfurs, more specifically methyl sulfonates. These protecting groups are described in more detail in Greene, T. W., Wuts, P. G. M. Protecting Groups in Organic Syntheses, John Wiley and Sons, New York, 1991, p. 17-18. By the term "protected amino" it is meant carbamates having 1 to 2 carbon atoms, amides having 1 to 4 carbon atoms, typically n-formyl and n-acetyl, and imines such as n-benzyliene. These protecting groups are further described in Greene Protecting Groups in Organic Syntheses. By the term "inhibiting an insect or mite" it is meant that there is a decrease in the number of living insects or mites or a decrease in the number of eggs. By "inactivating amount" it is meant that an amount of compound is used to cause measurable reduction in the treated insect or mite population. Typically from about 1 to about 1,000 ppm (or 0.01 to 1 Kg/acre) of compound is used.

These compounds are typically active against *Macrosteles fascifrons* (aster leafhopper), *Spodoptera exiqua* (beet armyworm), *Meloidogyne arenaria* (peanut root knot nematode), *Aphis gossypii* (cotton aphids), *Tetranychus urticae* (two spotted spider mite), *Diabrotica undecimpunctata howardi* (Southern corn rootworm), *Heliothis zea* (cotton bollworm), *Peregrinus maidis* (corn planthopper), *Heliothis virescens* (Tobbacco budworm), *Blattella germanicus* (German cockroach), *Ostrinia nubilalis* (European cornborer), *Nephatettix cinciticeps* (green rice leafhopper), *Nilaparvata lugens* (brown planthopper), and *Chilo suppressalis* (rice stem borer)and the like.

The synthetic compounds are typically prepared by modifying the rhamnose sugar, modifying the forosamine sugar, or starting with 9-or 17- pseuodoaglycone and then substituting a nonsugar or different sugar with rhamnose or forosamine sugar. Synthetic compounds were also prepared by modifying the 5,6,5-tricyclic and/or 12-membered macrocyclic lactone part of the compounds naturally produced or of the pseudoaglycone of the natural compounds as described in the Examples herein. The compounds claimed herein include all isomers and any acid addition salts of the compounds and their isomers.

The compounds claimed herein exist in several diastereomeric isomers. Because there are multiple stereogenic centers, it is anticipated that the diastereomeric isomers will have utility as insecticides. Although some diastereomeric isomers may be more effecacious than others, all the diastereomeric isomers are equivalent to the claimed invention.

In Example 17 Part A, the rhamnose sugar at R5', R6' and R7' of Formula I is modified by adding long chain alkyl groups, aromatic groups and/or the addition of long chain alkyl groups, aromatic groups with halogen substitutents. Esters were also made at the R5', R6' and R7' position. Additional sugars were also placed at these positions. Another type of modification was to add hetero substituents containing atoms such as nitrogen, sulfur, phosphorus and silicon. In addition to the modifications on the rhamnose sugar, the double bond between the C5 and C6 position in Formula I was modified by epoxidation or hydrogenation.

In Example 17, Part C, the forosamine sugar was modified by changing the substitution on the nitrogen from H or methyl to a long chain alkyl group, acyl group, quaternary ammonium salt or N-oxide. In Example 17, Part D, the molecule was modified at the double bond at the C13 and C14 positions by hydrogenation, epoxidation, reduction and adding alkyl groups and nitrogen groups such as hydroxyl amines and cyanide. In Example 17, Part E, the C17 substituent of the macrocylic lactone is eliminated to give a double bond and/or the C5 and C6 positions of the molecule is modified with halogens, epoxides and alkoxides. In Example 17, Part F, at the C5 and C6 position, the molecule is modified by hydrogenation, epoxidation, halogenation, oxidation and the adding of hetero atoms substituents and esters.

In Example 17, Part I deoxygenated rhamnose analogs are prepared and further modified with ester and heteroatom substitutions.

Not only are the compounds disclosed herein useful in producing agricultural product, acid addition salts of these compounds are also desirous products. Acid addition salts may be prepared from the compounds disclosed in Formulas I, II, VI, VIII. The salts of the compounds are prepared using standard technology for preparing salts-which are well known to those skilled in the art. Salts can be neutralized to form an acid addition salt. Acid addition salts that are particularly useful include, but are not limited to, salts formed by standard reactions with both organic and inorganic acids such as sulfuric, hydrochloric, phosphoric, acetic, succinic, citric, lactic, maleic, fumaric, cholic, pamoic, mucic, glutamic, camphoric, glutaric, glycolic, phthalic, tartaric, formic, lauric, stearic, salicylic, methanesulfonic, benzenesulfonic, sorbic, picric, benzoic, cinnamic and like acids.

Compositions are prepared according to the procedures and formulas which are conventional in the agricultural or pest control art. The compositions may be concentrated and dispersed in water or may be used in the form of a dust, bait or granular formulation. The dispersions are typically aqueous suspensions or emulsions prepared from concentrated formulations of the compounds. The water-soluble or water-suspension or emulsifiable formulations are either solids, wettable powders, or liquids, known as emulsifiable concentrates or aqueous suspensions. Wettable powders may be agglomerated or compacted to form water dispersible granules. These granules comprise mixtures of compound, inert carriers and surfactants. The concentration of the compound is typically between about 0.1% to about 90% by weight. The inert carrier is typically attapulgite clays, montmorillonite clays and the diatomaceous earths or purified silicates.

Surfactants comprise typically about 0.5% to about 10% of the wettable powder, where the surfactants are typically sulfonated lignins, condensed napthalene-sulfonates, the napthalene-sulfonates, alkyl-benenesulfonates, alkysulfonates or nonionic surfactants such as ethylene oxide adducts of alkylphenols or mixtures thereof. Emulsifiable concentrates of the claimed compounds typically range from about 50 to about 500 grams of compound per liter of liquid, equivalent to about 10% to about 50%, dissolved in an inert carrier which is a mixture of a water immiscible solvent and emulsifiers. organic solvents include organics such as xylenes, and petroleum fractions such as high-boiling naphthlenic and olefinic portions of petroleum which include heavy and aromatic naphtha. Other organics may also be used such as terpenic solvents -rosin derivatives, aliphatic ketones such as cyclohexanone and complex alcohols. Emulsifiers for emulsifiable concentrates are typically mixed ionic and/or nonionic surfactants such as those mentioned herein or their equivalents.

Aqueous suspensions may be prepared containing water-insoluble compounds, where the compounds are dispersed in an aqueous vehicle at a concentration typically in the range of between about 5% to about 50% by weight. The suspensions are prepared by finely grinding the compound and vigorously mixing it into a vehicle of water, surfaccants, and dispersants as discussed herein. Inert ingredients such as inorganic salts and synthetic or natural gums may also be employed to increase the density and/or viscosity of the aqueous vehicle as is desired.

Precipitated flowables may be prepared by dissolving the active molecule in a water-miscible solvent and surfactants or surface active polymers. When these formulations are mixed with water, the active compound precipitates with the surfactant controlling the size of the resulting micro-crystaline precipitate. The size of the crystal can be controlled through the selection of specific polymer and surfactant mixtures.

The compounds may also be applied as a granular composition that is applied to the soil. The granular composition typically contains from about 0.5% to about 10% by weight of the compound. The compound is dispersed in an inert carrier which is typically clay or an equivalent substance. Generally, granular compositions are prepared by dissolving the compound in a suitable solvent and applying it to a granular carrier which has been preformed to the desirable particle size. The particle size is typically between about 0.5 mm to 3 mm. The granular compositions may also be prepared by forming a dough or paste of the carrier and compound, drying the combined mixture, and crushing the dough or paste to the desired particle size.

The compounds may also be combined with an appropriate organic solvent. The organic solvent is typically a bland petroleum oil that is widely used in the agricultural industry. These combinations are typically used as a spray. More typically, the compounds are applied as a dispersion in a liquid carrier, where the liquid carrier is water. The compounds may also be applied in the form of an aerosol composition. The compound is dissolved in an inert carrier, which is a pressure-generating propellant mixture. The aerosol composition is packaged in a container, where the mixture is dispersed through an atomizing valve. Propellant mixtures contain either low-boiling halocarbons, which may be mixed with organic solvents or aqueous suspensions pressurized with inert gases or gaseous hydrocarbons.

The amount of compound applied to the loci of insects and mites is not critical and can easily be determined by those skilled in the art. Generally the concentrations of from about 10 ppm to about 5,000 ppm provide the desired control. For crops such as soybeans and cotton, the rate of application is about 0.01 to about 1 kg/ha, where the compound is applied in a 5 to 50 gal/A spray formulation. The compounds may be applied to any locus inhabited by an insect or mite. Such locus typically is cotton, soybean and vegetable crops, fruit and nut trees, grape vines, houses and ornamental plants. The compounds of the present invention are also useful for the treatment of animals to control arthropods, *i.e.*, insects and arachnids, which are pests on animals. These arthropod pests typically attack their hosts on the external ("ecto") surface; agents which control such pests are referred to as "ectoparasiticides".

All animals are subject to attack by such pests, though the problems are most severe among vertebrate hosts. Human beings are potential hosts for many parasites, and in tropical areas and in areas with minimal sanitation, parasitic infections are a regular problem in medical practice. Also highly subject to attack by parasites are the numerous livestock animals, such as cattle, sheep, pigs, goats, buffalo, water buffalo, deer, rabbits, chickens, turkeys, ducks, geese, ostriches, and the like. Horses and other pleasure animals are subject to parasitic attack, as are mink and other animals grown for their fur, and rats, mice and other animals used in laboratory and research settings. Companion animals such as dogs and cats are highly subject to attack by parasites, and because of their close relationship with humans, such parasitism poses problems for the humans with whom they are associated. Fish, crustacea, and other aquatic species are also subject to parasitic attack. In short, parasitism involves as hosts essentially the whole range of animals.

The economic toll from ectoparasitic infestations is large. In the livestock realm, animals suffer reduced feed efficiency and growth rates. Milk and wool production suffer, and there is damage to fleece, hides, and pelts. Animals are rendered susceptible to secondary microbiological infections and to further parasite attack. Ectoparasites also cause considerable discomfort even when they are not severely detrimental to health and production.

Although a number of parasiticides are in use, they suffer from a variety of problems, including a limited spectrum of activity, environmental toxicity, the need for repeated treatment, and, in many instances, resistance by ectoparasites. Therefore, there is a continuing need for new ectoparasiticides.

The present compounds provide a new tool in the armamentarium for controlling ectoparasites. In this embodiment, the present invention is directed to a method for inhibiting or killing an arthropod pest on a host animal, which comprises contacting the pest with an effective amount of a compound of the present invention.

The present compounds can be used to control a wide variety of arthropod pests. Representative pests which can be controlled by the present compounds are the following:
Arachnids, Amblyomma americanum (Lone-star tick), Amblyomma maculatum (Gulf Coast tick), Argas persicus (fowl tick), Boophilus microplus (cattle tick), Chorioptes spp. (mange mite), Demodex bovis (cattle follicle mite), Demodex canis (dog follicle mite), Dermacentor andersoni (Rocky Mountain spotted fever tick). Dermacentor variabilis (American dog tick), Dermanyssus gallinae (chicken mite), Ixodes ricinus (common sheep tick), Knemidokoptes gallinae (deplumming mite), Knemidokoptes mutans (scaly-leg mite), Otobius megnini (ear tick), Psoroptes equi (scab mite), Psoroptes ovis (scab mite), Rhipicephalus sanguineus (brown dog tick), Sarcoptes scabiei (mange mite), Insects- Aedes (mosquitoes), Anopheles (mosquitoes), Culex (mosquitoes), Culiseta, Bovicola bovis (cattle biting louse), Callitroga homnivorax (blowfly), Chrysops spp. (deer fly), Cimex lectularius (bed bug), Cochliomyia spp. (screwworm), Ctenocephalides canis (dog flea), Ctenocephalides felis (cat flea), Culicoides spp. (midges, sandflies, punkies, or no-see-ums), Damalinia ovis (sheep biting louse), Dermatobia spp. (warble fly), Gasterophilus haemorrhoidalis (nose bot fly), Gasterophilus intestinalis (common horse bot fly), Gasterophilus nasalis (chin fly), Glossina spp. (tsetse fly), Haematobia irritans (horn fly, buffalo fly), Haematopinus asini (horse sucking louse), Haematopinus eurysternus (short nosed cattle louse), Haematopinus ovillus (body louse), Haematopinus suis (hog louse), Hydrotaea irritans (head fly), Hypoderma bovis (bomb fly), Hypoderma lineatum (heel fly), Linognathus ovillus (body louse), Linognathus pedalis (foot louse), Linognathus vituli (long nosed cattle louse), Lucilia spp. (maggot fly), Melophagus ovinus (sheep ked), Musca spp. (house fly, face fly), oestrus ovis (nose bot fly), Pediculus spp. (lice), Phlebotomus spp. (sandfly), Phormia regina (blowfly), psorophora spp. (mosquito), Pthirus spp. (lice), Reduvius spp. (assassin bug), Simulium spp. (black fly), Solenopotes capillatus (little blue cattle louse), Stomoxys calcitrans (stable fly), Tabanus spp. (horse fly), Tenebrio spp. (mealworms), Triatoma spp. (kissing bugs).

The present compounds' ectoparasiticidal activity is achieved when the compounds contact the pests. The contact can be of the egg, larvae, adult, or other life stage. "Contact" includes ingestion of the compound by the pest.

Techniques for delivering ectoparasiticides are well known to those skilled in the art. In general, a present compound is applied to the exterior surface of an animal, whereby it contacts pests already present on the host as well as those which arrive on the host's body within the efficacy period of the compound. Typically, the compound is formulated in a liquid formulation which is sprayed onto the animal's surface or poured onto the animal's surface. Another conventional treatment is a "dip", whereby cattle are treated by being substantially immersed in a dilute solution of an ectoparasiticide. For some hosts and pests, the formulation can be a dust, which is sprinkled onto the host, or a shampoo or cream which is employed in bathing the animal. Collars on cats and dogs are also employed as a way of delivering an ectoparasiticide directly to the animal's surface.

In another technique, an ectoparasiticide is applied to locations frequented by animals, so that pests are thereby contacted by the compound even as in direct application to the host. Application to pet bedding is well known, as is application to carpeting. For cattle, dusting bags are well known. These are positioned in a doorway where the cattle inevitably rub against the bag and pests are contacted by the present compound.

In yet another embodiment, the present compounds can be used to control insects and arachnids which are pests in the feces of cattle and other animals. In this embodiment, the compounds are administered orally and the compounds travel through the intestinal tract and emerge in the feces. Control of pests in the feces indirectly protects the animals from the pests.

The compounds are formulated for use as ectoparasiticides in manners known to those skilled in the art. In general, a formulation will include a compound of the present invention and one or more physiologically acceptable adjuvants. Formulations include concentrated versions, in which the present active agent is present in a concentration of from 0.001 to 98.0 percent, with the remaining content being physiologically acceptable carriers. Such formulations, especially those with less than 50 percent of the present compound, can sometimes be used directly, but these formulations can also be diluted with other physiologically acceptable carriers to form more dilute treating formulations. These latter formulations can include the active agent in lesser concentrations of from 0.001 to 0.1 percent.

In another embodiment, the present compounds are usefully combined with other ectoparasiticides or with anthelmentics, the latter also known as endoparasiticides ("endo" = internal, controlling internal parasites which are typically platyhelminthes and nemathelminthes). Representative such endoparasiticides include the following:
Abamectin, Albendazole, Avermectin, Bunamidine, Coumaphos, Dichlorvos, Doramectin, Epsiprantel, Febantel, Fenbendazole, Flubendazole, Ivermectin, Levamisole, Mebendazole, Milbemycin, Morantel, Moxidectin, Netobimin, Niclosamide, Nitroscanate, Oxfendazole, Oxibendazole, Piperazine, Praziquantel, pyrantel, Ricombendazole, Tetramisole, Thiabendazole, Clorsulon, Closantel, Diamphenethide, Nitroxynil, Oxyclozanide, Rafoxanide, Triclabendazole.

Representative other ectoparasiticides include the following:
Abamectin, Alphamethrin, Amitraz, Avermectin, Coumaphos, Cycloprothrin, Cyfluthrin, Cyhalothrin, Cypermethrin, Cyromazine, Deltamethrin, Diazinon, Diflubenzuron, Dioxathion, Doramectin, Famphur, Fenthion, Fenvalerate, Flucythrinate, Flumethrin, Hexaflumuron, Ivermectin, Lindane, Lufenuron, Malathion, Methoprene, Metriphonate, Moxidectin, Permethrin, Phosme, Pirimiphos, Propetamphos, Propoxur, Rotenone, Temephos, Tetrachlorvinphos, Trichlorfon, Zetacypermethrin, B.t. Biotoxins and Boric Acid.

### Examples

### General Experimental Section

All reagents and solvents were used directly as purchased from commercial suppliers and all reactions were conducted with constant magnetic stirring at ambient temperature (20-22°C), unless otherwise noted. All reactions involving organometallic, moisture sensitive, or metal hydride reagents were conducted in commercially available dry solvents under a dry nitrogen atmosphere. Partitions, extractions,or washes with NaCl, NaHCO₃, NH₄Cl, and other salts refer to saturated aqueous solutions of these salts. Reactions are typically "worked-up" by extraction of an organic solution of the products with one of the above salt solutions; the organic layer was dried with K₂CO₃, Na₂SO₄, or MgSO₄, filtered and evaporated *in vacuo.* Reversed-phase thin layer chromatography (RPTLC) was done on glass-backed octadecylsilane-bonded plates, 0.2 mm thickness from Whatman. Chromatography refers to flash chromatography and was performed on E. Merck silica gel 60 (230-400 mesh). Reversed-phase high performance liquid chromatography (RPHPLC) was performed on C18 bonded silica gel (Rainin Dynamax 60 A, 8µm). All melting points were determined in open capillaries and are uncorrected. ¹H NMR and ¹³C NMR spectra were determined at 300 or 400 MHz and 75 or 101 MHz, respectively, in CDCl₃. Mass spectral data were measured via electrospray ionization (ESI). Elemental analyses were provided by analytical laboratory of DowElanco or Midwest Microlabs.

### Example 1 Synthesis of Spinosyn A aglycone

A sample of Spinosyn A (8.50g, 11.61mmol) was dissolved in EtOH (100mL). Water (100mL) was added, and upon stirring, to this solution was added 4N H₂SO₄ (aq) solution (200mL). The reaction mixture was heated to reflux, under a nitrogen atmosphere, for 4.5 hr. It was cooled to room temperature, diluted with toluene (250mL) and ethyl acetate (300mL), and brine (300mL) was added. After extraction, the phases were separated. The water layer was extracted once more with ethyl acetate (100mL). The combined organic layers were washed successively with diluted brine (3x) and 5% aqueous NaHCO₃ (2x) and then dried over anhyd MgSO₄ and concentrated *in vacuo.* The residue was purified on a flash SiO₂ column (250g; Ethyl acetate). After concentration to dryness of the chromatographically pure fractions, Spinosyn A aglycone was obtained as a glassey solid (4.08g, 87%), [a]₅₈₉ = -145.6° (MeOH), [a]₅₈₉ = -131.7° (CHCl₃).

### Example 2 Synthesis of Spinosyn A 17-Psa

Spinosyn A (20.0 gms, 27.4 mmol) was dissolved in 300 ml of 1N H₂SO₄ and heated to 80°C, with mechanical stirring for 2 hr. The reaction was then cooled to room temperature. The precipitate was filtered with suction, and washed with fresh 1N H₂SO₄. The solid product was then dissolved in dichloromethane, washed with brine, dried over K₂CO₃, and evaporated under reduced pressure. The crude product was purified by silica gel chromatography with 70% EtOAc in Hexane to give Spinosyn A 17-Psa (14.46 gms; 89%) as a colorless glass.

### Example 3 Spinosyn A 9-Psa

A suspension of N-chlorosuccinimide (1.20 gm, 9.00 mmol) in dichloromethane (45 ml) was cooled to -78°C under nitrogen. Diethyl sulfide (1.07 ml, 9.90 mmol) was added neat to this suspension over 5 min. and the mixture was stirred at -78°C for 0.5 hr. Spinosyn J (2.15 gm, 3.00 mmol) in 8 mL dichloromethane was added slowly to this mixture over 15 min keeping the reaction temperature below -60°C. When the addition had been completed, the solution was stirred at -78°C for 6 hrs. Triethylamine (1.25 mL, 9.00 mmol) was then added dropwise and the solution was allowed to warmed to room temperature. The reaction was diluted with 40 mL dichloromethane and poured into 20 mL 1N sodium bisulfate and 30 mL water. The layers were separated and the organic layer extracted with 30 mL saturated sodium bicarbonate and dried over sodium sulfate. The solvent was evaporated under reduced pressure, and the residue was dissolved in MeOH (100 ml). Anhydrous K₂CO₃ (4.15 gm, 30.00 mmol) was added and the suspension was stirred at room temperature. After stirring for 2 hr., the mixture was cooled to 0°C and solvent evaporated to 1/5th its volume on a rotary evaporator. Dichloromethane (100 mL) and water (100 mL) were added and the layers separated. The aqueous layer was extracted with 3 x 50 mL dichloromethane and the organic extracts combined and washed with first with water then brine solution. The solution was dried over sodium sulfate, filtered and concentrated on a rotary evaporator to give 2.49 g tacky yellow oil. This product was purified by flash chromatography (250 gm silica gel, 5% MeOH in dichloromethane with 0.5% conc NH₄OH, to give Spinosyn A 9-Psa as a white foam (1.52 gm ; 93%) ¹HNMR (CDCl₃) δ 78 (br s, 1, H-13), 4.63 (m, 1, H-21), 4.43 (m, 2, H-1", H-9), 2.23 (s, 6, N(CH₃)2.

### Example 4 Preparation of Spinosyn with Culture Saccharopolyspora spinosa NRRL 18395

### Part A. Shake-flask Fermentation

The culture *Saccharopolyspora spinosa* NRRL 18395, either as a lyophilized pellet or as a suspension maintained in liquid nitrogen, was used to inoculate a vegetative medium having composition A or B (medium B is preferred for large scale production), where vegetative medium A was comprised of (amount in %) trypticase soy broth* 3.0, yeast extract 0.3, MgSO₄ • 7H₂O 0.2, glucose 0.5, maltose 0.4, deionized water q.s. 1 liter, without a pH adjustment (*purchased from Baltimore Biological Laboratories) and vegetative medium B was comprised of enzyme-hydrolyzed casein** 3.0, yeast extract 0.3, MgSO₄ • 7H₂O 0.2, glucose 1.0, deionized water q.s.1 liter, with a pH of 6.2 which was adjusted to 6.5 with sodium hydroxide, (**purchased from NZ Amine A, Sheffield Products, P.O. Box 638, Norwich, NY 13815). Slants or plates were prepared by adding 2.5% agar to vegetative seed medium A or B. The inoculated slant were incubated at 30°C for from about 10 to 14 days. The mature slant culture was scraped with a sterile tool to loosen the spores and remove and macerate the mycelial mat. About one-fourth of the loosened spores and culture growth thus obtained was used to inoculate 50 mL of a first-stage vegetative seed medium. Alternatively, the first-stage medium may be inoculated from a liquid nitrogen ampoule. When the culture is maintained in liquid nitrogen, ampoules are prepared using equal volumes of vegetative culture (48-72 hr. incubation, 30°C) and suspending medium. The suspending medium contains lactose (100 g), glycerol (200 mL) and deionized water (q.s. to 1 L). A liquid nitrogen ampoule is used to inoculate 100 mL of vegetative medium in 500-mL Erlenmeyer flasks (or 50 mL medium in 250-mL flasks). The cultures are incubated at 30°C for 48 hours on a shaker orbiting in a two-inch (5.08 cm) circle at 250. The incubated culture (5% v/v inoculum) is used to inoculate 100 mL of a production medium having the following composition: (amount in %) glucose 4.0, vegetable protein, partially hydrolyzed enzymatically* 1.5-3.0, cottonseed flour** 1.0, CaCO₃ (reagent or technical grade) 0.3, soybean oil 1.0, tap water q.s. 1 liter (presterilization pH adjusted to 7.0 with NaOH) *Sheftone H, Sheffield Products, **Proflo, Traders Protein, P.O. Box 8407, Memphis, TN 38108. The inoculated production medium was incubated in 500-mL Erlenmeyer flasks at 28-30°C for 6 to 8 days on a shaker orbiting in a two-inch circle at 250 rpm.

### B. Stirred Bioreactor Fermentation

In order to provide a larger volume of inoculum, 10 mL of incubated first-stage medium, prepared as described in Section A, was used to inoculate 400 mL of a second-stage vegetative medium having the same composition as that of the first-stage vegetative medium. This second-stage medium was incubated in a 2-L wide-mouth Erlenmeyer flask for about 48 hours at 30°C on a shaker orbiting in a two-inch circle at 250 rpm. Incubated second-stage vegetative medium (2 L) thus prepared was used to inoculate 80 to 115 liters of sterile production medium, prepared as described in Section A. Additional soybean oil is added to control foaming, if needed.

The inoculated production medium was allowed to ferment in a 165-L stirred bioreactor for 5 to 8 days at a temperature of 28°C. The airflow and agitator speed in the stirred vessel were computer controlled to maintain a dissolved oxygen level at or above 50% of air saturation.

### Example 5 Isolation of Spinosyn A, B, C and D

Fermentation broth (225 liters), prepared as described in Example 4, was filtered using a filter aid (1% Hyflo), and the separated biomass was washed with water (~ 50 L). The biomass was then agitated with methanol (~ 100 L) for about one hour and filtered. The methanol filtrate was concentrated to a volume of about 1 liter. The concentrate was extracted three times with diethyl ether (1 L each). The combined ether extracts were concentrated to a volume of about 200 mL. A portion of the concentrate (8 mL) was chromatographed on a silica gel column (RP-8 Lobar, size B, E.M. Science, a Division of E.M. Industries, 30 Inc.). This procedure was repeated for a total of 12 runs (cycles). The instrumental set-up and execution procedure to perform the preparative chromatography in an "Autoprep" mode is described as follows:

A complete "Autoprep" HPLX system was comprised of three Rainin Rabbit HPX pumps, one pressure module, one Gilson Model 20 1B HPLC fraction collector, one Isco-v4 absorbance detector and one Apple Macintosh Plus computer. The complete system is arranged according to instructions given in the Dynamax HPLC Method Manager manual from Rainin Instrument Company, Inc. The "Autoprep" HPLC configuration takes advantage of system automation to permit preparative separations to be run repetitively under virtually identical conditlons with virtually identical results. Collecting and pooling corresponding fractions from multiple runs provides chromatographic capacity without the need for a large column. Two solvent mixtures (A) and (B) were used in the isocratic mode at a flow rate of 8.0 mL/min. Solvent system A was comprises of 95 mL CH₃OH, 95 mL CH₃CN, 10 mL H₂O, and solvent system B was 100 mL CH₃OH, 100 mL CH₃CN, 0 mL H₂O. The isocratic mixture used contains 60% of solvent B.

The runtime for each cycle was 28.0 minutes. The eluates from the first 16 minutes of each run were discarded. The following eluates were collected in 6 time-factions, 2 minutes (16 mL) each. The automatically combined fractions from each of the 12 cycles resulted in 6 final fractions (chromatographic cuts). The presence of the active Spinosyn compounds was determined by analyzing each final fraction for mosquito larvae activity and also by analytical HPLC. The active fractions were then combined according to their activity and HPLC profiles and were further purified, using the same "Autoprep" HPLC and solvent system, but with a high resolution, 21.4-mm x 25-cm preparative column (Rainin Dynamax), prepacked with 8 µ C-18 reversed phase silica gel, to give Spinosyn A, B, C and D. Spinosyns A and D crystallize from CH₃OH/H₂O.

### Example 6 Purification of Spinosyn A and D

Fermentation broth (10 L) was prepared as described in Example 4 Sect. A, except that 1) 200 mL of production medium was used in 1-L flasks; 2) soybean oil was omitted from the production medium; and 3) incubation was at 30° for 4-6 days. The broth was filtered. The filtrate, containing 4 mcg of Spinosyn A/mL and no detectable quantities of Spinosyn B, C, or D/mL, was discarded. The biomass was washed with water and extracted for one hour with methanol. The extract (7 L) contained 72 mcg of Spinosyn A/mL and 7 mcg of Spinosyn D/mL. The methanol extract was concentrated to a volume of 5 L, and added to HP-20 resin (150 mL, Mitsubishi Chemical Industries, Ltd., Japan) in water (2 L). This mixture was stirred for one hour. The HP-20 resin mixture was then placed in a glass column. The initial effluent and the eluate using methanol:water (1:1, 1 L) were not active. The second eluate using methanol:water (7:3, 1 L) contained trace quantities of Spinosyn A. The following eluate using methanol (1 L) contained the Spinosyn A and Spinosyn D activity. The methanol eluate was concentrated and combined with 2 similar fractions from other work-ups and concentrated to dryness. The residue was dissolved in 75 mL of methanol:THF (4:1) and precipitated by addition into 10 volumes of acetonitrile. The mixture was filtered, and the filtrate was concentrated to dryness. The residue was dissolved in methanol (25 mL) and applied to a 5.5 x 90-cm column of LH-20 Sephadex (Pharmacia LKB Biotechnology Inc., U.S.A.), prepared in methanol, collectlng and analyzing 125 25-mL fractions, using the HPLC procedure described in Example 1.

Fractions containing the desired compounds were combined and concentrated. The residue was dissolved in methanol (10 mL) and applied to a 41.1-mm x 25-cm preparative column prepacked with 8µ C-18 reversed phase silica gel (Rainin Dynamax). The column was conditioned in methanol: acetonitrile:water (37.5:37.5:25). After sample application, the column was developed using a 180-min linear gradient of the following solvents: solvent system A 37.5 mL CH₃OH, 37.5 mL CH₃CN, 25 mL H₂O, and solvent system B was 45 mL CH₃OH, 45 mL CH₃CN, 10 mL H₂O. Fractions containing Spinosyn A were pooled, to dryness, dissolved in t-BuOH (5 mL) and lyophilized to give 778 mg of pure Spinosyn A. Fractions containing Spinosyn D were combined with D-containing fractions from 6 similar separations and were concentrated and chromatographed as described herein, using the same column but different solvents. The column was conditioned in methanol:acetonitrile: water(40:40:20). The solvent systems used to develop the column in a 180-min linear gradient operation were: solvent system A was comprises of 40 mL CH₃OH, 40 mL CH₃CN, 20 mL H₂O, and solvent system B was 95 mL CH₃OH, 95 mL CH₃CN, 10 mL H₂O. Fractions containing Spinosyn D were combined and concentrated. The residue was dissolved in t-BuOH (5 mL) and lyophilized to give 212 mg of Spinosyn D.

### Example 7 Isolation of Components Spinosyn E, F, G, H and J and the Spinosyn A 17-Psa

Fermentation broth (8 L), prepared using procedures similar to those described in Example 4, was treated as described in Example 4.

Fractions from the LH-20 Sephadex column containing the desired compounds were combined with corresponding fractions from similar fermentations. Since components E, F, G, H, J and the pseudoaglycone of A were produced in very small quantlties, numerous fermentations were required to provide sufficient quantities for further purification.

A pool of minor factors, prepared in this manner and containing approximately 1.6 grams of solid 15 material, was applied to an HPLC column (Rainin Dynamax) prepacked with 8 micron C-18 reversed phase silica gel (ODS), as described in Example 4. The column was conditioned in CH₃OH:CH₃CN:H₂O (75:75:50), and the gradient was run from 100% of solvent (A) to 50% (B) with the following solvent systems: Solvent system A 75% CH₃OH, 75% CH₃CN, 50% H₂O and solvent system B 95% CH₃OH, 95% CH₃CN, 10% H₂O were collected in 25-mL fractions. The following fractions 30 were pooled:

| Pool | Fractions |
|---|---|
| 1 | 31 - 44 |
| 2 | 45 - 63 |
| 3 | 64 - 69 |
| 4 | 70 - 80 |
| 5 | 81 - 130 |
| 6 | 131 - 160 |

A portion of pool 5 (100 mL) was concentrated to a residue, dissolved in methanol (1 mL) and applied to a 21.4-mm x 250-mm HPLC column (Rainin Dynamax), as described in Example 5. The column was conditioned using solvent system (A) of the following solvent systems: solvent system A 30:30:40 CH₃OH/CH₃CN/H₂O(1N NH₄OAc,pH 5), and solvent system B 95:95:10 CH₃OH/CH₃CN/H₂O (1N NH₄OAc,pH 5) were developed using a 120-minute linear gradient from 100% solvent (A) to 50% of solvent (B), collecting 15-mL fractions at 7.5 mL/min. Elution was continued at 50% (B) for an additional 60 minutes. The following fractions were pooled:

| Pool | Fraction | Component |
|---|---|---|
| 1 | 37 | F |
| 2 | 38 - 48 | E |
| 3 | 52 - 63 | B, G |
| 4 | 65 - 70 | H, J |

These pools were combined with pools from other chromatographic runs using similar starting materials. The combined pools were further purified using column chromatography, as described herein; desalted on HP-20 resins, using standard techniques; and concentrated and lyophilized to give the following components:

| Pool | Fraction | Component |
|---|---|---|
| E | 249 | 717 |
| F | 4 | 717 |
| G | 104 | 731 |
| H, J | 87 | 717 |
| Pseudo A | 288 | 590 |

### Example 8 Preparation of Spinosyn K, Spinosyn 0 and Spinosyn Y with Culture NRRL 18743

### A. Shake-flask Fermentation

The culture *Saccharopolyspora spinosa* NRRL 18743, either as a lyoshiiized pellet or as a suspension maintained in liquid nitrogen, was used to inoculate a vegetative medium having the following composition:

| Vegetative Medium | |
|---|---|
| Ingredient | Amount (g) |
| Trypticase soy broth* | 30 |
| Yeast extract | 3 |
| MgSO_{4_}H₂O° | 2 |
| Glucose | 5 |
| Maltose | 4 |
| Deionized water autoclave 30 min at 120°C | q.s. 1-L |

| | |
|---|---|
| * Baltimore Biological Laboratories, Cockeysville, MD | |

Slants or plates can be prepared by adding 2.5% agar to the vegetative medium. The inoculated slant was incubated at 30°C for about 10 to about 14 days. The mature slant culture was scraped with a sterile tool to loosen the spores and remove and macerate the mycelial mat. About one-fourth of the loosened spores and culture growth thus obtained was used to inoculate 50 ml of a first-stage vegetative medium. Alternatively, the first-stage medium may be inoculated from a liquid nitrogen ampoule.

Liquid-nitrogen-stock inoculum was prepared by homogenizing a vegetative culture, diluting 1:1 (volume:volume) with a sterile suspending agent of glycerol:lactose:water (2:1:7), and dispensing into sterile tubes (1.5 ml/tube). The diluted inoculum was then stored over liquid nitrogen in appropriate storage containers and used as a working stock inoculum for the cult vation of shake-flask cultures and fermenter seed inoculum.

Liquid nitrogen ampoule was quick thawed and 0.5ml was used to inoculate 50 ml of vegetative medium in 250-ml wide-mouth Erlenmeyer flasks. The cultures are incubated at 32°C for 48 hours on a shaker orbiting in a two-inch (5.08 cm) circle at 250 rpm.

The incubated cultures (5% v/v inocutum) was used to inoculate 25 ml of a production medium having the following composition:

| Production Medium | |
|---|---|
| Ingredient | Amount (g) |
| Glucose | 80 |
| Peptonized milk* | 20 |
| Cottonseed flour** | 30 |
| Corn steep liquor | 10 |
| CaCO₃ (tech. grade) | 5 |
| Methyl oleate | 30 |
| Tap water | q.s. to 1-L |

| | |
|---|---|
| * Peptonized Milk Nutrient, Sheffield Products, Norwich, NY | |
| **Proflo, graders Protein, Memphis TN | |

The inoculated production medium is incubated in 250-ml wide-mouth Erlenmeyer flasks at 30°C for 7 days on a shaker orbiting in a two-inch circle at 250 rpm.

### B. Stirred Reactor Fermentation

In order to provide a larger volume of inoculum, 10 ml of incubated first stage medium, prepared as described above, is used to inoculate 400 ml of a second-stage vegetative medium having the same composition as that of the first-stage medium. This second-stage vegetative medium is >98% pure. Spinosyn K containing pools from the first preparative HPLC separation and the repurification of Spinosyn O were combined, concentrated to 200 ml. and desalted in the same manner as Spinosyn 0. Fractions containing >98% pure component K were pooled, concentrated to dryness, and lyophilized from t-BuOH to give Spinosyn K (11.1 g; >99% pure).

### Example 9 Isolation of Spinosyn K, Spinosyn O and Spinosyn Y from Strain NRRL 18743

Fermentation broth (260-L) was prepared as substantially described in the Example above part B. Acetone (260-L) was added to the whole broth after adjusting the pH to 3.0 with 5N HCL. The resulting mixture was filtered through a ceramic filter to give filtrate (480-L) which was held over the weekend under refrigeration. The broth/acetone filtrate was adjusted to pH 12 with 25% NaOH and refiltered twice through the ceramic filter prior to loading onto a steel column (10-L, 10 cm x 122 cm) containing HP-20ss resin (Mitsubishi Chemical Industries, Ltd., Japan) at a flow rate of 0.5-L/minute. The column was washed with CH₃CN - CH₃OH - 0.1% aq.NH₄OAc (adjusted to pH 8.1 with NH₄OH) (25:25:50; 20-L). Spinosyn K, O and Y were eluted with CH₃CN - CH₃OH - 0.1% aq. NH₄OAc (adjusted to pH 8.1 with NH₄OH) (95:95:10; 30-L) at a flow rate of 1-L/minute. The equate (30-L) was concentrated, redissolved in CH₃OH, reconcentrated to dryness, redissolved in CH₃OH (100 ml), then precipitated into CH₃CS (2-L). The resulting precipitate was removed by filtration, washed with CH₃CN, and discarded: the combined filtrate and wash (3-L) was concentrated to dryness. The resulting residue was redissolved in dichloromethane (50 ml) and applied to a column (7.5 cm x 50 cm) of silica gel (EM grade 62. 60 - 200 mesh) equilibrated in acetonitrile. The column was eluted with CH₃CN (10-L), then CH₃CN - CH₃OH (9:1: 20-L), followed by CH₃CN - CH₃OH (8:2: 10L), collecting 1-L fractions. Fractions 11 - 30 were pooled and concentrated to dryness. The resulting residue was dissolved in CH₃OH (50 ml) and applied (in 10 runs) to a preparative reverse phase HPLC column (Rainin Dynamax-60Å 8 µm C18, 41.4 mm ID x 25 cm with 41.4 mm x 5 cm guard module) equilibrated in H₂O - CH₃OH - CH₃CN; (50:175:175, containing 0.1% NH₄OAc). The column was eluted at a flow rate of 40 ml/minute with a 60 minute linear gradient from H₂O CH₃OH - CH₃CN; (50:175:175, containing 0.1; NH₄OAc) to H₂O - CH₃OH CH₃CN: (10:45:45, containing 0.1; NH₄OAc). Progress of the separation was monitored with a variable wavelength UV detector tuned to 250 nm. The first three peaks collected (10 runs pooled) corresponded to the elution of minor Spinosyn Y (pool 1, 1-L), Spinosyn K (pool 2, 8-L) and Spinosyn O (pool 3, 4-L). Spinosyn K was concentrated to a small volume, then desalted by rechromatographing on the same column, eluting without buffer. The effluent corresponding to the UV absorption peak was concentrated to dryness, dissolved in t-BuOH, and lyophilized to give pure Spinosyn K (7.3 g). Spinosyn O was desalted and lyophilized in like manner to give pure Spinosyn O (1.4 g). Spinosyn Y was desalted by similar chromatography (Rainin Dynamax-60A 8 pm C18 column, 21.4 mm ID x 25 cm with 21.4 mm x 5 cm guard module) and lyophilized in like manner to give pure Spinosyn Y (46 mg).

### Example 10 Preparation of Spinosyn J, Spinosyn L, Spinosyn M, and Spinosyn N with NRRL 18719

The culture *Saccharopolyspora spinosa* NRRL 18719 was used to obtain Spinosyn J, Spinosyn L, Spinosyn M, and Spinosyn N.

### A. Shake-flask Fermentation

The culture *Saccharopolyspora spinosa* NRRL 18719, either as a lyophilized pellet or as a suspension maintained in liquid nitrogen, was used to inoculate a vegetative medium having the following composition:

| Vegetative Medium | |
|---|---|
| Ingredient | Amount (g) |
| Trypticase broth* | 30 |
| Yeast extract | 3 |
| MgSO₄ • 7H₂O | 2 |
| Glucose | 5 |
| Maltose | 4 |
| Deionized water Autoclave 30 min at 120°C | q.s. 1 L |

| | |
|---|---|
| * Baltimore Biological Laboratories, Cockeysville, MD | |

Slants or plates were prepared by adding 2.5% agar to the vegetative medium. The inoculated slant was incubated at 30°C for about 10 to about 14 days. The mature slant culture was scraped with a sterile tool to loosen the spores and to remove and macerate the mycelial mat. About one-fourth of the loosened spores and culture growth thus obtained were used to inoculate 50 ml of a first-stage vegetative medium. Alternatively, the first-stage medium may be inoculated from a liquid nitrogen ampoule.

When the culture is maintained in liquid nitrogen, ampoules are prepared by homogenizing a vegetative culture (48-72 hours incubation, 30°C), diluting 1:1 (volume:volume) with a sterile suspending agent, and dispensing into sterile tubes (1.5 ml/tube). The suspending agent contains lactose (100 g), glycerol (200 ml), and deionized water (q.s. to 1 L).

A liquid nitrogen ampoule was used to inoculate 100 ml of vegetative medium in 500-ml Erlenmeyer flasks (or 50 ml of medium in 250-ml flasks). The cultures were incubated at 30°C for 48 hours on a shaker orbiting in a two-inch (5.08 cm) circle at 260 rpm.

The incubated culture (10% v/v inoculum) was used to inoculate 50 ml or 100 ml, dependent on the size of the Erlenmeyer flask, of a production medium having the following composition:

| Production Medium | |
|---|---|
| Ingredient | Amount (g) |
| Glucose | 80 |
| Peptonized milk* | 20 |
| Cottonseed flour** | 30 |
| Corn steep liquor | 10 |
| CaCO₃ (tech. grade) | 5 |
| Methyl oleate | 30*** |
| Tap water | q.s. to 1 L |

| | |
|---|---|
| pH adjusted to pH 7.0 with 1N NaOH, sterilized 40 min. at 120°C * Peptonized Milk Nutrient, Sheffield Products, Norwich, NY 13815 | |
| ** Proflo, Traders Protein, Memphis TN 38108 | |
| *** The amount of methyl oleate was 30 ml | |

The inoculated production medium was incubated in 250-ml or 500-ml Erlenmeyer flasks at 30°C for 7 to 10 days on a shaker orbiting in a two-inch circle at 260 rpm.

### B. Stirred Reactor Fermentation

In order to provide a larger volume of inoculum, 10 ml of incubated first stage medium, prepared as described in Section A, was used to inoculate 400 ml of a second-stage vegetative medium having the same composition as that of the first-stage medium. This second-stage vegetative medium was incubated in a 2-L wide-mouth Erlenmeyer flask for about 48 hours at 30°C on a shaker orbiting in a two-inch circle at 260 rpm. The incubated second-stage vegetative medium (2 L) was used to inoculate 80 to 115 liters of sterile production medium, prepared as described in Section A above.

The inoculated production medium was allowed to ferment in a 165-L stirred bioreactor for 7 days to 10 days at a temperature of 30°C. The air-flow and agitator speed in the stirred vessel were computer controlled to maintain a dissolved oxygen level at or above 60% to about 80% of air saturation.

The following tables illustrate the amount of Spinosyn J, Spinosyn L, Spinosyn M, and Spinosyn N that were produced by culture NRRL 18719

**Table I**

| Amount of Spinosyn compounds produced by shake-flask fermentation with culture NRRL 18719 | |
|---|---|
| Factor | Amount (mg/ml) |
| Spinosyn J | 661 |
| Spinosyn L | 133 |
| Spinosyn M | ND* |
| Spinosyn N | ND* |

| | |
|---|---|
| *Not determined by HPLC assay | |

**Table II**

| Amount of spinosyn compounds produced by stirred reactor fermentation with culture NRRL18719 | |
|---|---|
| Factor | Amount (mg) |
| Spinosyn J | 5,282 |
| Spinosyn L | 2,487 |
| Spinosyn M. | 136 |
| Spinosyn N | 71 |

Example 11 Spinosyn J, Spinosyn L, Spinosyn M, and Spinosyn N with culture NRRL 18719 Fermentation broth (105 L), prepared as described above, was adjusted to pH 10 (initially pH 6.8) by adding 5N NaOH. The resulting mixture was filtered through a ceramic filter. The filtrate was discarded, a mixture of acetone and water (1:1, 50 L) was added to the mycelial solids, and the resulting mixture was filtered. A second mixture of acetone and water (1:1, 50 L) was added to the mycelial solids, and the pH of the resulting mixture was adjusted to pH 3.0 with 25% sulfuric acid. The resulting mixture was filtered, and a third mixture of acetone and water (1:1 50 L) was added to the mycelial solid. The resulting mixture was filtered and the acidic filtrates were combined.

The combined filtrates were extracted with heptane (10 L). The phases were separated and the aqueous phase added to a second portion of heptane (10 L). The pH of the resulting mixture was adjusted to pH 10 with 5N NaOH. The resulting emulsion was diluted with 50 L of water. The phases were separated and the aqueous phase extracted with a third portion of heptane (10 L). The phases were separated and the second and third heptane extracts were combined and concentrated to a volume of about 4 liters. Upon standing, the concentrate separated into 3 phases: aqueous, emulsion, and organic. The organic phase was lyophilized to give 15.29 g of crude product.

The crude product was dissolved in methanol (500 mL), filtered, and concentrated to dryness *in vacuo*. The residue was dissolved in a second portion of methanol (20 ml) and applied to a column of LH-20 SEPHADEX (Pharmacia LKB Biotechnology, Inc., Piscataway, NJ, 7.5 cm x 46cm), eluting with methanol and collecting 25 ml fractions. Using the HPLC system, the fractions were analyzed to determine which fractions contained the spinosyn compounds. Fractions 18-50 were combined and concentrated to dryness.

The residue was dissolved in a mixture of methanol, acetonitrile, and water (5:5:1) and chromatographed in 1 ml portions on a preparative reverse-phase HPLC column (Rainin Dynamax-60A, C18, 41.4 mm x 300 mm, 8 mm particles, 60A pore, Woburn, MA). The column was eluted with a mixture of methanol, acetonitrile and water (87.5:87.5:25) with ammonium acetate added to a final concentration of 0.1% (pH 7.6). The fractions were analyzed using an HPLC system, combining like fractions and concentrating to give three semi-pure concentrates A, B, and C.

Semi-pure concentrate C was rechromatographed on the system described in the preceding paragraph, loading 200 mL on each of 10 runs. The fractions from each of the runs were combined and concentrated to give preparations C1 and C2. Preparation C2 was chromatographed a third time; however, water was used in place of the 0.1% ammonium acetate (desalting step). Fractions containing Spinosyn L in at least 99.5% HPLC purity were combined and concentrated. The residue was crystallized from ethanol/water (1:1) to give 2.4 g of spinosyn L.

Preparation C1 and semi-pure concentrate B were combined and desalted as described in the preceding paragraph (12 x 200 mL runs); however, the desired compound was eluted with a mixture of methanol, acetonitrile, and water (11:11:3). The fractions containing Spinosyn J in at least 99.5% HPLC purity were combined and concentrated. The residue was dissolved in hot *t*-butanol and lyophilized to give 4.3 g of Spinosyn J.

Semi-pure concentrate A was chromatographed as described above, except the desired compounds were eluted with a mixture of methanol, acetonitrile, and water (37.5:37.5:25), with ammonium acetate added to final concentration of 0.1%. The fractions from each of the runs (4) were combined and concentrated to give preparations A1, A2, and A3.

Preparation A1 was chromatographed using the column described above; however, the column was eluted with a mixture of methanol, acetonitrile, and water (2:2:1). Fractions containing Spinosyn M in at least 99.5% HPLC purity were combined and concentrated. The residue was dissolved in *t*-butanol and lyophilized to give 136 mg of Spinosyn M.

Preparation A2 was chromatographed and processed as described in the preceding paragraph to give 71 mg of Spinosyn N.

### Example 12 Preparation of Spinosyn Q with Culture NRRL 18823

### A. Shake-flask Fermentation

The culture *Saccharopolyspora spinosa* NRRL 18823, either as a lyophilized pellet or as a suspension maintained in liquid nitrogen, was used to inoculate a vegetative 5 medium having the following composition:

| Vegetative Medium 1 | |
|---|---|
| Ingredient | Amount (g) |
| Trypticase Broth* | 30 |
| 0 Yeast extract | 3 |
| MgSO₄ 7H₂O | 2 |
| Glucose | 5 |
| Deionized water Autoclave 30 min at 120°C | q.s. 1 L |

| | |
|---|---|
| * Baltimore Biological Laboratories, Cockeysville, MD | |

The first-stage medium may be inoculated from a liquid nitrogen ampoule. Such ampoules are prepared by homogenizing a vegetative culture (48 - 72 hours incubation, 30°C) diluting 1:1 (volume:volume) with a sterile suspending agent, and dispensing into sterile tubes (1.5 ml/tube). The suspending agent contains lactose (100 9), glycerol (200) ml, and deionized water(q.s. to 1 L). A liquid nitrogen ampoule is used to inoculate 50 ml of vegetative medium in 250-ml wide-mouthed Erlenmeyer flasks. The cultures are incubated at 32°C for 48 hours on a shaker orbiting in a two-inch (5.08 cm) circle at 250 rpm.

The incubated culture (58 v/v inoculum) was used to inoculate 50 ml Erlenmeyer flask, of a production medium having the following composition:

| Production Medium | |
|---|---|
| Ingredient | Amount (g) |
| Glucose | 80 |
| Peptonized milk* | 20 |
| Cottonseed flour** | 30 |
| Corn steep liquor | 10 |
| CaCO₃ (tech. grade) | 5 |
| Methyl oleate | 30*** |
| Tap water | q.s. to 1 L |

| | |
|---|---|
| pH adjusted to pH 7.0 with 1N NaOH, sterilized 40 min. at 120°C * Peptonized Milk Nutrient, Sheffield Products, Norwich, NY | |
| ** Proflo, Traders Protein, Memphis, TN | |
| *** The amount of methyl oleate was 30 ml | |

The inoculated production medium is incubated in 250-ml Erlenmeyer flasks at 30°C for 7 days on a shaker orbiting in a two-inch circle at 250 rpm.

### B. Stirred Reactor Fermentation

In order to provide a larger volume of inoculum, 10 ml of incubated first stage medium prepared as described in Section A above, is used to inoculate 400 ml of a second-stage vegetative medium having the same composition as that of the first-stage medium. This second-stage vegetative medium is incubated in a 2 L wide-mouth Erlenmeyer flask for about 48 hours at 32°C on a shaker orbiting in a two-inch circle at 260 rpm. Incubated second-stage vegetative medium (2 L) thus prepared is used to inoculate 115 liters of sterile production medium, prepared as described in Section A above.

The inoculated production medium is allowed to ferment in a 165 L stirred bioreactor for 7 days at a 5 temperature of 30°C. The air-flow and agitator speed in the stirred vessel are computer controlled to maintain a dissolved oxygen level at or above 60% to about 80% of air saturation.

### Example 13 Isolation of Spinosyn Q, Spinosyn R, Spinosyn S and Spinosyn T from Culture NRRL 18823

Fermentation broth (100 L; harvest titer Spinosyn H, 303 pg/ml, Spinosyn Q, 50 pg/ml), prepared as described in the Example above, was refrigerated two days prior to processing. Acetone (100 L) was added to the whole broth after adjusting the pH to 3.0 with 5N HCl. The resulting mixture was filtered through a ceramic filter to give filtrate (170 L) which was held over the weekend under refrigeration. The broth/acetone filtrate was adjusted to pH 13 and refiltered through the ceramic filter prior to loading onto a steel column (10 L; 10 cm x 122 cm) containing HP-20SS resin (Mitsubishi Chemical Industries, Ltd., Japan) at a flow rate of 1 L/minute. The column was fluted at a flow rate of 1 L/minute with a gradient mixed from solvent "A" (0.1% aq. NH₄OAc, adjusted to pH 8.1 with NH₄OH) and solvent "B" (CH₃CN CH₃OH 1:1), collecting 4 L fractions. The pumping system was programmed to generate a gradient from 0 to 50% B in one minute, followed by a gradient from 50 to 100% B in 90 minutes, followed by isocratic delivery of 100% B for an additional 15 minutes. HPLC analysis indicated that fraction 17 (4 L), contained predominantly Spinosyn R with additional more polar materials and a small amount of Spinosyns T and H; fractions 18-22 contained predominantly Spinosyn H with lesser amounts of Spinosyns R and Q and small amounts of Spinosyn S and more polar materials; fractions 23-24 contained Spinosyns H and Q. HPLC analysis of the pools suggested the following total quantities: Spinosyn H. 23.0 g; Spinosyn Q. 3.4 g; Spinosyn R 2.0 g; Spinosyn S. 0.2 g; Spinosyn T. 0.2 g.

### Example 14 Recovery of Spinosyn Q, Spinosyn R, Spinosyn S, and Spinosyn T from a Spinosyn Q-producing strain

Fermentation broth (85 L; harvest titer Spinosyn H, 302 pg/ml, Spinosyn Q, 44 pg/ml), prepared as Q-producing strain, was refrigerated overnight prior to processing. Acetone (90 L) was added to the whole broth after adjusting the pH to 3.0 with 5N HCl. The resulting mixture was filtered through a ceramic filter to give filtrate (176 L) which was held over the weekend under refrigeration. The broth/acetone filtrate was adjusted to pH 13 with 50% NaOH and refiltered through the ceramic filter (140 L filtrate) prior to loading onto a steel column (10 L; 10 cm x 122 cm) containing HP-20SS resin (Mitsubishi Chemical Industries, Ltd., Japan) at a flow rate of 1 L/minute. The column was eluted at a flow rate of 1 L/minute with a gradient mixed from solvent "A" (0.1% NH₄OAC, adjusted to pH 8.1 with NH₄OH) and solvent "B" (CH₃CN - CH₃OH 1:1), collecting 4 L (approx.) fractions. The pumping system was programmed to generate a gradient from 0 to 50% B in one minute, followed by a gradient from 50 to 100% B in 90 minutes, followed by isocratic delivery of 100% B for an additional 10 minutes. HPLC analysis indicated that pool 1 (fractions 16 - 21; 24.5 L), contained Spinosyns H (12.32 g) and Q (0.34 g); pool 2 (fractions 22 - 25; 16L) contained Spinosyns H (4.66 g), Q (2.06 g), R, S and T.

### A. Isolation of pure component Q

Pool 2 was concentrated to dryness, redissolved in dichloromethane (50 ml), and applied to a glass column (5.5 cm x 30 cm) containing silica gel (EM grade 62, 60 - 200 mesh) and equilibrated in dichloromethane.

The column was washed with dichloromethane (3 L), then developed with dichloromethane - methanol (95.5), collecting 250 ml fractions. Fractions 3 through 15 were combined and concentrated to residue, then dissolved in ethanol/water (400 ml) and allowed to stand at room temperature over the weekend. The resulting crystals were washed with cold ethanol/water (1:1) and dried to give 6.1 g of dried crystals containing 68.7% Spinosyn H and 31.2% Spinosyn Q by HPLC analysis. The dried crystalline material was dissolved in tetrahydrofuran/methanol (1:1) and applied to a preparative reverse phase HPLC column (Rainin Dynamax 60A 8 pm C18, 41.4 mm ID x 25 cm with 41.4 mm x 5 cm guard module) in 12 runs. The column was fluted at a flow rate of 50 ml/minute with a gradient mixed from solvent "A" (H₂O - CH₃CN; 30:35:35 containing 0.1% NH₄OAc) and solvent "B" (H₂O - CH₃CN - CH₃OH; 10:45:45 containing 0.1% NH₄OAc). The pumping system was programmed to generate a gradient from 50 to 100% B in 60 minutes. Progress of the separation was monitored with a variable wavelength UV detector tuned to 250 nm.

Peak 1, containing Spinosyn H (99%; 6 L) eluted first, followed by Spinosyn Q. Combined peak 2 (containing Spinosyn H. 20%, component Q. 80%; 8 L) from all (12) runs was concentrated to 500 ml, reapplied to the same column, and eluted under the same mobile phase conditions in 5 runs. Pool 2 (2 L), containing 99% pure Spinosyn Q was desalted by applying it to the same column equilibrated in H₂O - CH₃OH - CH₃CN (20:40:40). The column was eluted with H₂O- CH₃OH - CH₃CN (10:45:45), collecting 10 three-minute fractions. Fractions 2 through 7 were combined, concentrated to residue, and dissolved in hot EtOH (80 ml). An equal volume of H₂O was added and the solution was allowed to cool overnight. The resulting crystals were collected on a filter, washed with cold EtOH - H₂O (1:1), and dried to give 1.5 g pure Spinosyn Q.

### Example 15 Insecticidal Compositions

| A. Aqueous Suspension | |
|---|---|
| Spinosyn Compound | 12.5% |
| TERGITOL TMN-6 (nonionic surfactant) | 1.0% |
| ZIOSYL 200 (silica) | 1.0% |
| AF-100 (silicon based antifoam agent) | 0.2% |
| Xanthan solution (2%) | 10.0% |
| MAKON 10 (10 moles ethyleneoxide nonylphenol surfactant) | 9.0% |
| Tap Water | 66.3% |

| B. Emulsifiable Concentrate | |
|---|---|
| Spinosyn Compound | 12.4% |
| EXXON 200 (naphthalene solvent) | 83.6% |
| TOXIMUL H (nonionic/anionic surfactant blend) | 2.0% |
| TOXIMUL D (nonionic/anionic surfactant (blend) | 2.0% |

### Example 16 Formulations of the Invention

| A. Feed Premix | |
|---|---|
| Spinosyn Compound | 10% |
| Rice hulls | 85 |
| Light mineral oil | 5 |

| B. Feed Premix | |
|---|---|
| Spinosyn Compound | 25% |
| Alfalfa meal | 60 |
| Powdered clay | 5 |
| Molasses | 10 |

| C. Suspension | |
|---|---|
| Spinosyn Compound | 30% |
| Naphthalenesulfonate salt | 5 |
| Nonionic surfactant | 5 |
| Fumed silica | 1 |
| Water | 59 |

| D. Drip-On Solution | |
|---|---|
| Spinosyn Compound | 20% |
| Nonionic surfactant | 0.8 |
| Propylene glycol | 15 |
| Water | 64.2 |

| E. Drip-On Suspension | |
|---|---|
| Spinosyn Compound | 10 |
| Nonionic surfactant | 1 |
| Light mineral oil | 89 |

| F. Injectable Solution | |
|---|---|
| Spinosyn Compound | 15% |
| Propylene glycol | 85 |

| G. Injectable Suspension | |
|---|---|
| Spinosyn Compound | 25% |
| Propylene glycol | 15 |
| Water | 60 |

| H. Injectable Suspension | |
|---|---|
| Spinosyn Compound | 30% |
| Polyvinylpyrrolidone | 2 |
| Water | 68 |

### Example 17 Synthetic Modifications

### Part A Rhamnose-Modified Derivatives

### Example A1 - (5,6-Dihydro Derivative of Compound 9-O-(2,3,4-Tri-O-ethyl-α-L-rhamnosyl) Spinosyn A 9-Psa

Compound 9-*O*-(2,3,4-Tri-*O*-ethyl-α-L-rhamnosyl) Spinosyn A 9-Psa (106 mg, 0.137 mmol) was dissolved in 5 mL toluene. To this solution was added tris-triphenylphoshine Rhodium(I)chloride (11.9mg, 0.0128 mmol). Evacuated the flask head space and introduced nitrogen three times. Evacuated and introduced hydrogen three times. Maintained flask under hydrogen with balloon and heated reaction to 110-120°C. After 3.5 hr. cooled flask to RT and evacuated hydrogen and replaced with nitrogen. Evaporated off tolune solvent and replaced with 20 mL ether. Extracted ether solution with 3 x 10 mL 1N HCl. Combined acid extracts and neutralized with 10 ml 4N NaOH. Extracted neutralized suspension with 3 x 10 mL ether, combined ether extracts, washed with 20 mL brine, dried over K₂CO₃, and evaporated *in vacuo.* Gave compound 5,6-dihydro derivative of compound 9-*O*-(2,3,4-tri-*O*-ethyl-α-L-rhamnosyl) Spinosyn A 9-Psa, 39.4mg, 36.8% partial ¹H-NMR δ 6.84 (1H, bs), 1.01 (1H, m), 0.68 (1H, m).

### Example A2 - (5S,6R)-Epoxy-2'-O-(p-trifluoromethyl)benzoyl Spinosyn Q

Compound (5*S*,6*R*)-epoxy-Spinosyn Q (406 mg, 0.543 mmol) was dissolved in dry CH₂Cl₂ (10 ml). DMAP (369 mg, 3.0 mmol) was added, followed by (p-trifluoromethyl)benzoyl chloride (623 mg, 445 µl, 2.98 mmol). After stirring for 14 hrs. at RT, triethylamine (2 ml) was added and stirring was continued for 30 min. The solution was diluted with EtOAc (100 ml) and toluene (30 ml), then washed successively with brine, 5% aq. NaHCO₃ (2x) and water (1x) and dried over K₂CO₃. The organic layer was concentrated *in vacuo*. The residue was purified over a flash SiO₂ column (80 g / EtOAc) to furnish compound (5*S*,6*R*)-Epoxy-2'-*O*-(p-trifluoromethyl)benzoyl Spinosyn Q; 391 mg, 78% IR ν 1728, 1664, 1324, 1272 cm⁻¹; ¹H-NMR (CDCl₃) δ 8.12 (1H, bd: 8.2 Hz), 7.67 (1H, bd: 8.2 Hz), 6.54 (1H, bs) 5.42 (1H, dd: 3.2, 1.8 Hz) 1.31 (3H, CH₃, bs).

### Example A3 - 5,6-Dihydro-3'-O-ethyl Spinosyn J

Compound 5,6-dihydro-Spinosyn J (399 mg, 0.554 mmol) was dissolved in CH₂Cl₂ (5.0 ml). K₂CO₃ (1.10 g) was added. The reaction flask was immersed in a water bath (10°C) and 15% aqueous NaOH (10 ml) was added with vigorous stirring, followed by tetrabutylammonium hydrogen sulfate (0.90 g). 1-Iodoethane (5.0 ml) was added and the reaction mixture was vigorously stirred at RT under N₂ for 72 hrs. CH₂Cl₂ (100 ml) and water (50 ml) were added. The phases were separated. The organic layer was washed with water (2 x), dried over K₂CO₃ and concentrated *in vacuo.* The residue was purified over a flash SiO₂ column (100 g / EtOAc). Concentration of pure fractions furnished compound 5,6-dihydro-3'-*O*-ethyl Spinosyn J; 217 mg, 52% ¹H-NMR (CDCl₃) δ 6.72 (1H, bs), 3.57 (1H, m), 3.49 (2H, m), 2.13 (6H, 2x CH₃, s), 1.14 (3H, CH₃, t: 7.3 Hz) ppm;

### Example A4 - 5,6-Dihydro-3'-O-n-propyl Spinosyn J

Compound 5,6-dihydro-Spinosyn J (86 mg, 0.119 mmol) was dissolved in CH₂Cl₂ (3.0 ml). K₂CO₃ (0.68 g) was added. The reaction flask was immersed in a water bath (10°C) and 20% aqueous NaOH (6 ml) was added with vigorous stirring, followed by tetrabutylammonium hydrogen sulfate (0.81 g). 1-Iodopropane (1.20 ml) was added and the reaction mixture was vigorously stirred at RT under N₂ for 20 hrs. CH₂Cl₂ (100 ml) and water (50 ml) were added. The phases were separated. The organic layer was washed with water (2 x), dried over K₂CO₃ and concentrated *in vacuo.* The residue was purified over a flash SiO₂ column (25 g / EtOAc). Concentration of pure fractions furnished compound 5,6-dihydro-3'-*O*-n-propyl Spinosyn J; 55.0 mg, 60% ¹H-NMR (CDCl₃) δ 6.78 (1H, bs), 3.55 (3H, m), 2.16 (6H, 2x CH₃, s), 0.89 (3H, CH₃, t: 7.5 Hz) ppm; ¹³C-NMR (CDCl₃) δ 72.5 (O-CH₂-), 23.9 (CH₂) and 11.2 (CH₃) ppm.

### Example A5 - 5,6-Dihydro-3'-O-n-butyl Spinosyn J

Compound 5,6-dihydro-Spinosyn J (93 mg, 0.129 mmol) was dissolved in CH₂Cl₂ (3.0 ml). K₂CO₃ (0.70 g) was added. The reaction flask was immersed in a water bath (10°C) and 20% aqueous NaOH (10 ml) was added with vigorous stirring, followed by tetrabutylammonium hydrogen sulfate (0.66 g). 1-Iodobutane (2.0 ml) was added and the reaction mixture was vigorously stirred at RT under N₂ for 20 hrs. CH₂Cl₂ (100 ml) and water (50 ml) were added. The phases were separated. The organic layer was washed with water (2 x), dried over K₂CO₃ and concentrated *in vacuo.* The residue was purified over a flash SiO₂ column (25 g / EtOAc). Concentration of pure fractions furnished compound (5,6-dihydro-3'-*O*-n-butyl Spinosyn J; 63 mg, 63% ¹H-NMR (CDCl₃) δ 6.78 (1H, bs), 3.57 (3H, m), 2.16 (6H, 2x CH₃, s), 0.87 (3H, CH₃, t: 7.2 Hz) ppm; Elemental Analysis: for C₄₄H₇₃NO₁₀ calc. C 68.10, H 9.48, N 1.80; found C 68.23, H 9.65, N 1.83.

### Example A6 - 5,6-Dihydro-3'-O-n-propyl Spinosyn J Hemiglutarate salt

Compound 5'6-dihydro-3'-*O*-n-propyl Spinosyn J (88.3mg, 0.115mmol) was dissolved in 3mL acetone and a solution of glutaric acid (7.5mg, 0.056mmol) in 2mL acetone was added. Stirred solution at RT overnight. Evaporation of the solvent under vacuum gave compound 5,6-dihydro-3'-*O*-propyl-Spinosyn J hemiglutarate salt, 86.1mg. Anal. calcd. for C₄₃H₇₁NO₁₀ • 1/2(C₅H₈O₄) C 65.99, H 9.12, N 1.69; Found C 65.30, H 10.00, N 1.77; mp. 74-84°C.

### Example A7 - 5,6-Dihydro-2'-O-ethyl Spinosyn H

Compound 5,6-dihydro Spinosyn H (462 mg, 0.64 mmol) was dissolved in CH₂Cl₂ (6 ml). Potassium carbonate powder (1.0 g) was added. The reaction flask was placed in a water cooling bath (ca. 10°C). With vigorous stirring, 10% aq. NaOH solution (15 ml) was added, followed by solid NBu₄HSO₄ (1.2 g). Ethyl iodide (4.0 ml) was then added. The reaction mixture was vigorously stirred at RT under nitrogen for 46 hrs. Methylene chloride (100 ml) and H₂O (100 ml) were added. After extraction phases were separated. The organic phase was dried over anh. K₂CO₃ and concentrated *in vacuo.* The residue was purified over a flash SiO₂ column (80 g / EtOAc) to give the pure compound (5,6-dihydro-2'-*O*-ethyl Spinosyn H; 336 mg, 70%: ¹H-NMR (CDCl₃) δ 6.69 (1H, bs), 4.59 (1H, bs), 3.37 (3H, CH₃, s), 3.30 (3H, CH₃, s), 2.06 (6H, 2x CH₃, s) ppm.

### Example A8 - 5,6-Dihydro-2'-O-n-propyl Spinosyn H

Compound 5,6-dihydro Spinosyn H (488 mg, 0.68 mmol) was dissolved in CH₂Cl₂ (8 ml). Potassium carbonate powder (1.1 g) was added. The reaction flask was placed in a water cooling bath (ca. 10°C). With vigorous stirring, 10% aq. NaOH solution (20 ml) was added, followed by solid NBu₄HSO₄ (0.95 g). n-Propyl iodide (5.0 ml) was then added. The reaction mixture was vigorously stirred at RT under nitrogen for 48 hrs. Methylene chloride (100 ml) and H₂O (100 ml) were added. After extraction phases were separated. The organic phase was dried over anh. K₂CO₃ and concentrated in vacuo. The residue was purified over a flash SiO₂ column (80 g / EtOAc) to give pure compound (5,6-dihydro-2'-*O*-n-propyl Spinosyn H; 355 mg, 69%: ¹H-NMR (CDCl₃) δ 6.72 (1H, bs), 4.63 (1H, d: 1.3 Hz), 3.41 (3H, CH₃, s), 3.34 (3h, CH₃, s), 2.10 (6H, 2x CH₃, s), 0.67 (3H, CH₃, t: 7.4 Hz) ppm.

### Example A9 - (5R,6S)-5,6-Epoxy-3'-O-n-propyl Spinosyn L and (5S,6R)-5,6-epoxy-3'-O-n-propyl Spinosyn L

Compound 3'-*O*-n-propyl Spinosyn L (1.07 g, 1.38 mmol) was dissolved in methylene chloride (25 mL) under a nitrogen atmosphere and *m*-CPBA (50%, 1.88 g, 5.44 mmol) was added and the reaction stirred for 24 h. The reaction mixture was diluted with ethyl acetate (50 mL) and extracted with 10% aqueous sodium sulfite (3 x 25 mL) followed by saturated aqueous sodium bicarbonate (3 x 25 mL). The organic layer was washed with brine (25 mL) and dried over anhydrous potassium carbonate to give a yellow solid (0.80 g). The crude product was purified by reverse-phase HPLC (methanol:0.1% aq ammonium hydroxide, 90:10) to give (5*R*,6*S*)-5,6-epoxy-3'-*O*-*n*-propyl Spinosyn L (26.6 mg, 2.4%): partial ¹H NMR δ 6.68 (bs, 1H), 4.78 (s, 1H), 4.67 (m, 1H), 4.37 (d, 1H), 4.21 (q, 1H), 3.58 (m, 1H); and (5*S*,6*R*)-5,6-epoxy-3'-*O-n*-propyl Spinosyn L (114 mg, 10.4%): partial ¹H NMR d 6.54 (bs, 1H), 4.78 (s, 1H), 4.63 (m, 1H), 4.38 (d, 1H),4.20 (m, 1H), 3.60 (m, 1H).

### Example A10 - (5R,6S)-3'-Deoxy-5,6-epoxy-3'-methylene Spinosyn J and (5S,6R)-3'-Deoxy-5,6-epoxy-3'-methylene Spinosyn J

Compound 3'-deoxy-3'-methylene Spinosyn J (640 mg, 0.896 mmol) was dissolved in methylene chloride (100 mL). The solution was cooled to 0°C and *m*-CPBA (1.06 g, ca. 3 mmol) was added in a few portions. Stirring at 0°C was continued for 1 h. 10% Aqueous NaHSO₃ solution (100 ml) was added, followed by methylene chloride (150 mL). After extraction, phases were separated and the organic phase was worked up in the usual manner. The crude product was purified over a silica gel column and then separated by HPLC (88:12, MeOH/H₂O) to give a) compound 5*R*,6*S*-3'-deoxy-5,6-epoxy-3'-methylene Spinosyn J (20 mg, 3%) white solid: ¹H NMR δ 6.64 (s, 1 H), 5.23 (t, *J* = 1.7, 1 H), 5.07 (d, *J* = 1.5, 1 H), 4.69 (d, J = 1.6, 1 H), 3.41 (s, 3 H), 3.26 (s, 3H); and compound 5*S*,6*R*-3'-deoxy-5,6-epoxy-3'-methylene Spinosyn J (170 mg, 26%) white solid: ¹H NMR δ 6.47 (s, 1 H), 5.18 (s, 1 H), 5.02 (s, 1 H), 4.64 (s, 1 H), 3.36 (s, 3 H), 3.20 (s, 3H).

### Example A11 - A mixture of compound 5,6-dihydro-3'-O-n-propyl Spinosyn J (60% - 85%) and compound 3'-O-n-propyl Spinosyn L (40% - 15%)

A mixture of compound 3'-*O*-n-propyl Spinosyn J (60% - 85%) and compound 3'-*O*-n-propyl Spinosyn L (40% - 15%) (8.35 g) was dissolved in ethanol (250 mL). Cyclohexene (60 mL) was added. The mixture was cooled under nitrogen to 0°C and 10% Pd/C catalyst (7.0 g) was added. The reaction mixture was gently heated to reflux for 3 h. It was then cooled to 0°C and pyridine (2 mL) was added, the catalyst was filtered, the filtrate was concentrated, then dissolved in ethyl ether (300 mL). The usual work-up afforded a mixture of compound 5,6-dihydro-3'-*O*-n-propyl Spinosyn J (60% - 85%) and compound 3'-*O*-n-propyl Spinosyn L (40% - 15%) (7.40 g) as a white solid: ¹H NMR δ 6.73 (s, ca. 0.8 H), 6.63 (s, ca. 0.2 H), 5.37 (s, ca. 0.2 H), 0.84 (t, *J* = 7.4, 3 H).

### Example A12 - A mixture of compound 5,6-dihydro-3'-O-n-propyl Spinosyn J (60% - 85%) and compound 3'-O-n-propyl Spinosyn L (40% - 15%) citrate salts

A mixture of compound 5,6-dihydro-3'-*O*-n-propyl Spinosyn J (60% - 85%) and compound 3'-*O*-n-propyl Spinosyn L (40% - 15%) (205 mg, 0.269 mmol) was dissolved in acteone (8 mL) and a solution of citric acid monohydrate (56.7 mg, 0.269 mmol) in acetone (3 mL) was added and the solution stirred for 2 h. The solvent was removed by rotary evaporation to give a mixture of compound 5,6-dihydro-3'-*O*-n-propyl Spinosyn J (60% - 85%) and compound 3'-*O*-n-propyl Spinosyn L (40% - 15%) citrate salts (211 mg).

### Example A13 - 5,6-Dihydro-3'-O-isopropyl Spinosyn J

3'-*O*-Isopropenyl Spinosyn J (280 mg, 0369 mmol) was reacted during 20 min with triethylsilane (110 mg, 0.94 mmol) and TFA (340 mg, 3.0 mmol) following the procedure described for compound 3'-O-isopropyl Spinosyn J (Example A101). After HPLC (90:10, MeOH/H₂O) separation of the crude product, this gave 5,6-dihydro-3'-O-isopropyl Spinosyn J (47 mg, 17%) as a white solid: ESI MS m/z 763 (M + 1).

### Example A14 - 5,6-Dihydro-3'-epi-N-formyl-3'-propyl Spinosyn J and 5,6-dihydro-3'-epi-3'-propyl Spinosyn J

Compound 3'-allyl-3'-epi Spinosyn J (218 mg, 0.287 mmol) was dissolved in ethanol (30 mL). The solution was cooled to 0°C under nitrogen. 10% Pd/C (911 mg) was added, followed by cyclohexene (10 mL, not purified before use). The mixture was heated to reflux under nitrogen for 3 h. Triethylamine (1 mL) was added. After work-up and chromatography on silica gel, the crude product was separated on HPLC (94:6, MeOH/H₂O). This gave a) 5,6-dihydro-3'-epi-*N*-formyl-3'-propyl Spinosyn *J* (28 mg, 12%) as a yellowish glassy solid: ¹H NMR δ 8.04 (s, 1H), 6.81 (s, 1 H), 3.47 (s, 3 H), 3.39 (s, 3 H), 2.71 (s, 1.5 H), 2.19 (s, ca. ca. 1.5 H). ESI MS m/z 766 (M + 1) and b) 5,6-dihydro-3'-epi-3'-propyl Spinosyn J (94 mg, 43%) as a white solid: ¹H NMR δ 6.75 (s, 1 H), 3.42 (s, 3 H), 3.34 (s, 3 H), 2.13 (s, 6 H).

### Example A15 - 5,6-Dihydro-3'-O-vinyl Spinosyn J

5,6-Dihydro-Spinosyn J (1.85 g, 2.57 mmol) was dissolved in butyl vinyl ether (50 mL, an excess). Mercuric acetate (4.2 g, an excess) was added. The reaction mixture was heated to reflux for 4 h. Solid potassium carbonate (10 g) was then added. The usual work-up and chromatography on silica gel (ethyl acetate) afforded 5,6-dihydro-3'-*O*-vinyl spinosyn J (1.02 g, 53%) as a white solid: ¹H NMR δ 6.74 (s, 1 H), 6.31 (dd, *J*_{*1*} = 16.9, *J*_{*2*} = 6.5, 1 H), 4.30 (m, 2 H), 3.90 (m, 2 H), 3.38 (s, 3 H), 3.36 (s, 3 H).

### Example A16 - (14S)-5,6,13,14-Tetrahydro-3'-O-n-propyl Spinosyn J

5,6-Dihydro-3'-*O*-*n*-propyl Spinosyn J (1.88 g, 2.47 mmol) was reacted with lithium tri-*tert*-butoxyaluminohydride (97% powder, 2.40 g, 9.16 mmol) in Et₂O (100 mL) during 3.5 hrs., according to the procedure given for (14*S*)-13,14-dihydro-3'-*O*-ethyl Spinosyn M. After work-up and chromatography on silica gel (ethyl acetate) this afforded (14*S*)-5,6,13,14-tetrahydro-3'-*O*-*n*-propyl Spinosyn J (1.440 g, 76%) as a white solid: ¹H NMR δ 4.66 (d, *J* = 1.4, 1 H), 4.64 (m, 1 H), 3.41 (s, 3 H), 3.34 (s, 3 H), 2.09 (s, 3 H), 1.00 (d, *J* = 6.6, 3 H).

### Example A17 - 5,6-Dihydro-3'-methoxymethyl-Spinosyn J

3'-methoxymethyl-Spinosyn J (243 mg, 0.319 mmol) and palladium on charcoal (10%, 200 mg) were placed in a 25-mL round-bottomed flask. Ethanol (7.25 mL) and cyclohexene (1.75 mL, 17.3 mmol) were added and the resulting solution was heated at reflux temperature for 3 hrs. The mixture was successively filtered through Celite and a PTFE filter (Gelman, Acrodisc 13 CR, 0.2 mm). The mixture was concentrated under reduced pressure and the residue chromatographed on a reversed-phase column (Kromasil' C18, Silica ODS, 100Å, 10m, spherical, 25 cm X 20 mm) using 85% MeOH and 15% water (containing 0.1% v/v conc. aqueous NH₄OH) to yield an amorphous white solid (68 mg, 28%) MS m/z 764.7 (calc. for M+H 764.5).

### Example A18 - 4"-N-Desmethyl-4"-N-(2-fluoroethyl)-5,6-dihydro-3'-O-propyl Spinosyn J

This compound was prepared according to the method in Example CZ from 1-bromo-2-fluoroethane (0.20 mL, 0.34 g, 2.7 mmol), NaI (0.04 g, 0.3 mmol), (*i*-Pr)₂NEt (0.40 mL, 0.30 g, 2.3 mmol), 4"-*N*-desmethyl-5,6-dihydro-3'-*O*-propyl Spinosyn J (0.300 g, 0.401 mmol), and DMF (2 mL). MPLC (25:75 to 50:50 EtOAc/hexane) gave 0.235 g (74%) of 4"-*N*-desmethyl-4"-*N*-(2-fluoroethyl)-5,6-dihydro-3'-*O*-propyl Spinosyn J as a white powder.

### Example A19 - 4"-N-Desmethyl-5,6-dihydro-3'-O-propyl Spinosyn J

F-TEDA (0.98 g, 2.8 mmol) was added to a solution of 5,6-dihydro-3'-*O*-n-propyl Spinosyn J (0.94 g, 1.23 mmol) in CH₃CN (10 mL). After 10 min, the mixture was partitioned between EtOAc and 0.1 M HCl. The organic layer was washed sequentially with NaHCO₃ and brine. The organic layer was dried (MgSO₄), filtered, and evaporated. MPLC (SiO₂, 5:95 to 10:90 MeOH/CH₂Cl₂) gave 0.64 g (70%) of 4"-*N*-desmethyl-5,6-dihydro-3'-*O*-propyl Spinosyn J as a white powder (5,6-dihydro-3'-*O*-n-propyl Spinosyn J, 0.25 g (27%) was also recovered).

### Part C Modifications within the Forosamine Substituent

### Example C1 - 5,6-Dihydro-4"-N-(2-methyl-1-propyl) Spinosyn B

This compound was prepared according to the method in Example C81 from 1-iodo-2-methylpropane (0.10 mL, 0.16 g, 0.87 mmol), (*i*-Pr)₂NEt (0.23 mL, 0.17 g, 1.3 mmol), 5,6-dihydro Spinosyn B (0.31 g, 0.43 mmol), and DMF (2 mL). MPLC (SiO₂, 40:60 EtOAc/hexane) gave 0.16 g (48%) of 5,6-dihydro-4"-*N*-(2-methyl-1-propyl) Spinosyn B as a white powder. Anal. Calcd for C₄₄H₇₃NO₁₀: C, 68.10; H, 9.48; N, 1.80. Found: C, 68.10; H, 9.37; N, 1.87.

### Example C2 - Compound 3",4"-dehydro-4''-desamino-5,6-dihydro Spinosyn A

5,6-Dihydro Spinosyn A N-oxide (1.26 g, 1.68 mmol) was dissolved in dry THF (10 ml). The solution was cooled to -78°C and pyridine (5 ml) was added. Upon stirring, trifluoroacetic anhydride (1.8 ml, 2.67 g, 12.7 mmol) was added. After 16 hrs. Et₂O (120 ml) was added, the mixture was extracted with diluted brine (2x), then with 10% aqueous KHCO₃ (2x), dried over K₂CO₃ and concentrated *in vacuo.* The residue was separated over a flash SiO₂ column (Et₂O) to give 3'',4''-dehydro-4''-desamino-5,6-dihydro Spinosyn A (122 mg, 10.5%) as a white solid: ¹H NMR d 6.82 (bs, 1H), 5.61 (m, 1H), 5.53 (bd, *J* = 10 Hz, 1 H), 4.66 (q, *J* = 6 Hz, 1 H), 1.23 (d, *J* = 6.3 Hz, 3 H), 1.19 (d, *J* = 6.6 Hz, 3H), 1.15 (d, *J* = 7.0 Hz, 3H).

### Example C3 - Compound 5,6-dihydro-N-formyl Spinosyn B and compound 4''-desamino-5,6-dihydro-4"-keto Spinosyn C

5,6-Dihydro spinosyn A (1.43 g, 1.95 mmol) was dissolved in methylene chloride (25 ml) and pyridine (6.0 ml) was added. The flask was cooled at +10°C. Bromine (3.0 ml) was added with vigorous stirring under N₂. After 30 min. water (2.0 ml) was added and stirring was continued for 1 hr. Et₂O (200 ml) was added, the mixture was successively extracted with: water, 10% aqueous NaHSO₃, water, and 10% aqueous KHCO₃, dried over anh. K₂CO₃, filtered and concentrated. The crude product was divided into two equal parts. One part was purified by flash chromatography (SiO₂/Et₂O) and separated by repeated RP (C-18) HPLC (92:8, MeOH/H₂O) to give a) 5,6-dihydro-N-formyl spinosyn B (230 mg, 31%) as a white solid: ¹H NMR d 7.96 (bs) and 7.93 (bs, total 1 H), 6.74 (bs, 1 H), 2.64 (s, 3 H), 1.14 (d, *J =* 5.8 Hz, 3 H), 1.05 (d, *J* = 6.9 Hz, 3 H), 1.02 (d, *J* = 6.3 Hz, 3 H) and b) fractions enriched to ca. 50 % in 4"-desamino-5,6-dihydro-4''-keto spinosyn C (168 mg, ca. 12%). The above procedure was repeated on a 5-times larger scale, which led to pure 4''-desamino-5,6-dihydro-4"-keto spinosyn C (212 mg, 3.1%), a white solid: ¹H NMR d 6.79 (bs, 1 H), 4.90 (dd, *J* = 8.2 Hz, 2.5 Hz), 3.95 (q, *J* = 6.7 Hz); ¹³C NMR d 208.7, 203.2, 172.6, 149.6, 145.1, 100.9, 95.4, 17.6 (CH₃), 16.1 (CH₃), 15.3 (CH₃), 9.2 (CH₃).

### Example C4 - Compound 4''-desamino-5,6-dihydro-4"(S)-hydroxy Spinosyn C

4"-Desamino-5,6-dihydro-4''-keto Spinosyn C (192 mg, 0.273 mmol) was dissolved in dry Et₂O (50 ml). The solution was cooled to 0°C and lithium tri-*t*-butoxyaluminohydride (97%, 145 mg, 0.55 mmol) was added in one portion. Stirring at the same temperature was continued for 15 min. The reaction mixture was then carefully quenched with brine. Ethyl ether (100 ml) was added and the mixture was extracted with aqueous 2N NaOH (2x), then with 10% aq. KHCO₃, dried over K₂CO₃ and concentrated. The crude product was purified over a flash SiO₂ column (EtOAc) and separated by RP (C-18) HPLC (92:8, MeOH/H₂O) to give 4''-desamino-5,6-dihydro-4''(*S*)-hydroxy spinosyn C (91 mg, 47%) as a white solid: ¹H NMR d 6.81 (bs, 1 H), 4.43 (bd, *J* = 7.4 Hz, 1 H), 3.57 (m, 1 H), 1.21 (d, *J* = 6.1 Hz, 6H), 1.12 (d, *J* = 6.9 Hz, 3H).

### Example C5 - Compound 4''-desamino-5,6-dihydro-4"(S)-methoxy Spinosyn C

4''-Desamino-5,6-dihydro-4"(*S*)-hydroxy Spinosyn C (66 mg, 0.093 mmol) was dissolved in methylene chloride (1.5 ml). Iodomethane (2.0 ml) was added, followed by 40% aqueous NaOH solution (3.0 ml) and tetrabutylphosphonium bromide (320 mg). The reaction mixture was vigorously stirred at RT / N₂ and powdered KOH (0.60 g) was added. Stirring was continued for 2 hrs. Water (10 ml) and Et₂O (100 ml) were added, the mixture was washed with water, then with 10% aq. KHCO₃ solution (2x). The organic phase was dried over anh. K₂CO₃, filtered and concentrated *in vacuo*. The residue was purified over a flash SiO₂ column (CH₂Cl₂-Et₂O, 8:2) to afford 4''-desamino-5,6-dihydro-4"(*S*)-methoxy Spinosyn C (40.0 mg, 59%) as a white solid: ¹H NMR δ 6.82 (bs, 1 H), 4.43 (dd, *J* = 9.4 Hz, 1.9 Hz), 3.52 (s, 3 H), 3.46 (s, 3 H), 3.45 (s, 3 H), 3.32 (s, 3 H), 1.25 (d, *J =* 6.3 Hz, 3 H), 1.20 (d, *J* = 6.1, 3 H), 1.14 (d, *J* = 6.8 Hz, 3 H), 0.77 (t, *J* = 7.4 Hz, 3 H).

### Part D Modification of the 13' and 14' position on the tricyclic portion of the compound in Formula 1

### Example D1 (14S)-5,6,13,14-Tetrahydro Spinosyn A

The reaction was run as described in Example D5 starting with Spinosyn A (106.8 mg, 0.15 mmol). This gave (14*S*)-5,6,13,14-tetrahydro Spinosyn A(85.5 mg ; 80% yield) as a colorless, slightly sticky solid, FDMS, m/e (relative intensity) 736 (M⁺, 100).

### Example D2 - (14R)-5,6,13,14-Tetrahydro Spinosyn A

To a solution of (14*R*)-13,14-dihydro Spinosyn A (210.3 mg, 0.29 mmol) in benzene (9 ml), tris(triphenylphosphine)rhodium(I) chloride (47.2 mg, 0.05 mmol) was added and the reaction mixture was placed under one atmosphere of hydrogen . The mixture stirred at room temperature for 4 days, then the solvent was evaporated at room temperature under reduced pressure. The product was purified by chromatography on silica, eluting with 5% methanol in dichloromethane. Product was further purified by chromatography on silica, eluting with 70% ethyl acetate in dichloromethane. This gave (14R)-5,6,13,14-tetrahydro Spinosyn A (93.3 mg ; 44% yield) as as colorless glass, FDMS, m/e (relative intensity) 735 (M⁺, 100).

### Example D3 - (14S)-5,6,13,14-Tetrahydro-3'-deoxy Spinosyn J

To a solution of 3'-deoxy Spinosyn J (108.8 mg, 0.16 mmol) in ethyl acetate (50 ml), 5% paladium on aluminum oxide (100 mg) was added. The reaction mixture was placed under 60 psi of hydrogen and stirred at room temperature for 8 hour. After filtration of the catalyst, the solvent was evaporated at room temperature under reduced pressure. The residue was purified by chromatography on silica, eluting with 2.5% ethanol in ethyl acetate. This gave (14*S*)-5,6,13,14-tetrahydro-3'-deoxy Spinosyn J (50.5 mg ; 45% yield) as a white solid, FDMS, m/e (relative intensity) 707 (65), 706 (MH⁺, 100).

### Part E Modification of the Pseudoaglycone at the C-17 position of the tricyclic portion of the Compound Formula 1

### Example E1 - (5R,6R)-5-(2-Hydroxyethoxy)-6-bromo Spinosyn A 17-Psa

To a suspension of Spinosyn A (154.1 mg, 0.21 mmol) in ethylene glycol (5 ml), *N*-bromosuccinimide (79.8 mg, 0.45 mmol) was added. The reaction mixture was heated to 80°C for 1.5 hour and then cooled to room temperature. The mixture was diluted with dichloromethane and washed with water. The dichloromethane was then washed with brine, dried with K₂CO₃, and evaporated at room temperature. The products were seperated by chromatography on silica, eluting with 70% ethyl acetate in hexane and then 5% methanol in dichloromethane. This gave compound a) Spinosyn A 17-Psa (41.7 mg ; 34% yield) as a colorless glass. (5*R*,6*R*)-5-(2-hydroxyethoxy)-6-bromo Spinosyn A 17-Psa was further purified by chrcmatography on silica, eluting with 70% ethyl acetate in hexane. This gave pure compound b) (5R,6R)-5-(2-hydroxyethoxy)-6-bromo Spinosyn A 17-Psa (50.2 mg ; 33% yield) as a colorless glass, FDMS, m/e (relative intensity 734 (65), 732 (MH⁺, 60), 189 (100).

### Example E2 - (5S,6R)-5,6-Epoxy-17-O-trimethylsilyl Spinosyn A 17-Psa

To a solution of (5*S*,6*R*)-5,6-epoxy Spinosyn A 17-Psa (503.7 mg, 0.83 mmol) in ether (20 ml), chlorotrimethyl silane (211 ml, 1.66 mmol) and diisopropylethylamine (289 ml, 1.66 mmol) were added. The reaction mixture was stirred at room temperature for 23 hours, then additional chlorotrimethyl silane (105 ml, 0.83 mmol) and diisopropylethylamine (144 ml, 0.83 mmol) were added. The reaction mixture continued to stir at room temperature another 18 hours. The ether was then decanted and the residue precipitate was triturated with fresh ether. The ether was combined, washed with aqueous saturated NaHCO₃, dried with K₂CO₃ and evaporated at room temperature under reduced pressure. The crude product was purified by chromatography on silica, eluting with 50% ethyl acetate in hexane. This gave (5*S*,6*R*)-5,6-epoxy-17-*O*-trimethylsilyl Spinosyn A 17-Psa (521.1 mg ; 92% yield) as a colorless glass, FDMS, m/e (relative intensity) 679 (75), 678 (M⁺, 100), 190 (30), 100 (35).

### Example E3 - (5S,6R)-5,6-Epoxy Spinosyn A 17-Psa

To a solution of Spinosyn A 17-Psa (1.0 gm, 1.71 mmol) in dichloromethane (45 ml), m-chloroperoxybenzoic acid (591.7 mg, 3.34 mmol) was added. The reaction mixture stirred at room temperature for 23 hour. The mixture was then diluted with dichloromethane and washed with aqueous saturated NaHCO₃, dried with K₂CO₃, and evaporated at room temperature under reduced pressure. The product was purified by chromatography on silica, eluting with 15% acetone in dichloromethane. This gave (5*S*,6*R*)-5,6-epoxy Spinosyn A 17-Psa (1.04 gm ; ~100% yield) as a colorless glass, FDMS, m/e (relative intensity) 607 (M⁺, 70), 190 (100), 101 (99).

### Part F Modification of the C-5 and C-6 positions of the tricyclic portion of compound Spinosyn A

### Example F1 - (5R,6R)-5-(2-Hydroxyethoxy)-6-bromo Spinosyn A

To a suspension of Spinosyn A (1.0 gm, 1.37 mmol) and N-bromosuccinimide (289.5 mg, 1.63 mmol) in ethylene glycol (20 ml), 5 N HCl (295 µl, 1.47 mmol) was added. The reaction mixture turned yellow and was stirred at room temperature for 1.5 hour, during which time the color faded to clear. The mixture was poured into saturated aqueous NaHCO₃, and extracted with ether. The ether was dried with MgSO₄ and evaporated at room temperature under reduced pressure. This gave crude (5*R*,6*R*)-5-(2-hydroxyethoxy)-6-bromo Spinosyn A (896.8 mg, 75% yield). The compound could be purified by chromatography on silica, eluting with 5% methanol in dichloromethane, giving (5*R*,6*R*)-5-(2-hydroxyethoxy)-6-bromo Spinosyn A as a white glass, FDMS, m/e (relative intensity) 873 (100), 871 (M⁺, 80).

### Example F2 - (5S,6R)-5,6-Dihydroxy-5,6-dihydro Spinosyn A

To a suspension of Spinosyn A (487.8 mg, 0.67 mmol) and trimethylamine-*N*-oxide dihydrate (108 mg, 0.97 mmol), in t-butylalcohol (3 ml) with water (0.5 ml), pyridine (60 ml, 0.74 mmol) was added followed by osmium tetroxide (0.1 M; 40 ml, 0.004 mmol). The reaction mixture was heated to reflux under a nitrogen atmosphere for 19 hours. The dark colored mixture was then cool to room temperature and poured into aqueous 20% NaHSO₃, and extracted with ether. The ether was washed with brine, dried with MgSO₄, and evaporated at room temperature under reduced pressure. The product was purified by chromatography on silica, eluting with 7% methanol in dichloromethane. This gave (5*S*,6*R*)-5,6-dihydroxy-5,6-dihrdro Spinosyn A (282.1 mg; 55% yield) as a beige solid, FDMS, m/e (relative intensity) 766 (M⁺, 60), 765 (100).

### Example F3 - (5S,6R)-Spinosyn A carbonate

To a solution of (5*S*,6*R*)-5,6-dihydroxy Spinosyn A (85 mg, 0.11 mmol) and ethylene carbonate (109.2 mg, 1.2 mmol) in benzene (3 ml), anhydrous K₂CO₃ (40.2 mg, 0.29 mmol) was added and the mixture was heated to relfux. After 2.5 hours the mixture was cooled to room temperature and diluted with dichloromethane. The dichloromethane was washed with water, brine, dried with K₂CO₃ and evaporated at room temperature under reduced pressure. The product was purified by chromatography on silica, eluting with 7% methanol in dichloromethane. This gave (5*S*,6*R*)-Spinosyn A carbonate (65.1 mg ; 75% yield) as a white solid, FDMS, m/e (relative intensity) 792 (M⁺, 70), 791 (100).

### Example F4 - (5S,6S)-5-Acetoxy-6-bromo Spinosyn A and (5R,6R)-5-Acetoxy-6-bromo-5,6-dihydro Spinosyn A

To a solution of Spinosyn A (511.7 mg, 0.7 mmol) in glacial acetic acid (5 ml), N-bromosuccinimide (152.9 mg, 0.86 mmol) was added. The reaction mixture was stirred at room temperature for 24 hours and was then slowly poured into 5 N NaOH (35 ml). The aqueous mixture was then saturated with NaCl, and extracted with ether. The ether was washed with brine, dried with K₂CO₃, and evaporated at room temperature under reduced pressure. This gave a crude mixture (544.2 mg). The mixture could be purified by chromatography on silica, eluting with 7% methanol in dichloromethane, and the isomers seperated by preparative HPLC on a C₁₈ column, eluting with acetonitrile : methanol : 0.1% NH₄OAc (41 : 41 : 18 to 42 : 42 : 16 in a 60 minute linear gradient). This gave pure (5*S*,6*S*)-5-acetoxy-6-bromo Spinosyn A, FDMS, m/e (relative intensity) 874 (50), 873 (98), 872 (48), 871 (M⁺, 100) and (5*R*,6*R*)-5-acetoxy-6-bromo-5,6-dihydro Spinosyn A, FDMS, m/e (relative intensity) 874 (60), 873 (90), 872 (45), 871 (M⁺, 100) as white solids.

### Example F5 - 5,6-Dibromo Spinosyn A 17-Psa

To a solution of Spinosyn A (257.2 mg, 0.35 mmol) in carbon tetrachloride (10 ml), bromine (18 ml, 0.35 mmol) was added. The reaction mixture was stirred at room temperature for 20 hours, during which time the reaction color faded to a light yellow. Solvent was then evaporated at room temperature under reduced pressure, however, tlc (eluting with 10% methanol in dichloromethane) showed the reaction to be incomplete. The residue was redissolved in carbon tetrachloride (10 ml) and bromine (18 µl, 0.35 mmol) was added. The reaction mixture was stirred at room temperature for 3 days, in which time the reaction color faded. The mixture was then diluted with dichloromethane and washed with aqueous 5% sodium thiosulfate. The dichloromethane was dried with MgSO₄ and evaporated at room temperature under reduced pressure. The product was purified by chromatography on silica, eluting with 3% methanol in dichloromethane. This gave 5,6-dibromo Spinosyn A 17-Psa (142.9 mg ; 54% yield) as a white solid, FDMS, m/e (relative intensity) 750 (M⁺, 10), 189 (30), 101 (100).

### Example F6 - 5,6-Dihydro Spinosyn A(246088)

To a solution of Spinosyn A (504 mg ; 0.69 mmol) in dry benzene (30 ml), tris(triphenylphosphine)rhodium chloride (50.6 mg ; 0.055 mmol) was added and mixture was place in an atmospheric hydrogenator under hydrogen. After magnetic stirring (temperature was not monitored) 6 hr and then standing under N₂ atmosphere for 19 hr, the NMR of a small aliquot showed that reaction was about 2/3 complete. An additional tris(triphenylphosphine)rhodium chloride (50 mg ; 0.055 mmol) was added and the mixture was replaced under hydrogen atmosphere. After magnetic stirring and additional 24 hr the mixture was evaporated at room temperature under reduced pressure. The residue was intially purified by flash chromatography on silica with 5% MeOH in dichloromethane, giving the crude product (489.1 mg) as a yellow glass. The product was further purified by delta-prep reverse phase HPLC eluting with CH₃CN [38%]: MeOH [38%]: 0.05% NH₄OAc [24%] with a gradient to CH₃CN [45%]: MeOH [45%]: 0.05% NH₄OAc [10%]. This gave 5,6-dihydro Spinosyn A (275.4 mg ; 54% yield) as a pale yellow glass. Partial ¹H-NMR (CDCl₃) δ 6.82(1H, bs), 4.79(1H, s), 4.61(1H, m), 4.39(1H, bd), 4.17(1H, bq), 3.23(1H, m), 2.90(1H, m), 2.76(1H, m), 2.50(1H, m), 0.97(1H, m), 0.64(1H, m).

### Example F7 - (5S,6R)-5,6-Epoxy Spinosyn A and (5R,6S)-Epoxy Spinosyn A

To a solution of a 6:1 mixture of (5*S*,6*R*) and (5*R*,6*S*)-epoxy Spinosyn A, *N*-oxide(2.6 gms ; 3.4 mmol) in 300 ml chloroform under nitrogen, triphenylborane (823 mg, 3.4 mmol) was added and the mixture stirred at room temperature for 6 hr. The reaction was then diluted with dichloromethane and washed with 1 N NaOH. The dichloromethane was dried with K₂CO₃, and evaporated under reduced pressure giving an off white glass (2.57 gms). The crude material was purified by delta-prep reverse phase HPLC eluting with MeOH [42%] : CH₂CN [42%] : 0.25% NH₄OAc [16%] for 22 minutes and then MeOH [45%] : CH₂CN [45%] : 0.25% NH₄OAc [10%] for 14 minutes. This gave (5*R*,6*S*)-epoxy Spinosyn A (0.26 gms, 11%) partial ¹H-NMR (CDCl₃) d 6.71(1H, bs), 4.86(1H, s), 4.71(1H, m), 4.42(1H, bd), 4.30(1H, bq), 2.56(1H, dt), 2.47(1H, dd) and (5*S*,6*R*)-5,6-epoxy Spinosyn A (1.3 gms ; 54% yield) partial ¹H-NMR (CDCl₃) δ 6.59(1H, bs), 4.86(1H, s), 4.68(1H, m), 4.42(1H, bd), 4.23(1H, bq), 2.59(1H, dt), 2.45(dd) both as white solids.

### Example F8 - (5S,6R)-Epoxy Spinosyn D and (5R,6S)-Epoxy Spinosyn D

The reaction was run as described in Example F8, starting with a 6:1 mixture of (5*S*,6*R*) and (5*R*,6*S*)-epoxy Spinosyn D, N-oxide (450 mg , 0.58 mmol). This gave (5*R*,6*S*)-epoxy Spinosyn D (16.4mg, 4%) partial ¹H-NMR(CDCl₃) δ 6.71(1H, bs), 4.59(1H, m), 1.40(3H, s), and (5*S*,6*R*)-epoxy Spinosyn D (82.1 mg; 19% yield) partial 1H-NMR (CDCl₃) δ 6.59(1H, bs), 4.85(s), 4.68(1H, m), 4.42(1H, bd), 4.23(1H, bq), 2.56(1H, dt), 2.41(1H, dd), 1.39(3H, s) both as white solids. Low yields are due to loss on reverse phase HPLC column.

### Example F9 - Mixture of (5S,6R) and (5R,6S)-Epoxy Spinosyn A, N-oxide

To a solution of Spinosyn A (212.2 mg, 0.238 mmol ; 82.1% purity) in 10 ml of dichloromethane, *m*-chloroperoxybenzoic acid (135 mg, 0.771mmol) was added and the mixture stirred at room temperature for 3 days. The reaction was partitioned between saturated NaHCO₃ and dichloromethane. The phases were seperated and the aqueous was extracted with fresh dichloromethane. The organics were combined, dried with MgSO₄, and solvent was evaporated under reduced pressure, giving a white solid (246.8 mg). The crude product was purified by chromatography on silica eluting with 10% MeOH in dichloromethane going to 20% MeOH in dichloromethane in a one step gradient. This gave a 6:1 mixture of (5*S*,6*R*) and (5*R*,6*S*)-epoxy Spinosyn A, N-oxide as a white solid (181.2 mg ; ∼100%); partial ¹H-NMR (CDCl₃) δ 6.71 and 6.60 (1H, s), 4.87 (1H, s), 4.76(m, 1H), 4.70(1H, m), 4.32(1H, m), 4.26(1H, m), 3.42(s), 3.31(s), 3.24(s)

### Example F16 - (5S,6R) and (5R,6S)-Epoxy Spinosyn D, N-oxide

The reaction was run as described in Example F10 starting with Spinosyn D (517.9 mg , 0.70 mmol). This gave a 6:1 mixture of (5*S*,6*R*) and (5*R*,6*S*)-epoxy Spinosyn D, N-oxide (450.4 mg ; 83% yield) as a yellow glass Partial ¹H-NMR (CDCl₃) δ 6.71 and 6.60(1H, s),1.40(3H, s).

### Example F11 - (5R,6R)-5,6-Dibromo-4"-keto Spinosyn Aand (5R,6R)-5,6-dibromo Spinosyn B

Compound Spinosyn A (1.98 g, 2.70 mmol) was dissolved in CH₂Cl₂ (90 ml). Triethylamine (10 ml) was added and the reaction mixture was cooled to 0°C. Bromine (5.0 g, 27.8 mmol) was added with vigorous stirring. After 15 min. the temperature was raised to RT and stirring was continued for another 30 min.. The mixture was diluted with CH₂Cl₂ (100 ml) and washed successively with water (2 x) and 10 % aqueous NaHSO₃ (2 x). The organic layer was dried over K₂CO₃ and concentrated *in vacuo*. The residue was chromatographed on a flash SiO₂ column (230-400 m, 120 g/ EtOAc, then EtOH) to give : a) compound (5*R*,6*R*)-5,6-dibromo-4''-keto Spinosyn A (342 mg, 15%) IR (KBr) ν 1720, 1660, 740, 595 cm⁻¹; ¹H-NMR (CHCl₃) d: 6.70 (1H, bs), 4.90 (1H, dd: 7.7, 2.9 Hz), 4.72 (3H, m:W_{H/2}= 12 Hz, 3.95 (1H, q: 6.6 Hz) and b) compound (5*R*,6*R*)-5,6-dibromo Spinosyn B; 1.22 g, 51%) IR (KBr) ν 3410, 1720, 1665 cm⁻¹; ¹H-NMR (CHCl₃) δ: 6.71 (1H, bs), 4.78 (3H, m: W_{H/2}= 12 Hz), 2.50 (3H, CH₃, s).

### Example F12 - 5,6-cis Dihydroxy-5,6-dihydro Spinosyn B (a 2:1 mixture of, respectively (5S,6R) and (5R,6S) isomers)

Compound Spinosyn A (3.26 g, 4.45 mmol) was dissolved in acetone (70 ml). Water (30 ml) was added, followed by N-methylmorpholine-N-oxide (645 mg. 5.5 mmol). After the N-oxide dissolved, OsO₄ was added (160 mg of a 2.5% solution in t-BuOH, .0015 mmol). The reaction mixture was stirred at RT. After 24 hrs. EtOAc (100 ml) and benzene (100 ml) were added. The mixture was successively washed with 10% aq. NaHSO₃ solution (1 x), water (1 x) and 5% aq. NaHCO₃ solution (1 x). The organic layer was dried over K₂CO₃ and concentrated *in vacuo.* The residue was separated over a flash SiO₂ column (EtOAc, then 10% MeOH in EtOAc) to give a mixture of *5,6-cis* dihydroxy-5,6-dihydro Spinosyn A (a 2:1 mixture of (5*S*,6*R*) and (5*R*,6*S*) isomers, 2.236 g, 66%) and a mixture of 5,6-*cis* dihydroxy-5,6-dihydro Spinosyn B (a 2:1 mixture of (5*S*,6*R*) and (5*R*,6*S*) isomers (339 mg, 10%) ¹H-NMR (CDCl₃) d: 6.72 (1H, bs), 4.76 (1H, bs), 3.92 (0.65 H, m), 3.85 (0.35 H, m), 2.34 (3H, CH₃, s).

### Example F13 - Dichloroketene acetal of (5S,6R)-5,6-dihydroxy Spinosyn A (5S,6R)-5,6-bis(trichloro-acetoxy) Spinosyn A

(5*S*,6*R*)-5,6-dihydroxy-5,6-dihydro Spinosyn A (376 mg, 0491 mmol) was dissolved in dry CH₂CL₂ (10 ml). Pyridine (3 ml) was added, followed by N,N-dimethylaminopyridine (600 mg). The reaction mixture was stirred at RT under nitrogen and trichloroacetyl chloride (1.00 ml, an excess) was added. After 1 hr. the mixture was diluted with EtOAc (100 ml) and PhH (50 ml). The solution was successively washed with brine (1x) and 5% aq. NaHCO₃ (2x) and dried over Na₂SO₄. The organic layer was concentrated *in vacuo*. The residue was separated by flash column chromatography on a flash SiO₂ column (60 g/ EtOAc) to give: compound a) dichloroketene acetal of (5*S*,6*R*)-5,6-dihydroxy Spinosyn A (104 mg, 25%) IR (CHCl₃) ν: 1805, 1722, 1660 cm⁻¹; ¹H-NMR (CDCl₃) d: 6.62 (1H, bs), 4.96 (1H, dd: 7.8, 3.3 Hz), 4.85 (1H, dd:7.8, 3.9 Hz), 2.20 (6H, 2x CH₃, s) and compound b) (5*S*,6*R*)-5,6-bis(trichloroacetoxy) Spinosyn A (129 mg, 25%) IR (CHCl₃) ν 1772, 1720, 1662 cm⁻¹; 1H-NMR (CDCl₃) : 6.76 (1H, bs), 5.66 (1H, m: W_{H/2}=8 Hz), 5.50 (1H, dd: 5.7, 3.3 Hz) 2.19 (6H, 2x CH₃,s) ppm; CI MS m/z 1056 (M+1). contaminated, which readily crystallized from Et₂O to give pure compound 5-keto-6,7-en-6-hydroxy Spinosyn A (1.01 g, 67%) IR (CHCl₃) ν 1720, 1665, 1648 cm⁻¹; ¹H-NMR (CDCl₃) d: 6.68 (1H, bs), 3.84 (1H, dd: 9.2, 9.0 Hz), 2.13 (6H, 2x CH₃, s); CI MS m/z 762 (M+1).

### Example F14 - (5R,6R)-5,6-Dibromo Spinosyn A

Compound Spinosyn A (1.59 g, 2.17 mmol) was dissolved in dry CH₂Cl₂ (30 ml). The solution was stirred at RT under nitrogen. Phenylselenenyl bromide (98%, 1.05 g, 4.34 mmol) was added in one portion. After 30 min. of stirring the solution was cooled to 0°C. MCPBA (2.5 g, ca. 7 mmol) was added. Stirring at 0°C was continued for 30 min. Triethylamine (3 ml) was then added and after stirring for 10 min., the mixture was diluted with benzene (150 ml) and EtOAc (50 ml). The solution was washed successively with 10% aqueous NaHSO₃ (2x), brine (1x) and 5% aqueous NaHCO₃ (2x). The organic phase was dried over K₂CO₃ and concentrated *in vacuo.* The residue was purified by flash column chromatography (SiO₂ / EtOAc) and then by HPLC (C-18 coated, 6m, 100 A packing; 5% H₂O in MeOH as mobile phase). Pure fractions afforded a) unreacted compound Spinosyn A and b) compound (5*R*,6*R*)-5,6-dibromo Spinosyn A (297 mg, 16%) ¹H-NMR (CDCl₃) d: 6.69 (1H, bs), 4.74 (3H, m: W_{H/2}= 12 Hz), 2.17 (6H, 2x CH₃, s) ppm; CI MS m/z 894 (M+3), 892 (M+1).

### Example F21 - (5S)-5,6-Dihydro-5-hydroxy Spinosyn A(6S)-5,6-Dihydro-6-hydroxy Spinosyn A (6R)-5,6-Dihydro-6-hydroxy Spinosyn A

Mercuric trifluroactetate (1.12 g, 2.62 mmol) was suspended in 30 mL THF:water, 2:1 (V/V). To this yellow suspension was added compound Spinosyn A (0.98 g, 1.34 mmol). The reaction was then allowed to stir at RT for 30 min. The reaction was treated with 7 mL 1N NaOH followed by 2.5 mL 0.5M NaBH₄ in 3M NaOH. Reaction turned black supporting a precipitate. Allowed solids to settle to bottom of flask and decanted off liquid into a separatory funnel containing 50 mL ether. Separated layers and extracted aqueous with 2 x 25mL ether. Combined ether extracts, washed with 30 mL saturated aqueous NaHCO₃, dried over K₂CO₃ and concentrated *in vacuo*. The residue was purified by medium pressure liquid chromatogrpahy {230-400m SiO2, CH₂Cl₂:MeOH, 95:5 (V/V} to give: compound a) (5*S*)-5,6-dihydro-5-hydroxy Spinosyn A (349.7 mg, 30.4%) partial ¹H-NMR (CDCl₃) d 6.80 (1H, bs), 4.00 (1H, m), 2.95 (1H, m) 0.68 (1H, m); partial ¹³C-NMR (CDCl₃) d 148.64, 67.40, 45.79, 43.01 35.16 and a mixture of compounds (6*S*)-5,6-dihydro-6-hydroxy Spinosyn A and (6*R*)-5,6-dihydro-6-hydroxy Spinosyn A . Compounds (6*S*)-5,6-dihydro-6-hydroxy Spinosyn A and (6R)-5,6-Dihydro-6-hydroxy Spinosyn A were separated by HPLC (C-18 coated, 6m, 100 A packing; 10% H₂O in MeOH as mobile phase) to give compound b) (6*R*)-5,6-dihydro-6-hydroxy Spinosyn A (7.1 mg, 1%) partial ¹H-NMR (CDCl₃) d 6.84 (1H, bs), 3.48 (1H, m) 2.92 (1H, m) 0.85 (1H, m); ¹³C-NMR (CDCl₃) d 148.64, 72.66, 44.86, 43.96, 34.52 and compound c) (6*S*)-5,6-dihydro-6-hydroxy Spinosyn A (11.4 mg, 1%) partial ¹H-NMR (CDCl₃) d 6.80 (1H, bs), 4.11 (1H, m) 2.78 (1H, m) 1.40 (1H, m); ¹³C-NMR (CDCl₃) d 148.46, 66.52. 38.18, 37.65, 32.95.

### Example F16 - (5R,6R)-5-Acetoxy-6-thiomethoxy Spinosyn A

Compound Spinosyn A (2.64 g, 3.61 mmol) was dissolved in CH₃CN (dry, cont 1% of diethyl sulfide; 30 ml). To this solution maintained at RT was added dimethyl(methylthio)sulfonium tetrafluoroborate (1.10 g, 5.61 mmol) and the mixture was sonicated (50 W ultrasound cleaner) for 10 min. Anhydrous sodium acetate powder (2.5 g, an excess) was then added and the mixture was sonicated for 1 hr. The solution was diluted with EtOAc (50 ml) and PhH (100 ml) and successively washed with 5% aqueous K₂CO₃, 5% aq. NaHCO₃ and H₂O. The organic layer was dried over Na₂SO₄, and concentrated *in vacuo.* The residue was purified by flash column chromatography on SiO₂ and then by preparative HPLC (C-18 coated, 6m, 100 A packing; 8% H₂O in MeOH as mobile phase). Pure fractions afforded compound (5*R*,6*R*)-5-acetoxy-6-thiomethoxy Spinosyn A (2.07 g, 68%) ¹H-NMR (CDCl₃) d: 6.69 (1H, bs), 5.31 (1H, dd: 2.4, 2.2 Hz), 2.16 (6H, 2x CH₃, s), 2.11 (3H, CH₃, s), 2.01 (3H, CH₃, s); Elemental Analysis: for C₄₄H₇₁NO₁₂S calc. C 63.06, H 8.54, N 1.67; found C 63.15, H 8.77, N 1.76.

### Example F17 - (5R,6R)-5-Acetoxy-6-methylsulfonyl Spinosyn A

Compound (5*R*,6*R*)-5-acetoxy-6-thiomethoxy Spinosyn A (1.05 g, 1.25 mmol) was dissolved in CH₂Cl₂ (100 ml). The solution was cooled to 0°C and, upon stirring, m-CPBA (50%, 1.95 g, 5.6 mmol) was added in a few portions. After 40 min. the reaction mixture was diluted with CH₂Cl₂ (50 ml). The solution was washed successively with 10% aqueous NaHSO₃ (2x), water (1x) and 5% aq. NaHCO₃ (2x). The organic layer was dried over Na₂SO₄ and concentrated *in vacuo.* The residue was purified by a flash column chromatography (230-400m SiO₂ / EtOAc, then 10 % EtOH in EtOAc) to give compound (5*R*,6*R*)-5-acetoxy-6-methylsulfonyl Spinosyn A (966 mg, 89%) IR (CHCl₃) ν 1740, 1724, 1662, 1320, 1135 cm⁻¹; ¹H-NMR (CDCl₃) d: 6.57 (1H, bs), 5.52 (1H, dd:4.5, 4.0 Hz), 2.78 (3H, CH₃, s), 2.17 (6H, 2x CH₃, s), 1.98 (3H, CH₃, s).

### Example F18 - (5R,6R)-6-Bromo-5-hydroxy Spinosyn J

Compound Spinosyn A (1.33 g, 1.82 mmol) was dissolved in DMSO (15 ml). Water (5 ml) was added, causing precipitation. Upon stirring was introduced conc. H₂SO₄ (1.8 mmol). The precipitate dissolved immediately. The solution was cooled to 0°C and N-bromosuccinimide (322 mg, 1.8 mmol) was added. After stirring at 0°C for 15 min., the reaction mixture was poured onto 50 ml sat. aq. NaHCO₃. The mixture was extracted with Et₂O (3x). Combined organic extracts were washed with brine, dried over K₂CO₃ and concentrated *in vacuo*. The residue was purified by reversed phase HPLC (C-18 Dynamax column, 20% water in MeOH as mobile phase) to give compound (5*R*,6*R*)-6-bromo-5-hydroxy Spinosyn J (1.30 g, 86%) CI MS m/z 828 (M+1); ¹H-NMR (CDCl₃) d: 6.56 (1H, bs), 4.24 (2H, m: W_{H/2}= 14 Hz), 3.85 (1H, dd: 3.5, 3.5 Hz), 2.05 (6H, 2x CH₃, s).

### Example F19 - 5,6-Dihydro Spinosyn A 9-Psa

Compound Spinosyn A 9-Psa ( 46.5 mg, 0.0856 mmol) was dissolved in 5 mL toluene. To this solution was added tris-triphenylphoshine rhodium(I) chloride (8.5 mg, 0.0092 mmol). Evacuated the flask head space and introduced nitrogen three times. Evacuated and introduced hydrogen three times. Maintained flask under hydrogen with balloon and heated reaction to 110-120°C. After 3.5 hr cooled flask to RT and evacuated hydrogen and replaced with nitrogen. Evaporated off toluene solvent and replaced with 20 mL ether. Extracted ether solution with 3 x 10 mL lN HCl. Combined acid extracts and neutralized with 10 ml 4N NaOH. Extracted neutralized suspension with 3 x 10 mL ether, combined ether extracts, washed with 20 mL brine, dried over K₂CO₃, and evaporated *in vacuo*. Gave compound 5,6-dihydro Spinosyn A 9-Psa (29.5 mg, 63%) partial ¹H-NMR (CDCl₃) d 6.84 (1H, bs), 1.01 (1H, m), 0.68 (1H, m).

### Example F20 - 5,6-Dihydro Spinosyn A 17-Psa

The procedure described above in Example F25 was applied to compound Spinosyn A 17-Psa (270.8 mg, 0.458 mmol) with 32.8 mg [(Ph)₃P]₃RhCl. After evaporation of toluene the compound was purified directly by medium pressure liquid chromatography (230-400m SiO₂, CH₂Cl₂:MeOH, 95:5 (V/V). Gave compound 5,6-dihydro Spinosyn A 17-Psa (188.1 mg, 69.2%) partial ¹H-NMR (CDCl₃) d 6.84 (1H, bs), 3.63 (1H, m), 1.02 (1H, m), 0.68 (1H, m); partial ¹³C-NMR (CDCl₃) d 149.28, 72.48, 46.55, 27.00.

### Example F21 - 5,6-Dihydro Spinosyn J

The procedure described above in Example F25 was applied to compound Spinosyn J (1.00 g, 1.39 mmol) using 91.3 mg [(Ph)₃P]₃RhCl in 20 mL of toluene. This gave compound 5,6-dihydro Spinosyn J (0.70 g, 70%) partial ¹H-NMR (CDCl₃) d 6.84 (1H, bs) 3.82 (1H, dt), 1.01 (1H, m), 0.69 (1H, m).

### Example F22 - 5,6-Dihydro Spinosyn H

Compound Spinosyn H (2.57 g, 3.58 mmol) was dissolved in 20 mL toluene in a 250 mL round bottom flask. To this colorless solution was added a solution of [Ph₃P]₃RhCl (169.2 mg, 0.183 mmol) in 5 mL toluene. The flask was fitted with a reflux condensor and the flask head space was evacuated three times and nitrogen gas introduced. The nitrogen was evacuated three times and hydrogen gas introduced. The flask was maintained under 1 atm hydrogen with a balloon and heated to 100-110°C in an oil bath. After 10 hr., the heat was removed and the flask evacuated three times with nitrogen gas being introduced after each evacuation. The contents of the flask was filtered hot under a nitrogen atmosphere through a 3" column of celite. The celite was washed with 100 mL ether to elute the remaining compound. The filtrate was extracted with 3 x 50 mL 1N HCl. The acid extracts were combined, back extracted with 25 mL ether, and basified with 50 mL 4N NaOH. The white solid which precipitated was extracted into ether (2 x 25 mL) and the ether extracts washed with 25 mL brine solution. The ether solution was filtered through a plug of celite and evaporated *in vacuo.* This gave compound 5,6-dihydro Spinosyn H (2.24 g, 87.1%). Anal. Calcd. for C₄₀H₆₅NO₁₀: C 66.73, H 9.10, N 1.95; Found C 66.31, H 9.01, N 2.02. CI MS (m/z) 721 (M+1).

### Example F23 - 5,6-Dihydro Spinosyn A Hemiglutarate salt

Compound 5,6-dihydro Spinosyn A (232.2 mg, 0.316 mmol) was dissolved in 3 mL acetone. A solution of glutaric acid (20.8 mg, 0.157 mmol) in 3 mL acetone was added and the solution stirred at RT overnight. Removed the solvent *in vacuo* to give compound 5,6-dihydro Spinosyn A hemiglutarate salt (250.8 mg). Anal calcd for C₄₁H₆₇NO₁₀ · 1/2(C₅H₈O₄) C 65.31, H 8.94, N 1.75; Found C 65.14, H8.78, N 1.87; mp. 83-92°C.

### Example F24 - 5,6-Dihydro Spinosyn B

*Hydrogenation Procedure A:* A solution of Spinosyn B (1.10 g, 1.53 mmol) and (Ph₃P)₃RhCl (0.05 g, 0.09 mmol) in PhMe (15 mL) was placed on a Parr apparatus and maintained under an H₂ atmosphere (45-50 psi) for 2 h at 104-107°C. After cooling to room temperature, the mixture was evaporated *in vacuo* and the residue was decolorized (Et₂O/charcoal) and filtered. The filtrate was evaporated *in vacuo* and the residue was purified by MPLC (SiO₂, 5:95 → 10:90 MeOH/CH₂Cl₂) to give 0.90 g (82%) of 5,6-dihydro Spinosyn B as a white powder. Calcd for C₄₀H₆₅NO₁₀: C, 66.73; H, 9.10; N, 1.95. Found: C, 66.53; H, 9.14; N, 2.15.

### Example F25 - Synthesis of compound (5S,6R)-Epoxy-Spinosyn Q

Spinosyn Q (970 mg, 1.32 mmol) was dissolved in methylene chloride (150 ml). The solution was cooled to 0°C and m-CPBA (1.19 g of ca. 50% reagent, ca. 3.45 mmol) was added in one portion. Immediately after m-CPBA dissolved, the reaction mixture was set aside in a refrigerator. After 40 hrs. the reaction mixture was extracted with 10% aqueous solution of NaHSO₃ (2x), with water (1x) and with 5% aq. solution of NaHCO₃ (2x). The organic layer was dried over anh. K₂CO₃, filtered and concentrated *in vacuo.* The residue was separated over a flash SiO₂ column (230-400 m, 70 g / EtOAc). Concentration *in vacuo* of chromatographically homogenous fractions gave a white solid (694 mg). A white solid (88 mg) precipitated from a solution of this material upon attempted crystallization from dry Et₂O. The ether solution, upon evaporation, gave compound (5*S*,6*R*)-epoxy-Spinosyn Q, 606 mg, 61 %) ¹H-NMR d 6.51 (1H, bs), 4.73 (1H, d: 1.2 Hz), 3.87 (1H, dd: 1.2, 1.8 Hz), 2.16 (6H, bs 2x CH₃), 1.31 (3H, s, CH₃) ppm.

### Example F26 - Mixture of (5S,6R)-5,6-dihydro-5,6-dihydroxy Spinosyn A (65%) and (5R,6S)-5,6-dihydro-5,6-dihydroxy Spinosyn A (35%)

Spinosyn A (3.26 g, 4.45 mmol) was dissolved in acetone (70 mL). Water (30 mL) was added, followed by *N*-methylmorpholine *N*-oxide (645 mg, 5.5 mmol), and osmium tetroxide (2.5% solution in 2-methyl-2-propanol; 160 mg, 0.015 mmol). After 1 h another portion of OsO₄ (300 mg) was added. After 24 h the mixture was combined with 10% aqueous NaHSO₃ solution (200 mL) and worked up. The crude product was purified over a short flash column with silica gel (10 % MeOH in EtOAc) to furnish an unseparable mixture of (5*S*,6*R*)-5,6-dihydro-5,6-dihydroxy Spinosyn A (65%) and (5*R*,6*S*)-5,6-dihydro-5,6-dihydroxy Spinosyn A (35%) (2.24 g, 66%) as a white solid: ¹H NMR d 6.71 (s, 1 H), 3.93 (m, 0.65 H), 3.88 (m, 0.35 H), 2.11 (s, 6 H).

### Example F27 - 5,6-Dihydro Spinosyn B

A solution of Spinosyn B (1.10 g, 1.53 mmol) and (Ph₃P)₃RhCl (0.08 g, 0.09 mmol) in PhMe (15 mL) was subjected to an H₂ atmosphere (45-50 psi) at 104-107°C on a Parr apparatus. After 2 h, the mixture was filtered, and evaporated. The residue was dissolved into Et₂O and slurried with charcoal. This slurry was filtered and evaporated. MPLC (5:95 MeOH/CH₂Cl₂) gave 0.90 g (82%) of 5,6-dihydro Spinosyn B as a white powder. Anal. Calcd for C₄₀H₆₅NO₁₀: C, 66.73; H, 9.10; N, 1.95. Found: C, 66.53; H, 9.14; N, 2.15.

### Example F28 - 5,6-dihydro Spinosyn D

To a solution of Spinosyn D (2.5 g, 3.4 mmol) in absolute ethanol (60 mL), 10% Pd/C and cyclohexene was added in a 100 ml Parr vessel. This suspension was heated at 120°C for 48 h. After cooling, the reaction mixture was filtered through a pad of celite and concentrated *in vacuo* to give 2.4 g of 2:1 mixture of 5,6-dihydro Spinosyn D and Spinosyn D respectively. The residue (200 mg) was dissolved in dichloromethane (20 mL) and cooled to 0°C. MCPBA (95 mg, 0.45 mmol) was added to the reaction mixture and stirred at 25°C for 16 h. The reaction mixture was quenched with sodium bicarbonate solution and layers separated. Aqueous layer was extracted with dichloromethane (2 x 20 mL). The combined extracts were stirred with 10% NaHCO₃ for 3 h, washed with brine, dried over anh. Na₂SO₄ and concentrated *in vacuo.* The residue was purified on a prep HPLC on a C18 column, eluting with 10% water (0.1% NH₄OH) in methanol to give compound 5,6-dihydro Spinosyn D (10 mg). MS m/z 748. ¹H NMR (CDCl₃, 300 MHz) 6.85 (1H, s), 4.82 (1H, s), 4.65 (1H, m), 4.42 (1H, d), 4.20 (1H, m), 0.95 (3H, d).

### Part I DEOXY Rhamnose Derivatives

### Example I1 - 9-(2H-5-R-Acetoxy-5,6-dihydro-6-S-methyl-2-a-pyranyl)-spinosyn A 9-pseudoaglycone

Spinosyn A 9-pseudoaglycone (272 mg, 0.500 mmol) and 3,4-di-*O*-acetyl-6-deoxy-L-glucal (214 mg, 1.00 mmol) were dissolved in CH₂CL₂ (3 mL). Molecular sieves (4Å, 40 mg) and camphorsultonic acid (128 mg, 0.550 mmol) were added. After 18 h additional camphorsulfonic acid (20 mg, 0.086 mmol) was added. The mixture was stirred for 3 days. The mixture was diluted with CH₂Cl₂ (10 mL) and washed with a 1:1:1 mixture of water-saturate aqueous sodium bicarbonate-1.0 N NaOH. The aqueous layer was extracted with CH₂Cl₂ (twice) and the combined organic phases washed with water, dried with anhydrous sodium sulfate and evaporated to yield a dark oil. The residue was chromatographed on a reversed-phase column (Kromasil' C18, Silica ODS, 100Å, 10 m, spherical, 25 cm X 20 mm) using 90% MeOH and 10% water (containing 0.1% v/v conc. aqueous NH₄OH) to yield a white foam (44 mg, 11%). ¹H NMR d 5.92 - 5.73 (m, 4 H), 5.01 (br s, 1 H), 2.09 (s, 3 H).

### Example I2 - 5,6-Dihydro-9-(2-deoxy-3-O,4-O-diethyl-1-α-rhamnosyl)-spinosyn A 9-pseudoaglycone

9-(2-deoxy-3-*O*,4-*O*-diethyl-1-a-rhamnosyl)-spinosyn A 9-pseudoaglycone (200 mg, 273 mmol) and cyclohexene (0.40 mL, 3.95 mmol) were dissolved in absolute ethanol (4 mL). Palladium on charcoal (10%, 20 mg, 19 mmol) was added cautiously and the resulting mixture heated at reflux temperature for 3 hr. The mixture was stirred an additional 17 hr. at room temperature. The mixture was filtered through Celite and evaporated under a stream of nitrogen. The residue was chromatographed on a reversed-phase column (Kromasil' C18, Silica ODS, 100Å, 10 m, spherical, 25 cm X 20 mm) with 90% MeOH and 10% water (containing 0.1% v/v conc. aqueous NH₄OH) to yield a glassy solid (45 mg, 22%) ¹H NMR d 6.83 (br s, 1 H), 2H-multiplet at 5.9 - 5.7 absent.

### Example I3 - 5,6-Dihydro-9-(2-deoxy-3-O,4-O-di-n-propyl-1-α-rhamnosyl)-spinosyn A 9-pseudoaglycone

Cyclohexene (3.0 mL, 28.6 mmol) and palladium on charcoal (10%, 43 mg) were added to absolute ethanol (3 mL). A solution of 9-(2-deoxy-3-*O*,4-*O*-di-n-propyl-1-a-rhamnosyl)-spinosyn A 9-pseudoaglycone (188 mg, 0.248 mmol) in ethanol (2 mL) was added and the mixture heated at reflux temperature for 5.5 h. Additional palladium on charcoal was added to the reaction mixture at 1 h (38 mg) and at 4.5 h (67 mg). The mixture was cooled, filtered through Celite and concentrated to a thick oil. The residue was dissolved in Et₂O and washed with concentrated aqueous sodium bicarbonate and brine, dried over anhydrous potassium carbonate and evaporated to yield a light gray foam. This material was chromatographed on a reversed-phase column (Kromasil' C18, Silica ODS, 100Å, 10 m, spherical, 25 cm X 20 mm) with 92% MeOH and 8% water (containing 0.1% v/v conc. aqueous NH₄OH) to yield a white amorphous powder (80 mg, 42%). ¹H NMR d 6.73 (br s, 1 H), 2H-multiplet at 5.9 - 5.7 absent.

### Example 18- Insecticide and Miticide Utility

The compounds show activity against a number of insects and mites. More specifically, the compounds show activity against melon aphid, which is a member of the insect order *Homoptera.* Other members of the *Homoptera* include leafhoppers, planthoppers, pear pyslla, apple sucker, scale insects, whiteflies, spittle bugs as well as numerous other host specific aphid species. Activity has also been observed against greenhouse thrips, which are members of the order *Thysanoptera.* The compounds also show activity against Southern armyworm, which is a member of the insect order *Lepidoptera*. Other typical members of this order are codling moth, cutworm, clothes moth, Indianmeal moth, leaf rollers, corn earworm, European corn borer, cabbage worm, cabbage looper, cotton bollworm, bagworm, eastern tent caterpillar, sod webworm, and fall armyworm.

The compounds were useful for reducing populations of insects and mites, and were used in a method of inhibiting an insect or mite population which comprises applying to a locus of the insect or mite an effective insect- or mite-inactivating amount of a compound described in the Examples above. Results reported in the following tables, wherein the following abbreviations were used:
ALH refers to aster leafhopper
BAW refers to beet armyworm
CA refers to cotton aphid
NEM refers to peanut rootknot nematode
SCRW refers to southern corn rootworm
TBW refers to tobacco budworm
TSSM refers to two spotted spider mite
GECR refers to German cockroach

In conducting evaluations of insecticidal activity, each test compound was formulated as a 400 ppm solution, and this solution was then diluted with water to give lesser concentrations. The 400 ppm solution was prepared by combining 19.2 mL of .05% solution of Tween 20 (polyoxyethylene (20) sorbitan monolaurate) in water with a solution of 8 mg of the compound in .8 mL of acetone/EtOH (9/1).

Activity against aster leafhopper (*Macrosteles fascifrons*) was tested as follows. The test was run using concentrations of 400 ppm and 50 ppm. One ounce plastic cups containing a cotton wick was sprayed with 0.4 mL of formulated material using a flat-fan nozzle. The excess moisture was allowed to evaporate. Then five to ten carbon dioxide anesthetized adult leafhoppers were added to each cup. The cups were capped and held at room temperature for 24 hours. Percent mortality was then determined.

Activity against beet armyworm (*Spodoptera exiqua*) was evaluated as follows. The test is run using concentrations of 400 ppm and 50 ppm. A general purpose lepidoptera artificial diet was diluted to half strength with a 5% non nutritive agar. 8 mL of this diet material was dispensed into one ounce diet cups. One hour prior to treatment, 35 to 40 eggs were dispensed onto the diet surface. The cups were then sprayed with formulated material through a flat-fan nozzle. Treated cups were air dried prior to sealing with plastic caps. The cups were held for 6 days at room temperature. Activity was then rated based on the total number of live and dead larvae, and on the size of live larvae.

Activity against cotton aphid (*Aphis gossypii*) and two spotted spider mite (*Tetranychus urticae*) was evaluated as follows. Golden crookneck squash plants were grown to the expanded cotyledon stage (about 6 to 8 days). The plants were infested with cotton aphids and two spotted spider mites 16 to 24 hours before application of the test material by transfer of infested foliage cut from a stock colony. Immediately prior to spray application of the test material the transfer foliage is removed from the squash plants. The test is run using concentrations of 400 ppm and 50 ppm. The plants are sprayed with test solution using an atomizing sprayer at 17 psi. Both surfaces of the leaves are covered until runoff, and then allowed to dry. Activity of each compound was determined three days after treatment. Activity was rated as a percent based on the mites/aphids present in plants sprayed with solvent alone.

Activity against peanut root knot nematode (*Meloidogyne arenaria*) was evaluated as follows. Five untreated cucumber seeds are placed into the bottom of a clear one ounce cup, 20 g of clean white sand is added, and the cups were sprayed while rotating on a pedestal allowing 1.0 mL of a 400 ppm solution to be deposited on the sand. To each cup was dispensed 2.5 to 3.0 mL of deionized water containing 300 to 500 nematodes. The cups were held for 10 to 12 days in an environmental growth chamber at a temperature of 76 to 85°F and ambient humidity of 50 to 60%. After 10 to 12 days the cups were evaluated by inverting the cup and observing nematode mortality and feeding damage to the cucumber plants.

Activity on Southern corn rootworm (*Diabrotica undecimpuctata howardi* Barber) was evaluated by adding one mL of test solution containing a predetermined concentration of test compound to a cup containing a kernel of corn in 16 g of sterile soil. This produces a soil concentration of 24 ppm. After 1.5 to 2 hours of drying, five 4th instar corn rootworm larvae were added to the individual cups. Mortality was measured at 3-4 days by emptying the cup onto a pan and inspecting the soil for live rootworms.

Activity against tobacco budworm (*Heliothis virescens*) was evaluated as follows. A general purpose lepidoptera artificial diet was diluted to half strength with a 5% non nutritive agar. 8 mL of this diet material was dispensed into each one ounce diet cup. One hour prior to treatment 18 to 20 eggs were dispensed onto the diet surface. The cups were then sprayed with formulated material through a flat-fan nozzle. The test was run using concentrations of 400 ppm and 50 ppm. Treated cups were air dried prior to sealing with plastic caps. The cups were held for 6 days at room temperature. Activity was then rated based on the total number of live and dead larvae, and on the size of live larvae.

Activity against German cockroach (*Blattella germanicus*) was evaluated as follows. 8 mL of alfalfa based green insect diet material was dispensed into a one ounce diet cup. The cups were then sprayed with formulated material through a flat-fan nozzle. The test was run using concentrations of 400 ppm and 50 ppm. Treated cups were air dried for 24 hours and infested with five late third or early fourth instar German cockroaches. The cups were capped and held for ten days in an environmental growth chamber at a temperature of 76-85°C. Activity was then rated based on the total number of live and dead insects.

### Nematicide Utility

The method was practiced in accordance with standard techniques for the application of nematicides. In general, good nematicidal activity can be expected at rates of 1-10 lbs/acre. The compound can be formulated as described herein. When formulated as dispersions, nematicides are typically applied as aqueous drenches around growing plants or applied incrementally via irrigation systems. When applied as granules, nematicides may be incorporated into the soil before planting, or applied in a band on top of a seed row, or broadcast and then incorporated into the soil, or used as a side dressing to an established crop.

The following activity was found for the compounds described below:

| EXAMPLE NUMBER | INSECT | RATE | % MORTALITY |
|---|---|---|---|
| A1 | TBW | 400 | 100 |
| A1 | TSSM | 400 | 100 |
| A2 | TBW | 400 | 100 |
| A3 | TBW | 400 | 100 |
| A3 | TSSM | 400 | 100 |
| A4 | TBW | 400 | 100 |
| A4 | TSSM | 400 | 100 |
| A5 | TBW | 400 | 100 |
| A6 | TBW | 400 | 100 |
| A6 | TSSM | 400 | 100 |
| A7 | TBW | 400 | 100 |
| A7 | TSSM | 400 | 100 |
| A8 | TBW | 400 | 100 |
| A8 | TSSM | 400 | 100 |
| D1 | TBW | 80 | 90 |
| D3 | TBW | 64 | 95 |
| E1 | TBW | 400 | 100 |
| E1 | TSSM | 400 | 60 |
| E2 | TBW | 80 | 35 |
| E3 | NEMA | 400 | 100 |
| F1 | TBW | 400 | 100 |
| F2 | TBW | 400 | 100 |
| F3 | TBW | 64 | 24 |
| F4a | TBW | 400 | 100 |
| F4b | TBW | 400 | 100 |
| F6 | TBW | 64 | 100 |
| F6 | TSSM | 400 | 100 |
| F7a | TBW | 400 | 100 |
| F7a | TSSM | 400 | 90 |
| F7b | TBW | 400 | 100 |
| F8a | TBW | 400 | 100 |
| F8a | TSSM | 400 | 90 |
| F8b | TBW | 400 | 100 |
| F8b | TSSM | 400 | 90 |
| F9 | Intermed | | |
| F10 | Intermed | | |
| F11a | BAW | 56 | 80 |
| F11b | TBW | 400 | 100 |
| F12 | TBW | 400 | 100 |
| F13a | TBW | 400 | 100 |
| F13b | TBW | 400 | 60 |
| F14 | TBW | 400 | 60 |
| F15a | TBW | 400 | 100 |
| F16 | TBW | 400 | 100 |
| F17 | TBW | 400 | 100 |
| F18 | TBW | 400 | 100 |
| F19 | TBW | 400 | 100 |
| F20 | BAW | 400 | 100 |
| F21 | TBW | 400 | 100 |
| F22 | TBW | 400 | 100 |
| F23 | TBW | 400 | 100 |
| F23 | TSSM | 400 | 100 |
| F24 | TBW | 400 | 100 |
| F24 | TSSM | 400 | 100 |
| F25 | TBW | 400 | 100 |

### Example 19 Spinosyn derivatives of Example 17

### Control of Stomoxys calcitrans (stable fly) and Phormia regina (blow fly).

The test procedures were as follows.

The compound to be evaluated was dissolved in 1 part acetone/1 part ethanol to provide a stock solution of the compound at a concentration of 5,000 ppm; this solution was shaken for 15 minutes on a sonicator. Portions of the stock solution were placed in 15 ml test tubes, and portions of bovine serum were added to provide the desired dilution of the test compound. A dental wick was placed in each test tube and the serum allowed to saturate the wick.

For the blow fly test, approximately 20 blow fly larvae were placed onto the top center of the saturated dental wick, and the test tube was plugged with cotton and incubated at 27°C and 70% humidity for 24 and 48 hours. Larval mortality counts were then made, and adjusted for any mortality in the vehicle control, to determine percent efficacy against blow fly.

For the adult stable fly test, the saturated wick was placed on a filter paper in a petri dish, and approximately 10 chilled live, hungry stable flies were placed on the center of the dish bottom. The dish was covered and allowed to incubate at 27°C and 60% relative humidity for 48 hours. Mortality readings were made at each of 24 and 48 hours, and adjusted for any mortality in the vehicle control to determine percent efficacy against adult stable fly.

The results are reported below.

| | | | | |
|---|---|---|---|---|
| **Animal Science Data for Spinosoids** | | | | |

| Patent Example Number | Percentage of ASF killed at 100 ppm | Percentage of ASF killed at 10 ppm | Percentage of LBF killed at 100 ppm | Percentage of LBF killed at 10 ppm |
|---|---|---|---|---|
| A2 | 100% | | | |
| A6 | 100% | | | |
| A7 | 100% | | | |
| A8 | 100% | | | |
| D1 | | 100% | | <50% |
| D2 | 100% | <50% | | |
| D3 | 100% | | | |
| E1 | | | | <50% |
| F1 | | 60% | | 90% |
| F2 | | <50% | | <50% |
| F3 | | <50% | | <50% |
| F4a | | 90% | | <50% |
| F4b | | 60% | | < 50% |
| F5 | | <50% | | |
| F6 | | 100% | 100% | |
| F7b | | 100% | | 100% |
| F7a | | 100% | | 90% |
| F8b | | 100% | 100% | |
| F11a | <50% | | | |
| F11b | 100% | | | |
| F12 | 100% | | | |
| F14 | 100% | | | |
| F16 | 100% | | | |
| F17 | 100% | | | |
| F18 | 100% | | | |
| F20 | <50% | | | |
| F21 | 100% | | | |
| F22 | 100% | | | |
| F23 | 100% | | | |
| F24 | 100% | | | |

## Claims

1. A compound having the formula: 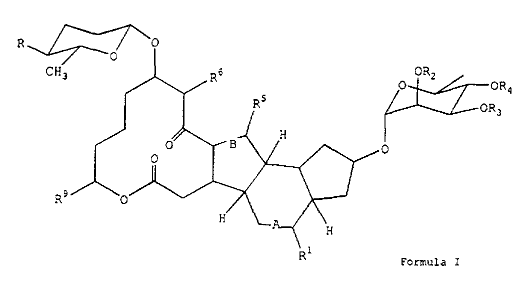 wherein A represents a single bond or an epoxide linkage;
B represents a single bond, a double bond, or an epoxide linkage;
R is 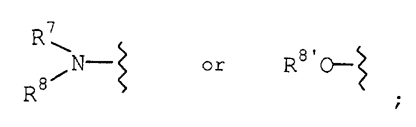 R¹ is hydrogen or methyl;
R², R³, and R⁴ are independently an alkyl having 1 to 4 carbon atoms, a haloalkyl having 1 to 4 carbon atoms, or alkanoyl having 1 to 4 carbon;
R⁵ is hydrogen, alkyl having 1 to 4 carbon atoms, alkyl amino having 1 to 4 carbon atoms, or alkyl hydroxyl amino having the formula 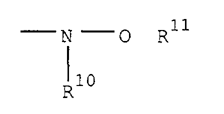 wherein R¹⁰ and R¹¹ are independently H or an alkyl having 1 to 4 carbon atoms or an alkanoyl having 1 to 5 carbon atoms;
R⁶ is hydrogen or methyl;
R⁷, R⁸, and R^{8'} are independently an alkyl having 1 to 4 carbon atoms, a haloalkyl having 1 to 4 carbon atoms, or alkanoyl having 1 to 4 carbon atoms; and
R⁹ is methyl or ethyl; or an acid addition salt thereof.

2. The compound of Claim 1 where the compound is an acid addition salt.

3. A compound of the formula: 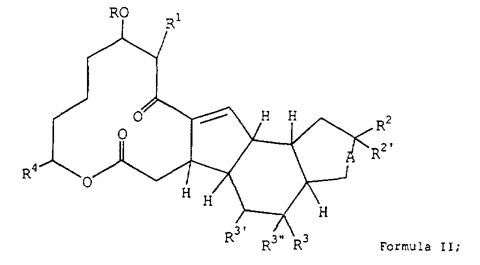 where R is 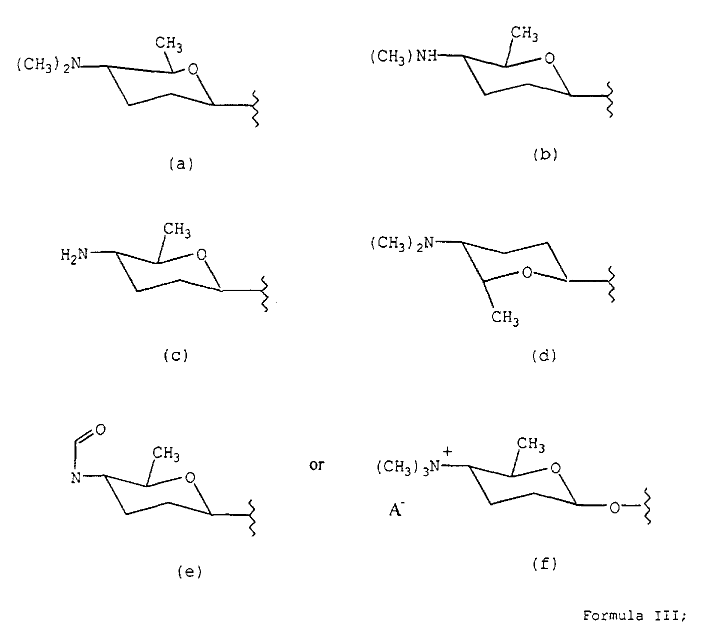 wherein R¹ and R³ are independently hydrogen or methyl; R⁴ is methyl or ethyl; and the following combinations are present: 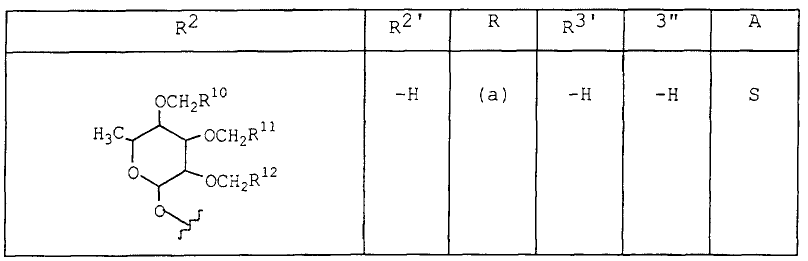 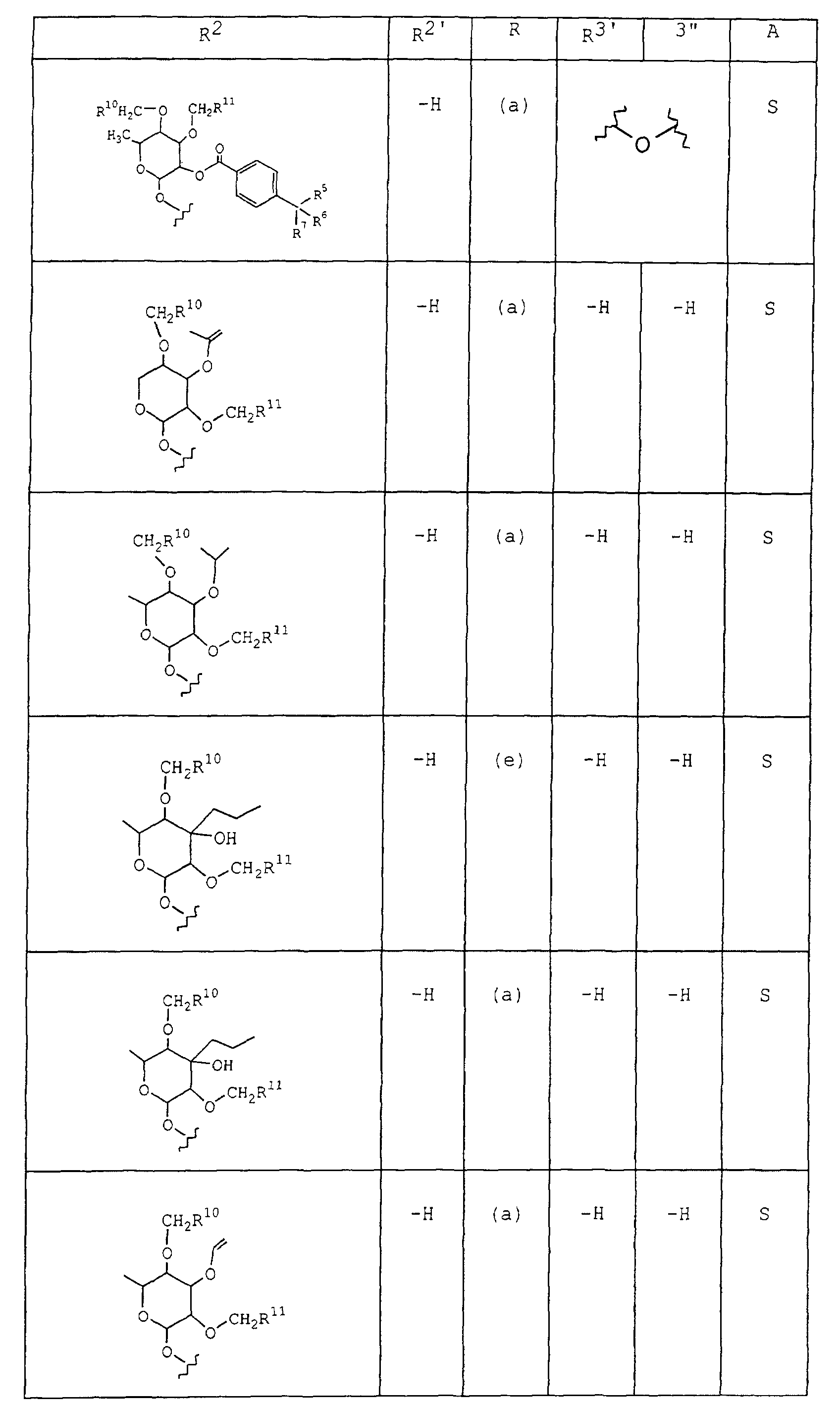 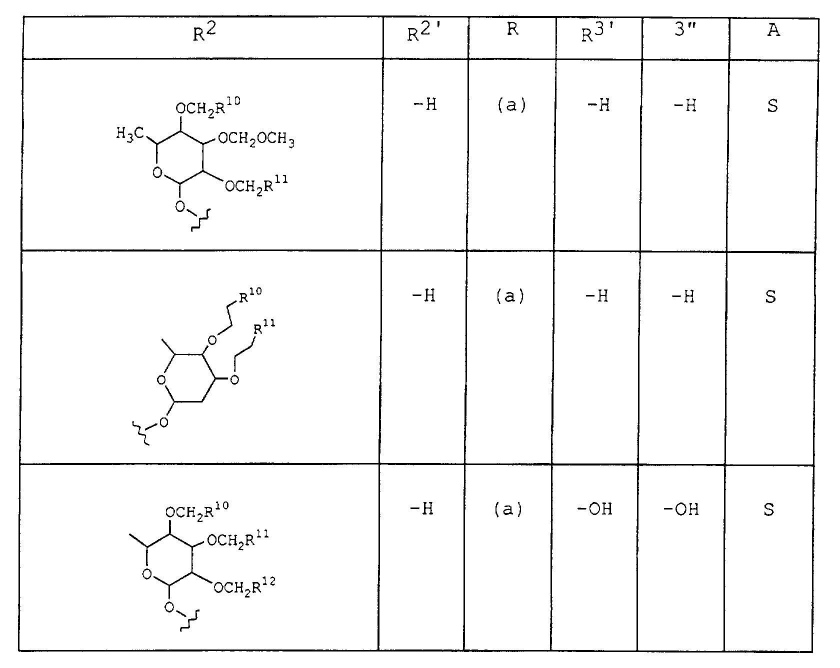 where R⁵, R⁶, R⁷, R⁸ and R⁹ are Cl, F, I, or Br;
R¹⁰, R¹¹, R¹², R¹³, R¹⁴ are independently H or an alkyl having 1 to 4 carbon atoms, or haloalkyl having 1 to 4 carbon atoms, where the halo is a halogen Br, Cl, F, or I;
A⁻ = Cl⁻, Br⁻, or I⁻;
S = single bond.

4. The compound of Claim 3, where the compound is an acid addition salt.

5. A compound of the formula (VI), or an acid addition salt thereof, 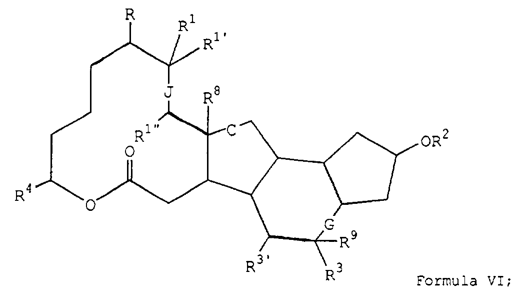 where:
R is specified in the table below, is H or is 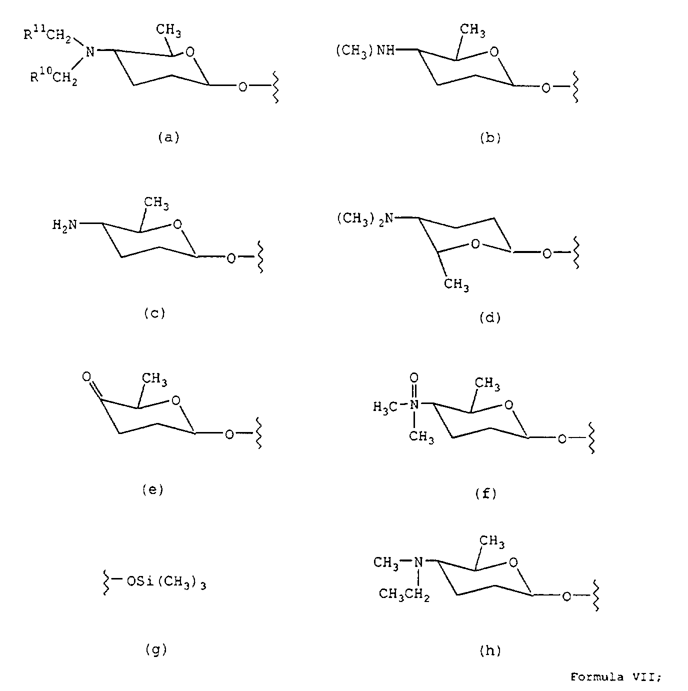 R² is specified in the table below, is H or is 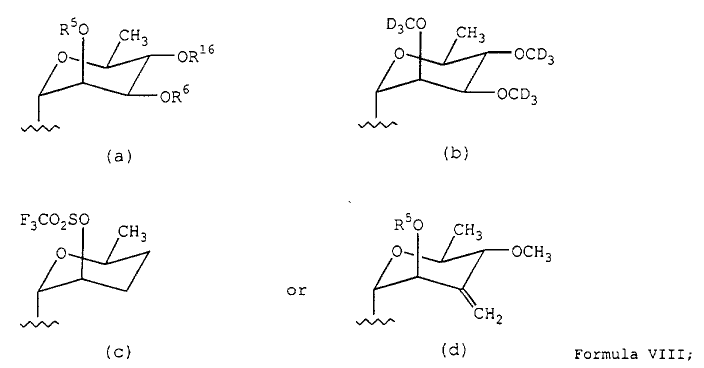 wherein R⁵, R⁶ and R¹⁶ are independently methyl, ethyl, propyl or butyl;
R¹ is H or methyl; R⁴ is methyl or ethyl; and the following combination is present:

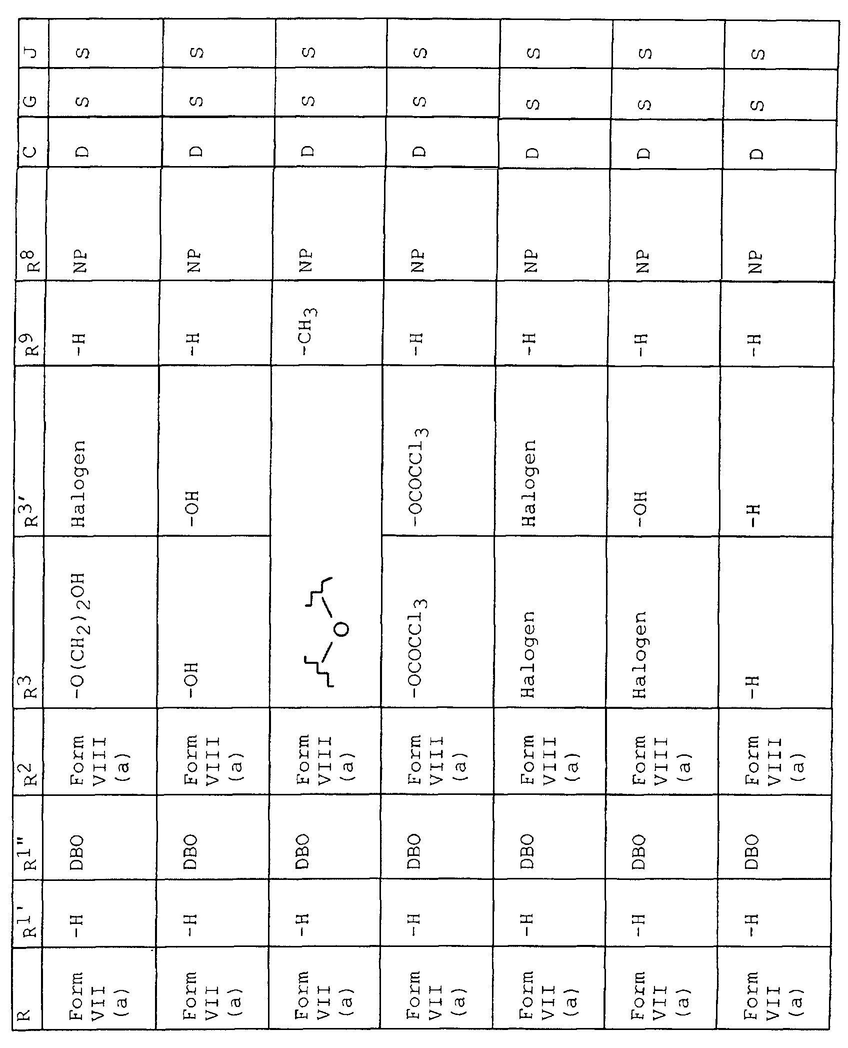

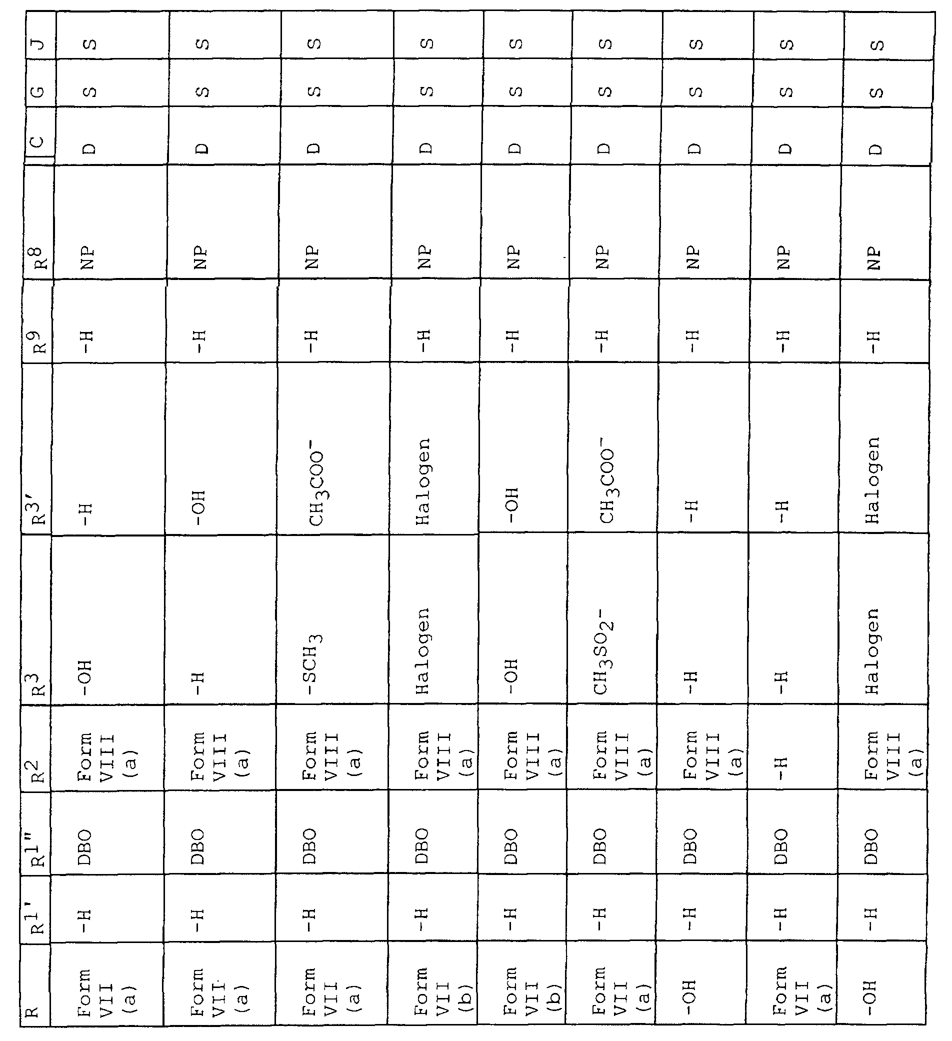

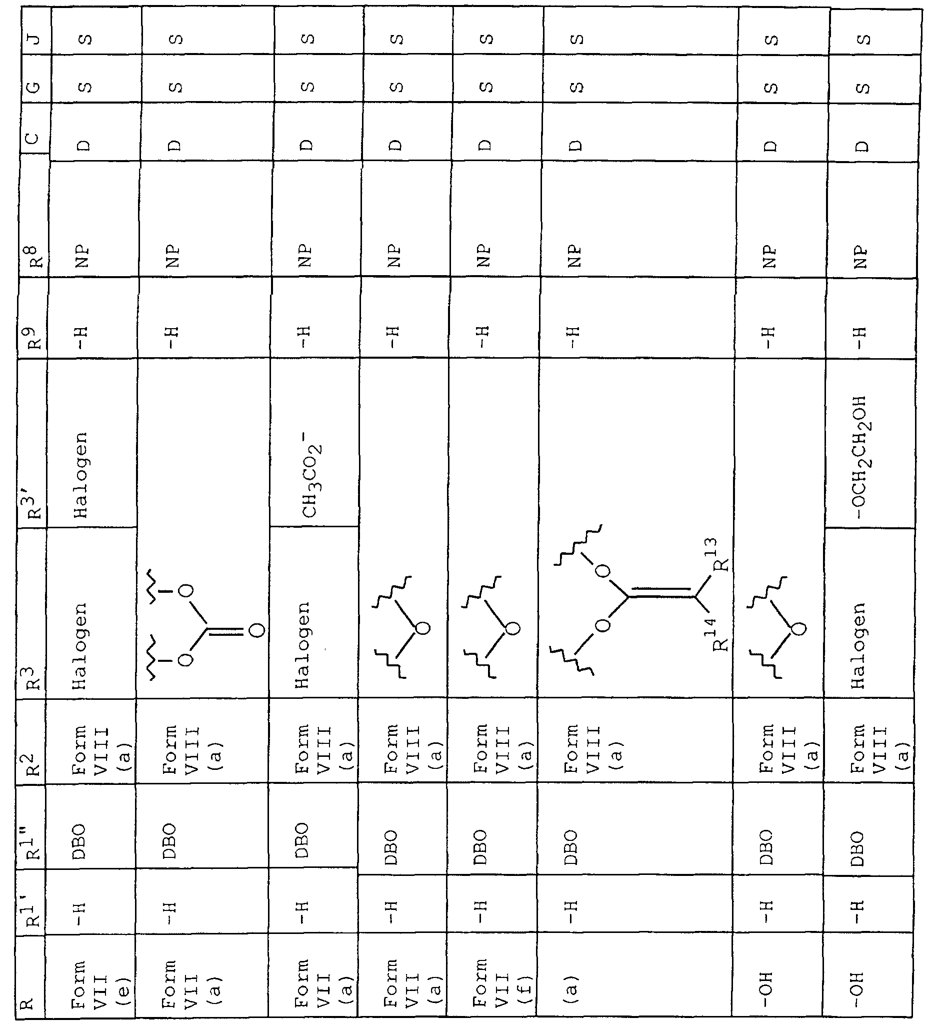

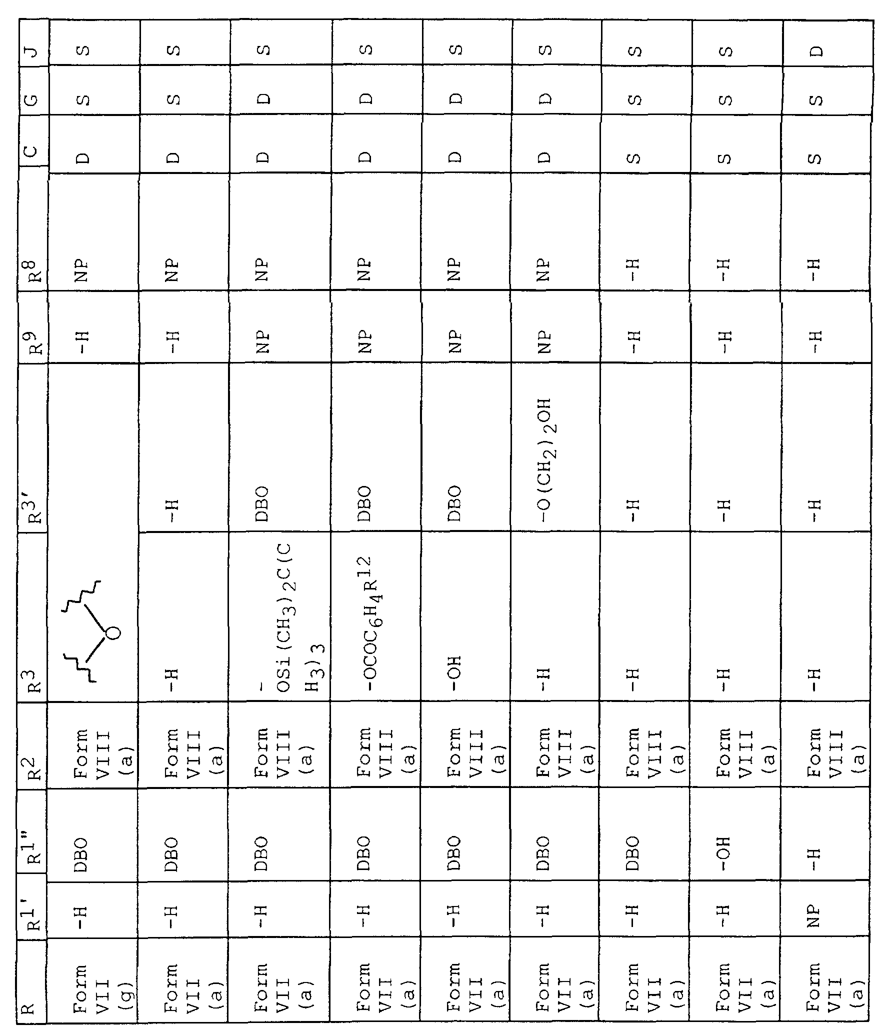

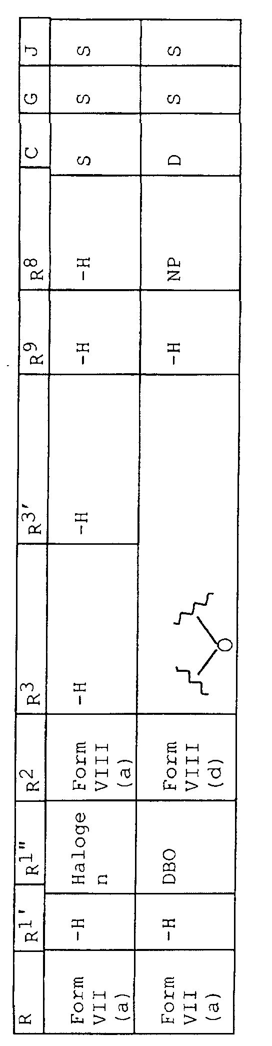

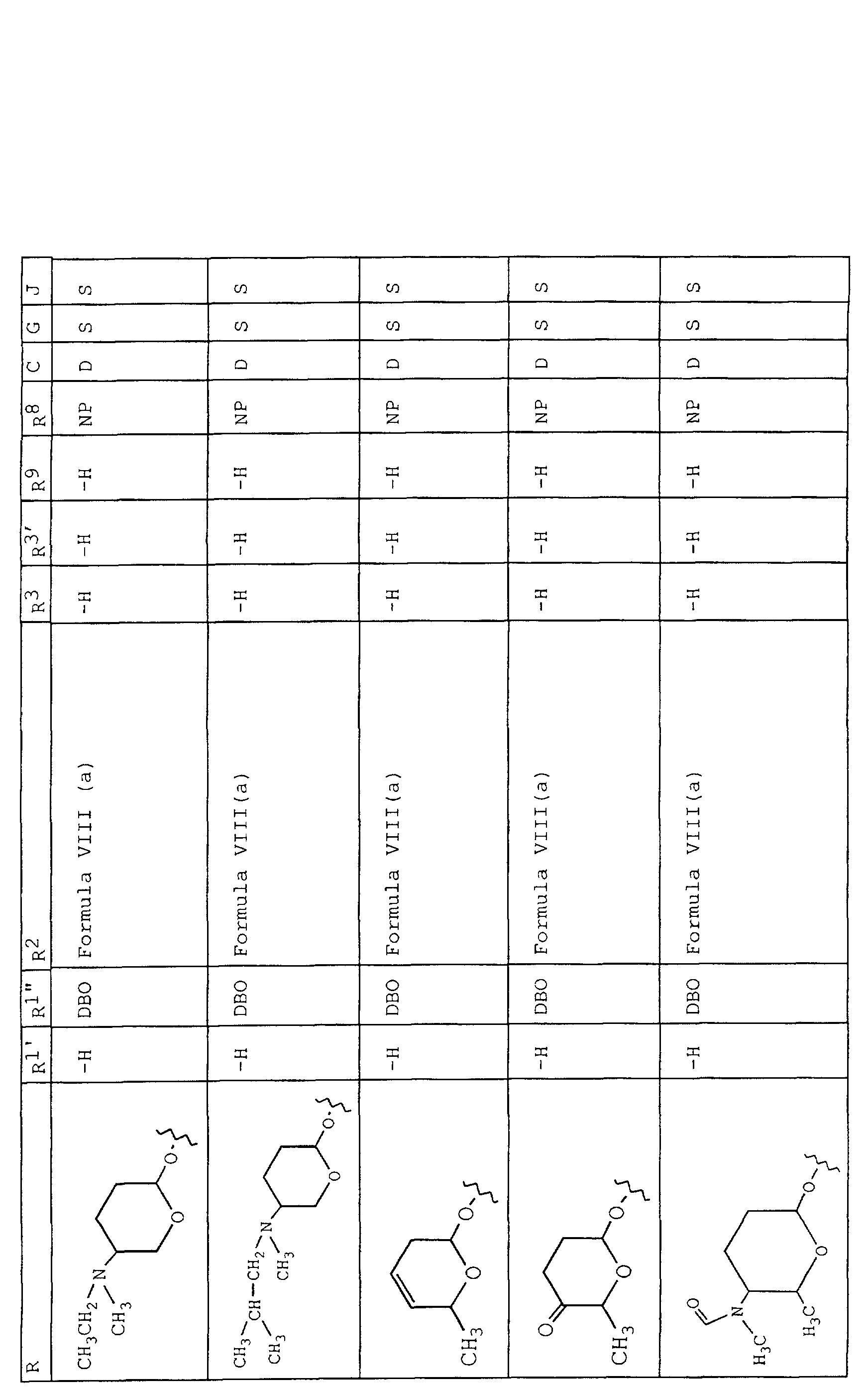

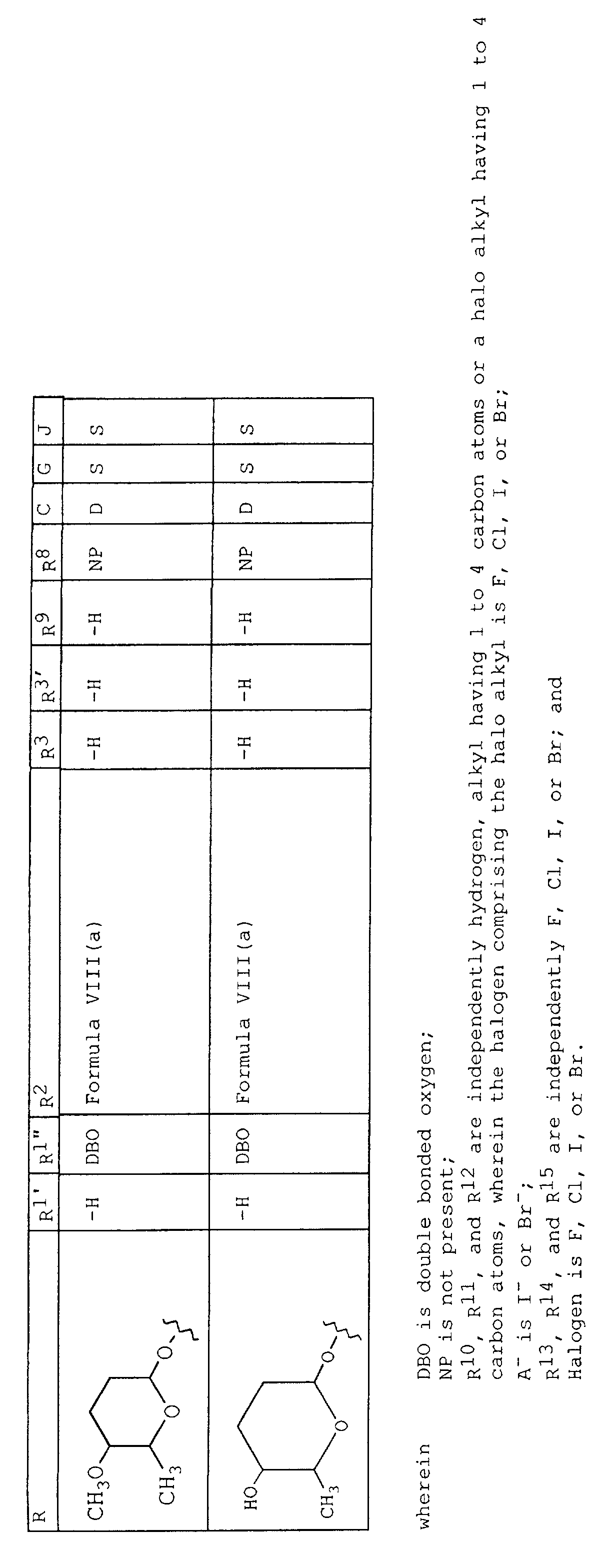

6. The compound of Claim 5 wherein the compound is an acid addition salt.

7. A compound of Claim 5 of the formula 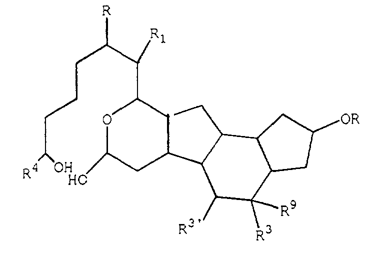 where
R is VII(a) 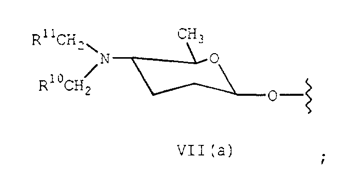 where R¹⁰ and R¹¹ are independently hydrogen, alkyl having 1 to 4 carbon atoms or a halo alkyl having 1 to 4 carbon atoms, wherein the halogen comprising the halo alkyl is F, Cl, I, or Br
R¹ is H or CH₃;
R² is VIII(a) 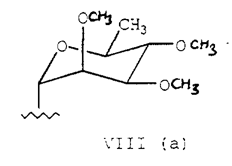 R³ and R^{3'} are H or CH₃;
R⁴ is CH₃ or C₂H₅; and
R⁹ is H or CH₃.

8. An insecticde, miticide, or ectoparasiticide composition comprising a compound of any one of claims 1, 3, or 5 as active ingredient.

9. A compound selected from the group consisting of:
(5,6-Dihydro Derivative of Compound 9-*O*-(2,3,4-Tri-*O*-ethyl-α-L-rhamnosyl) Spinosyn A 9-Psa;
(5*S*,6*R*)-Epoxy-2'-*O*-(p-trifluoromethyl)benzoyl Spinosyn Q; 5,6-Dihydro-3'-*O*-ethyl Spinosyn J;
5,6-Dihydro-3'-*O*-n-propyl Spinosyn J;
5,6-Dihydro-3'-*O*-n-butyl Spinosyn J;
5,6-Dihydrc-3'-*O*-n-propyl Spinosyn J Hemiglutarate salt;
5,6-Dihydro-2'-*O*-ethyl Spinosyn H;
5,6-Dihydro-2'-*O*-n-propyl Spinosyn H;
(5*R*,6*S*)-5,6-Epoxy-3'-*O*-*n*-propyl Spinosyn L(5*S*,6*R*)-5,6-epoxy-3'-*O*-*n*-propyl Spinosyn L;
(5*R*,6*S*)-3'-Deoxy-5,6-epoxy-3'-methylene Spinosyn J;
(5*S*,6*R*)-3'-Deoxy-5,6-epoxy-3'-methylene Spinosyn J;
5,6-dihydro-3'-*O*-n-propyl Spinosyn J;
5,6-Dihydro-3'-*O*-isopropyl Spinosyn J;
5,6-Dihydro-3'-epi-*N*-formyl-3'-propyl Spinosyn J;
5,6-dihydro-3'-epi-3'-propyl Spinosyn J;
5,6-Dihydro-3'-*O*-vinyl Spinosyn J;
(14S)-5,6,13,14-Tetrahydro-3'-*O*-*n*-propyl Spinosyn J;
5,6-Dihydro-3'-methoxymethyl-Spinosyn J;
4"-*N*-Desmethyl-4"-*N*-(2-fluoroethyl)-5,6-dihydro-3'-*O*-propyl Spinosyn J;
4"-*N*-Desmethyl-5,6-dihydro-3'-*O*-propyl Spinosyn J;
5,6-Dihydro-4"-N-(2-methyl-1-propyl) Spinosyn B;
3'',4''-dehydro-4"-desamino-5,6-dihydro Spinosyn A;
5,6-dihydro-N-formyl Spinosyn B;
4"-desamino-5,6-dihydro-4"-keto Spinosyn C;
4"-desamino-5,6-dihydro-4"(*S*)-hydroxy Spinosyn C;
4"-desamino-5,6-dihydro-4"(S)-methoxy Spinosyn C;
(14*S*)-5,6,13,14-Tetrahydro Spinosyn A;
(14*R*)-5,6,13,14-Tetrahydro Spinosyn A;
(14*S*)-5,6,13,14-Tetrahydro-3'-deoxy Spinosyn J;
(5*R*,6*R*)-5-(2-Hydroxyethoxy)-6-bromo Spinosyn A 17-Psa;
(5*S*,6*R*)-5,6-Epoxy-17-*O*-trimethylsilyl Spinosyn A 17-Psa;
(5*S*,6*R*)-5,6-Epoxy Spinosyn A 17-Psa;
(5*R*,6*R*)-5-(2-Hydroxyethoxy)-6-bromo Spinosyn A;
(5*S*,6*R*)-5,6-Dihydroxy-5,6-dihydro Spinosyn A;
(5*S*,6*R*)-Spinosyn A carbonate;
(5*S*,6*S*)-5-Acetoxy-6-bromo Spinosyn A(5*R*,6*R*)-5-Acetoxy-6-bromo-5,6-dihydro Spinosyn A;
5,6-Dibromo Spinosyn A 17-Psa;
5,6-Dihydro Spinosyn A;
(5*S*,6*R*)-5,6-Epoxy Spinosyn A
(5*R*,6*S*)-Epoxy Spinosyn A;
(5*S*,6*R*)-Epoxy Spinosyn D (5*R*,6*S*)-Epoxy Spinosyn D;
(5*S*,6*R*) Epoxy Spinosyn A, N-oxide;
(5*R*,6*S*) Epoxy Spinosyn A, N-oxide;
(5*S*,6*R*) Epoxy Spinosyn D, N-oxide;
(5*R*,6*S*) Epoxy Spinosyn D, N-oxide;
(5*R*,6*R*)-5,6-Dibromo-4"-keto Spinosyn A(5*R*,6*R*)-5,6-dibromo Spinosyn B;
5,6-*cis* Dihydroxy-5,6-dihydro Spinosyn B;
Dichloroketene acetal of (5*S*,6*R*)-5,6-dihydroxy Spinosyn A;
(5*S*,6*R*)-5,6-bis(trichloro-acetoxy) Spinosyn A;
(5*R*,6*R*)-5,6-Dibromo Spinosyn A;
(5*S*)-5,6-Dihydro-5-hydroxy Spinosyn A; (6*S*)-5,6-Dihydro-6-hydroxy Spinosyn A; (6*R*)-5,6-Dihydro-6-hydroxy Spinosyn A;
(5*R*,6*R*)-5-Acetoxy-6-thiomethoxy Spinosyn A;
(5*R*,6*R*)-5-Acetoxy-6-methylsulfonyl Spinosyn A;
(5*R*,6*R*)-6-Bromo-5-hydroxy Spinosyn J;
5,6-Dihydro Spinosyn A 9-Psa;
5,6-Dihydro Spinosyn A 17-Psa;
5,6-Dihydro Spinosyn J;
5,6-Dihydro Spinosyn H;
5,6-Dihydro Spinosyn A Hemiglutarate salt;
5,6-Dihydro Spinosyn B;
(5*S*,6*R*)-Epoxy-Spinosyn Q;
(5*S*,6*R*)-5,6-dihydro-5,6-dihydroxy Spinosyn A;
(5*R*,6*S*)-5,6-dihydro-5,6-dihydroxy Spinosyn A;
5,6-dihydro Spinosyn D;
9-(2H-5-R-Acetoxy-5,6-dihydro-6-S-methyl-2-a-pyranyl)-spinosyn A 9-pseudoaglycone;
5,6-Dihydro-9-(2-deoxy-3-*O*,4-*O*-diethyl-1-α-rhamnosyl)-spinosyn A 9-pseudoaglycone;
5,6-Dihydro-9-(2-deoxy-3-*O*,4-*O*-di-n-propyl-1-α-rhamnosyl)-spinosyn A 9-pseudoaglycone.

## Patentansprüche

1. Verbindung mit der Formel: 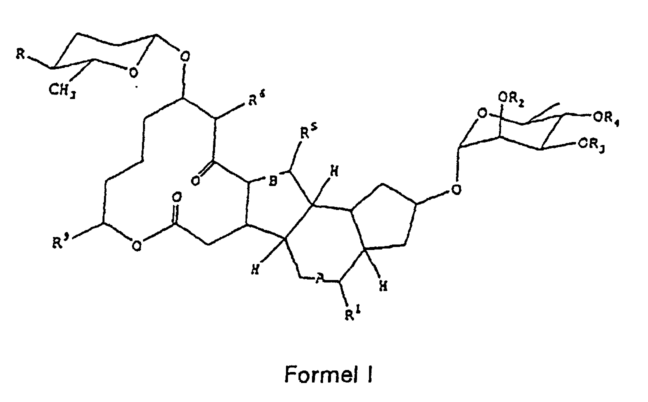 worin A für eine Einfachbindung oder eine Epoxid-Bindung steht;
B für eine Einfachbindung, eine Doppelbindung oder eine Epoxid-Bindung steht; R 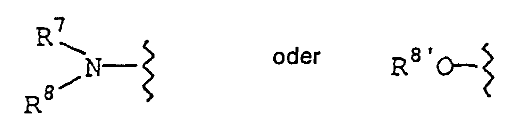 ist;
R¹ Wasserstoff oder Methyl ist;
R², R³ und R⁴ jeweils unabhängig ein Alkyl mit 1 bis 4 Kohlenstoff-Atomen, ein Halogenalkyl mit 1 bis 4 Kohlenstoff-Atomen oder ein Alkanoyl mit 1 bis 4 Kohlenstoff-Atomen sind;
R⁵ Wasserstoff, ein Alkyl mit 1 bis 4 Kohlenstoff-Atomen, ein Alkylamin mit 1 bis 4 Kohlenstoff-Atomen oder ein Alkylhydroxylamin mit der Formel 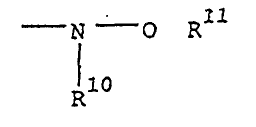 ist,
worin R¹⁰ und R¹¹ jeweils unabhängig H oder ein Alkyl mit 1 bis 4 Kohlenstoff-Atomen oder ein Alkanoyl mit 1 bis 5 Kohlenstoff-Atomen sind;
R⁶ Wasserstoff oder Methyl ist;
R⁷, R⁸ und R^{8'} jeweils unabhängig ein Alkyl mit 1 bis 4 Kohlenstoff-Atomen, ein Halogenalkyl mit 1 bis 4 Kohlenstoff-Atomen oder ein Alkanoyl mit 1 bis 4 Kohlenstoff-Atomen sind und
R⁹ Methyl oder Ethyl oder ein Säureadditionssalz davon ist.

2. Verbindung nach Anspruch 1, worin die Verbindung ein Säureadditionssalz ist.

3. Verbindung mit der Formel: 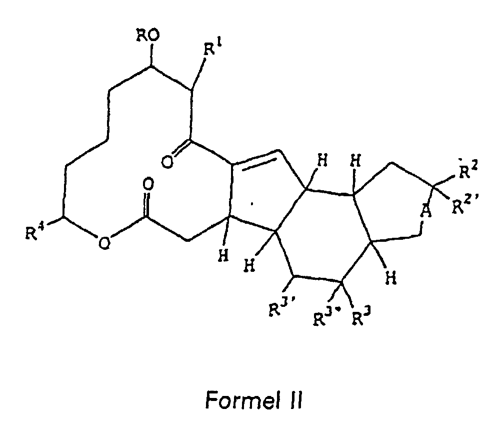 worin R 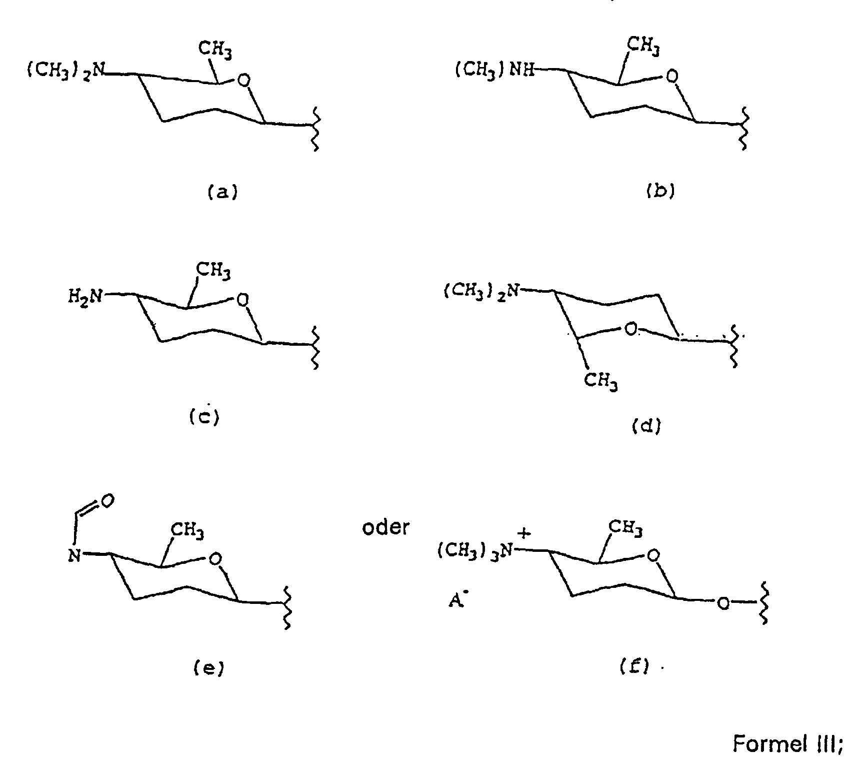 ist,
worin R¹ und R³ jeweils unabhängig Wasserstoff oder Methyl sind, R⁴ Methyl oder Ethyl ist, und die folgenden Kombinationen vorliegen: 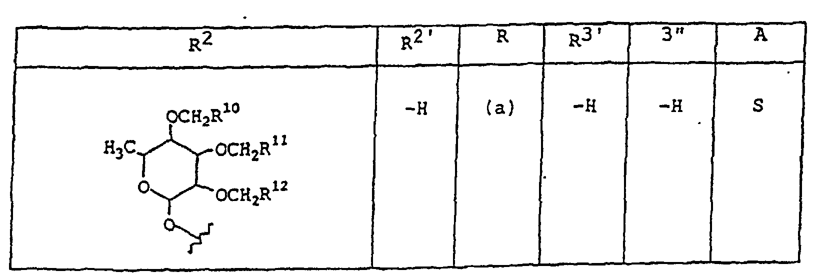 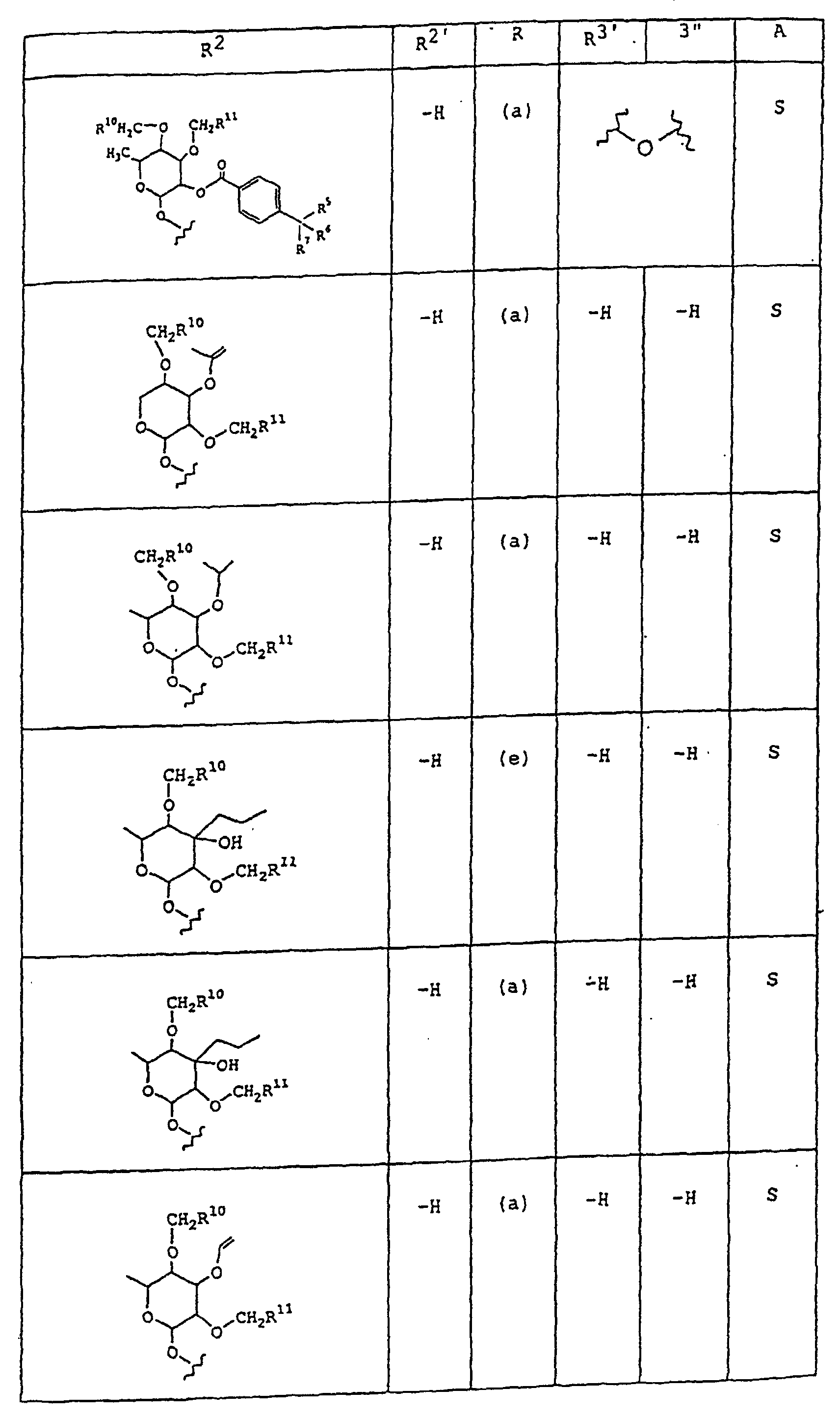 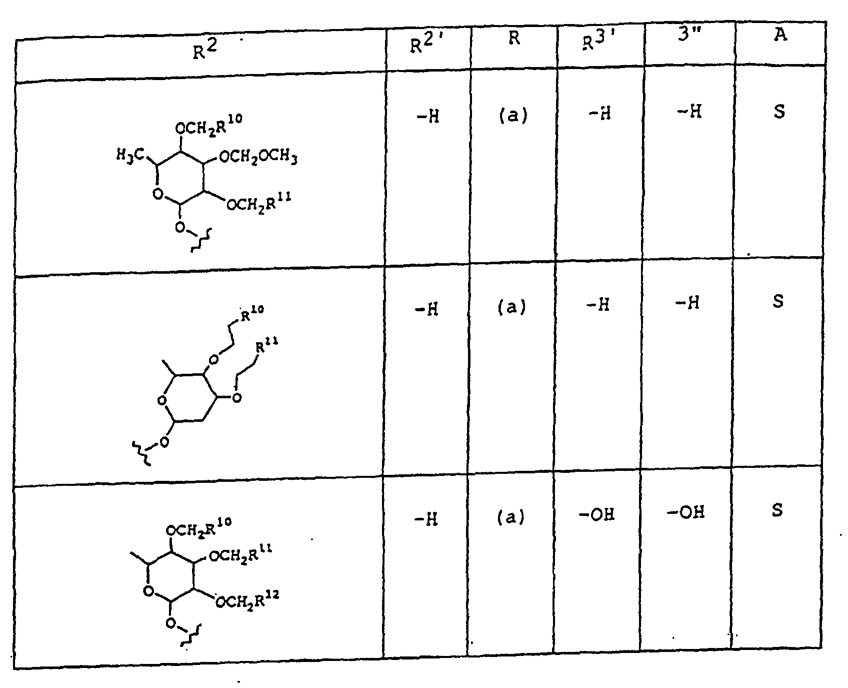 worin R⁵, R⁶, R⁷, R⁸ und R⁹ Cl, F, I oder Br sind;
R¹⁰, R¹¹, R¹², R¹³, R¹⁴ jeweils unabhängig H oder ein Alkyl mit 1 bis 4 Kohlenstoff-Atomen oder ein Halogenalkyl mit 1 bis 4 Kohlenstoff-Atomen sind;
worin das Halogen Br, Cl, F oder I ist;
A⁻ = Cl⁻, Br⁻ oder I⁻;
S = Einfachbindung.

4. Verbindung nach Anspruch 3, worin die Verbindung ein Säureadditionssalz ist.

5. Verbindung mit der Formel (VI) oder ein Säureadditionssalz davon, 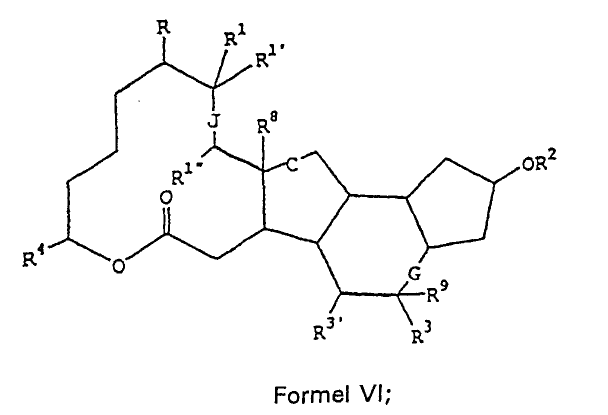 worin:
R in der Tabelle unten aufgeführt ist, H oder 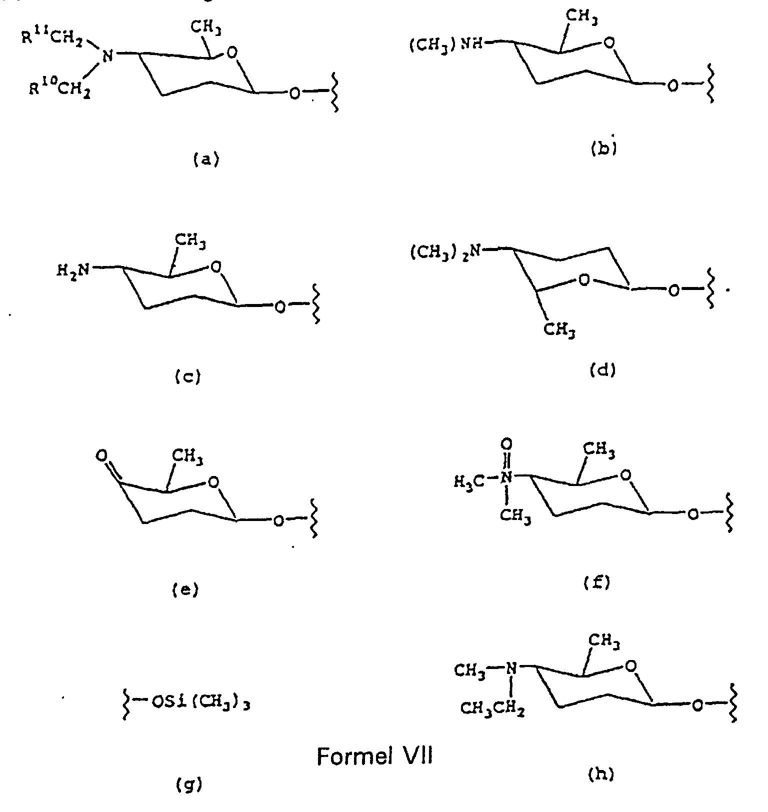
ist;
R² in der Tabelle unten aufgeführt ist, H oder 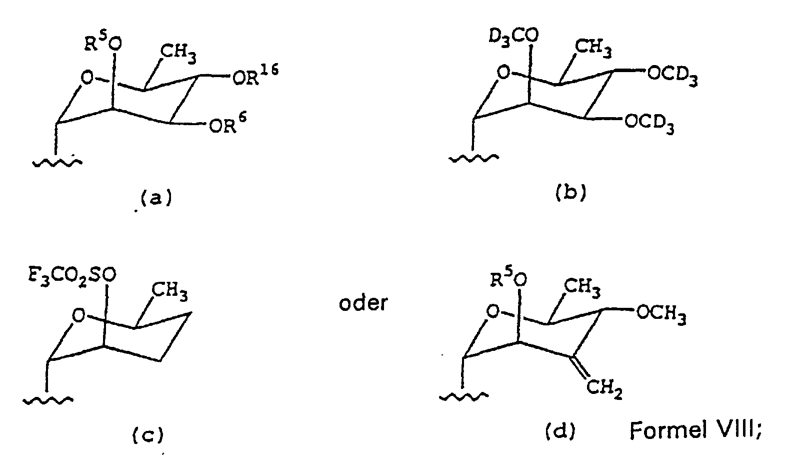 ist;
worin R⁵, R⁶, und R¹⁶ jeweils unabhängig Methyl, Ethyl, Propyl oder Buthyl sind; R¹ H oder Methyl ist;
R⁴ Methyl oder Ethyl ist,
und die folgende Kombination vorliegt: 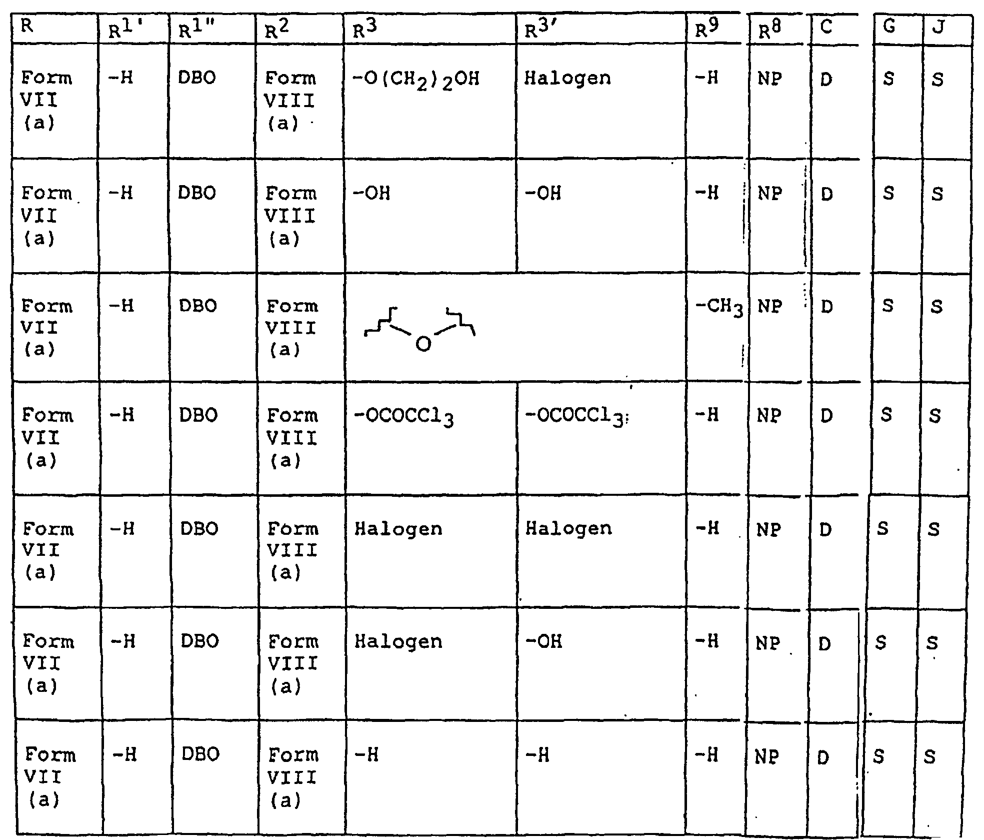 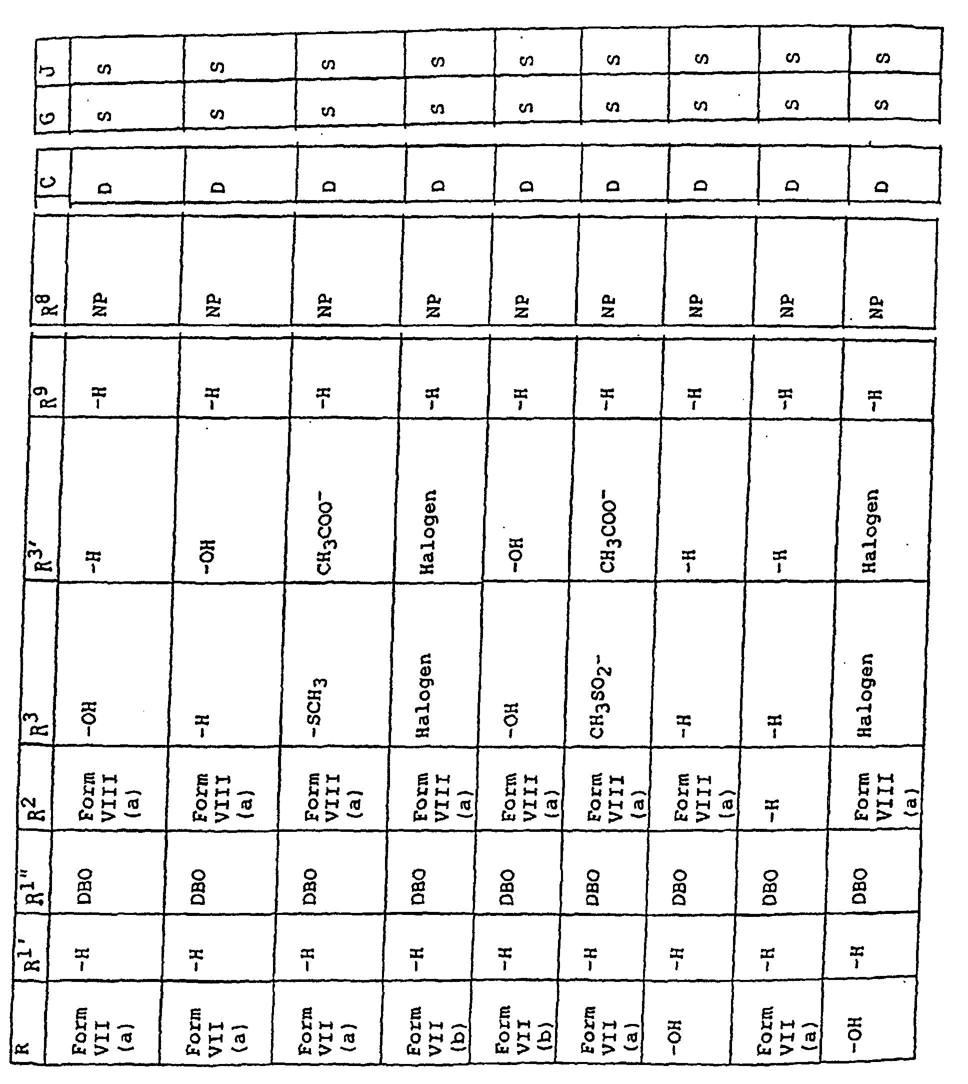 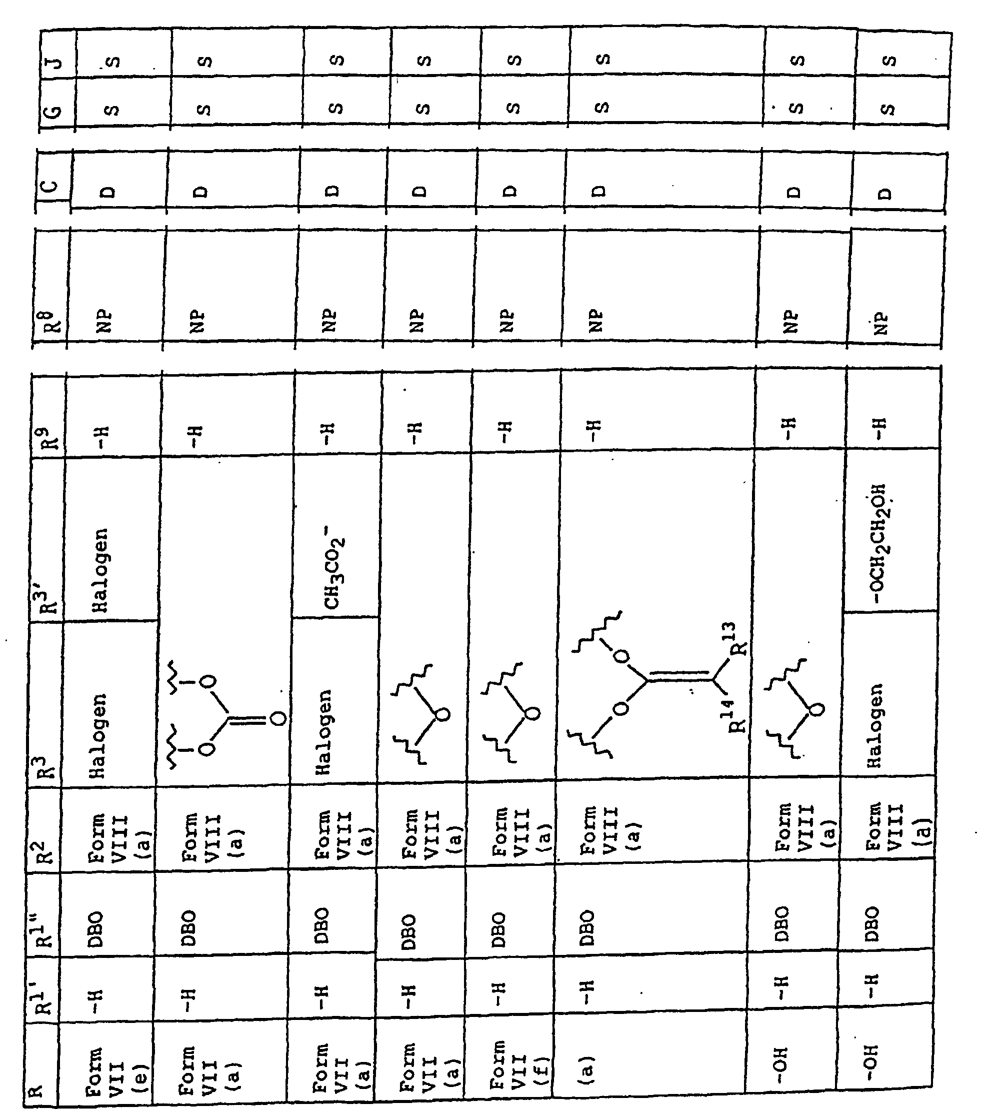 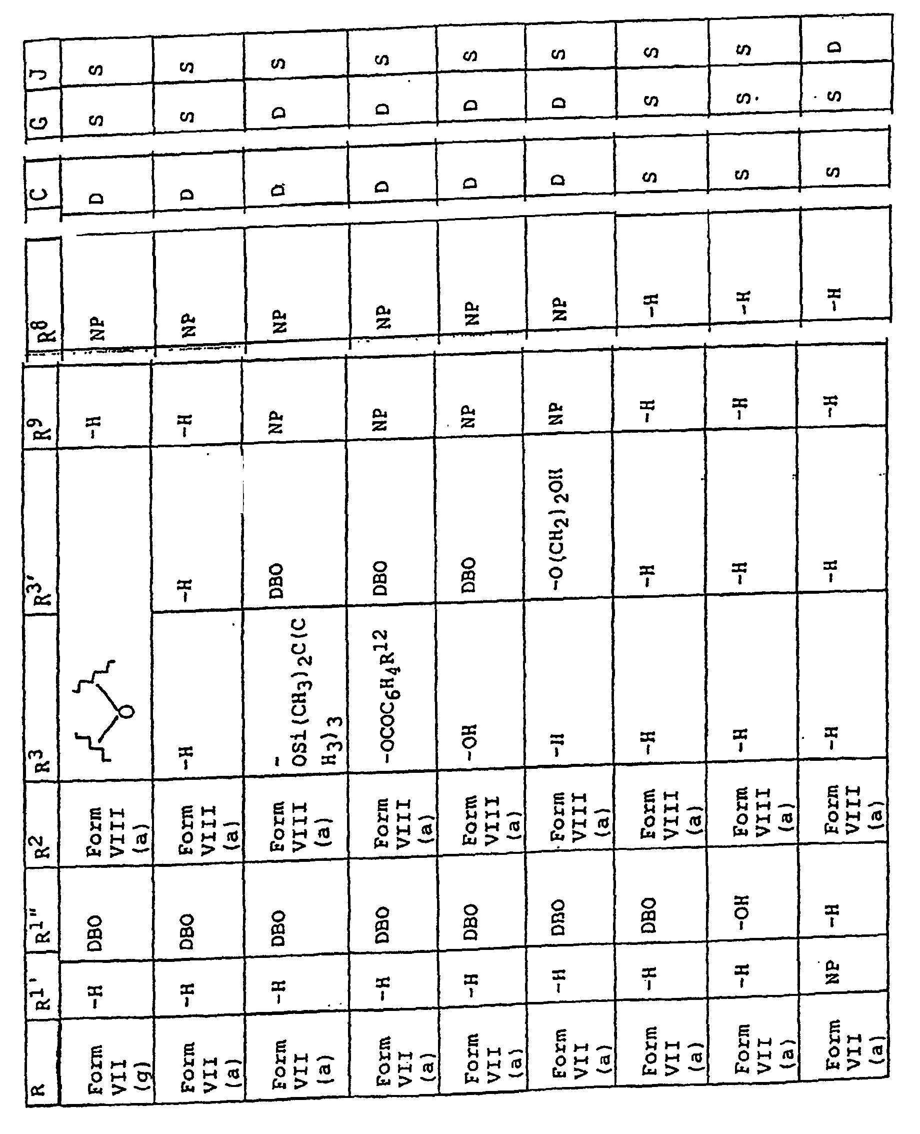 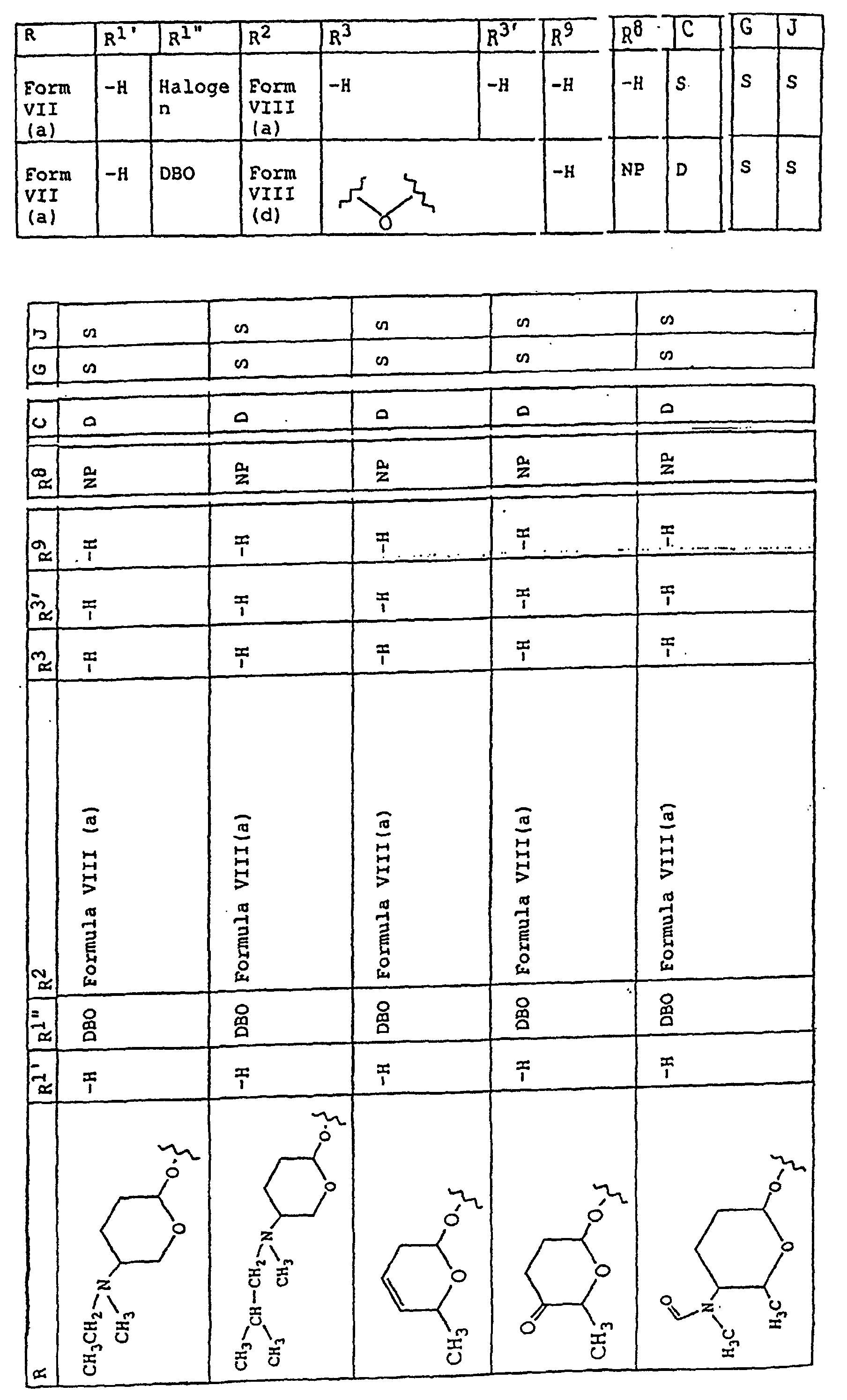 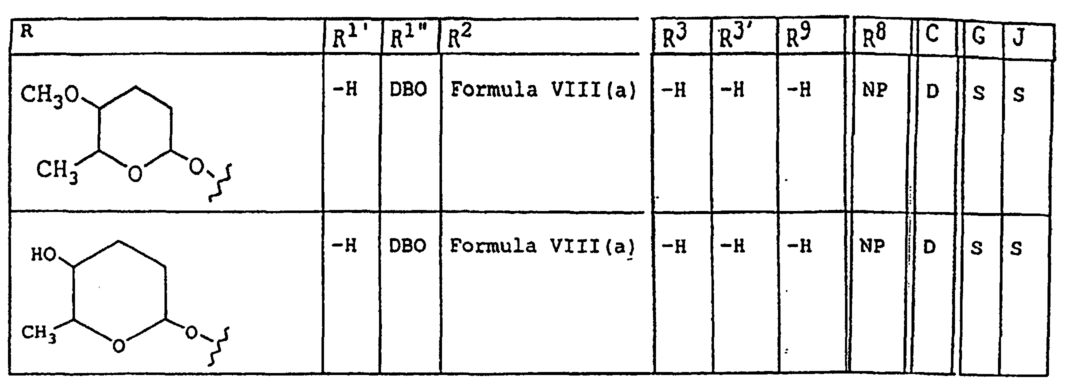 worin
DBO doppelt gebundener Sauerstoff ist;
NP nicht vorhanden bedeutet;
R¹⁰, R¹¹ und R¹² jeweils unabhängig Wasserstoff, ein Alkyl mit 1 bis 4 Kohlenstoffatomen oder ein Halogenalkyl mit 1 bis 4 Kohlenstoffatomen sind,
worin das Halogen, das das Halogenalkyl umfasst, F, Cl, I oder Br ist;
A⁻I⁻ oder Br⁻ ist;
R¹³, R¹⁴ und R¹⁵ jeweils unabhängig F, CI, I oder Br sind und
Halogen F, Cl, I oder Br ist.

6. Verbindung nach Anspruch 5, worin die Verbindung ein Säureadditionssalz ist.

7. Verbindung nach Anspruch 5 mit der Formel 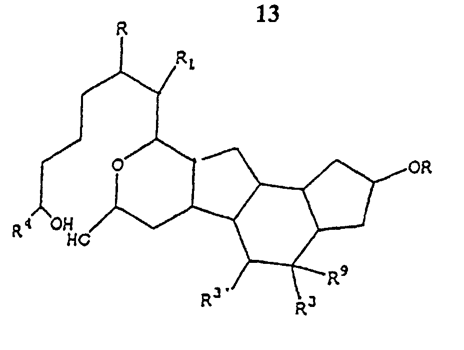 worin
R VII(a) ist, 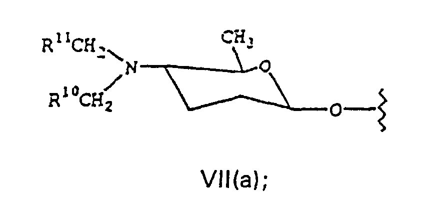 worin
R¹⁰ und R¹¹ jeweils unabhängig Wasserstoff, ein Alkyl mit 1 bis 4 Kohlenstoffatomen oder ein Halogenalkyl mit 1 bis 4 Kohlenstoffatomen sind,
worin das Halogen, das das Halogenalkyl umfasst, F, Cl, I oder Br ist,
R¹ H oder CH₃ ist;
R² VIII(a) ist, 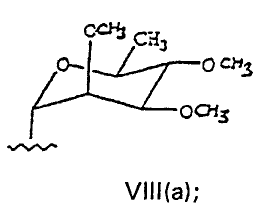 R³ und R^{3'} H oder CH₃ sind;
R⁴ CH₃ oder C₂H₅ ist und
R⁹ H oder CH₃ ist.

8. Ein Insektizid, ein Mitizid oder eine Ektoparasiten tötende Zusammensetzung, umfassend als Wirkstoff eine Verbindung nach einem der Ansprüche 1, 3, oder 5.

9. Verbindung, ausgewählt aus der Gruppe bestehend aus:
einem 5,6-Dihydro-Derivat der Verbindung 9-O-(2,3,4-tri-O-ethyl-α-L-rhamnosyl)-Spinosyn A 9-Psa;
(5S,6R)-Epoxy-2'-O-(p-trifuormethyl)benzoyl-Spinosyn Q;
5,6-Dihydro-3'-O-ethyl-Spinosyn J;
5,6-Dihydro-3'-O-n-propyl-Spinosyn J;
5,6-Dihydro-3'-O-n-butyl-Spinosyn J;
5,6-Dihydro-3'-O-n-propyl-Spinosyn J Hemiglutaratsalz;
5,6-Dihydro-2'-O-ethyl-Spinosyn H;
5,6-Dihydro-2'-O-n-propyl-Spinosyn H;
(5R,6S)-5,6-Epoxy-3'-O-n-propyl-Spinosyn L
(5S,6R)-5,6-Epoxy-3'-O-n-propyl-Spinosyn L;
(5R,6S)-3'-Desoxy-5,6-epoxy-3'-methylen-Spinosyn J;
(5S,6R)-3'-Desoxy-5,6-epoxy-3'-methylen-Spinosyn J;
5,6-Dihydro-3'-O-n-propyl-Spingosyn J;
5,6-Dihydro-3'-O-isopropyl-Spingosyn J;
5,6-Dihydro-3'-epi-N-formyl-3'-propyl-Spinosyn J;
5,6-Dihydro-3'-epi-3'-propyl-Spinosyn J;
5,6-Dihydro-3'-O-vinyl-Spinosyn J;
(14S)-5,6,13,14-Tetrahydro-3'-O-n-propyl-Spinosyn J;
5,6-Dihydro-3'-methoxymethyl-Spinosyn J;
4"-N-Desmethyl-4"-N-(2-fluorethyl)-5,6-dihydro-3'-O-propyl-Spinosyn J;
4"-N-Desmethyl-5,6-dihydro-3'-O-propyl-Spinosyn J;
5,6-Dihydro-4"-N-(2-methyl-1-propyl)-Spinosyn B;
3'',4''-Dehydro-4''-desamino-5,6-dihydro-Spinosyn A;
5,6-Dihydro-N-formyl-Spinosyn B;
4''-Desamino-5,6-dihydro-4''-keto-Spinosyn C;
4''-Desamino-5,6-dihydro-4''-(S)-hydroxy-Spinosyn C;
4''-Desamino-5,6-dihydro-4''-(S)-methoxy-Spinosyn C;
(14S)-5,6,13,14-Tetrahydro-Spinosyn A;
(14R)-5, 6,13,14-Tetrahydro-Spinosyn A;
(14S)-5,6,13,14-Tetrahydro-3'-desoxy-Spinosyn J;
(5S,6R)-5-(2-Hydroxyethoxy)-6-brom-Spinosyn A 17-Psa;
(5S,6R)-5,6-Epoxy-17-O-trimethylsilyl-Spinosyn A 17-Psa;
(5S,6R)-5,6-Epoxy-Spinosyn A 17-Psa;
(5R,6S)-5-(2-Hydroxyethoxy)-6-brom-Spinosyn A;
(5S,6R)-5,6-Dihydroxy-5,6-dihydro-Spinosyn A;
(5S,6R)-Spinosyn A-carbonat;
(5S,6R)-5-Acetoxy-6-brom-Spinosyn A;
(5R,6R)-5-Acetoxy-6-brom-5,6-dihydro-Spinosyn A;
5,6-Dibrom-Spinosyn A 17-Psa;
5,6-Dihydro-Spinosyn A;
(5S,6R)-5,6-Epoxy-Spinosyn A
(5R,6S)-Epoxy-Spinosyn A;
(5S,6R)-Epoxy-Spinosyn D;
(5R,6S)-Epoxy-Spinosyn D;
(5S,6R)-Epoxy-Spinosyn A, N-Oxid;
(5R,6S)-Epoxy-Spinosyn A, N-Oxid;
(5S,6R)-Epoxy-Spinosyn D, N-Oxid;
(5R,6S)-Epoxy-Spinosyn D, N-Oxid;
(5R,6S)-5,6-Dibrom-4''-keto-Spinosyn A;
(5R,6R)-5,6-Dibrom-Spinosyn B;
5,6-cis-Dihydroxy-5,6-dihydro-Spinosyn B;
Dichlorketenacetal von (5S,6R)-5,6-Dihydroxy-Spinosyn A;
(5S,6R)-5,6-bis(trichloracetoxy)-Spinosyn A;
(5R,6R)-5,6-Dibrom-Spinosyn A;
(5S)-5,6-Dihydro-5-hydroxy-Spinosyn A;
(6S)-5,6-Dihydro-6-hydroxy-Spinosyn A;
(6R)-5,6-Dihydro-6-hydroxy-Spinosyn A;
(5R,6R)-5-Acetoxy-6-thiomethoxy-Spinosyn A;
(5R,6R)-5-Acetoxy-6-methylsulfonyl-Spinosyn A;
(5R,6R)-6-Brom-5-hydroxy-Spinosyn J;
5,6-Dihydro-Spinosyn A 9-Psa;
5,6-Dihydro-Spinosyn A 17-Psa;
5,6-Dihydro-Spinosyn J;
5,6-Dihydro-Spinosyn H;
5,6-Dihydro-Spinosyn A Hemiglutarat-Salz;
5,6-Dihydro-Spinosyn B;
(5S,6R)-Epoxy-Spinosyn Q;
(5S,6R)-5,6-Dihydro-5,6-dihydroxy-Spinosyn A;
(5R,6S)-5,6-Dihydro-5,6-dihydroxy-Spinosyn A;
5,6-Dihydro-Spinosyn D;
9-(2H-5-R-Acetoxy-5,6-dihydro-6-S-methyl-2-a-pyranyl)-Spinosyn A 9-Pseudoaglycon;
5,6-Dihydro-9-(2-desoxy-3-O,4-O-diethyl-1-α-rhamnosyl)-Spinosyn A 9-Pseudoaglycon;
5,6-Dihydro-9-(2-desoxy-3-O,4-O-di-n-propyl-1-α-rhamnosyl)-Spinosyn A 9-Pseudoaglycon.

## Revendications

1. Composé de formule 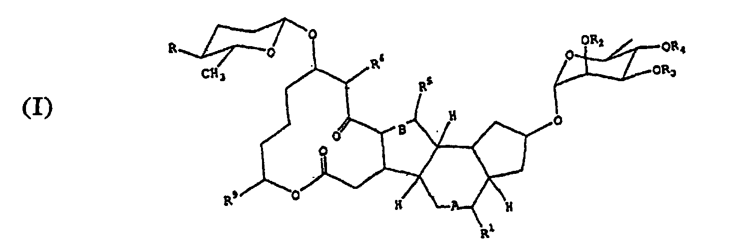 dans laquelle A représente une simple liaison ou une liaison époxyde, B représente une simple liaison, une double liaison ou une liaison époxyde, R représente un groupe 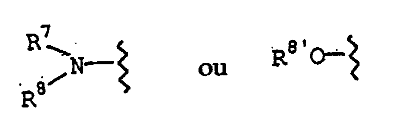 R¹ représente un atome d'hydrogène ou un groupe méthyle,
R², R³ et R⁴ représentent indépendamment chacun un groupe alkyle en C₁₋₄, halogénoalkyle en C₁₋₄ ou alcanoyle en C₁₋₄, R⁵ représente un atome d'hydrogène, un groupe alkyle en C₁₋₄, alkylamino en C₁₋₄ ou alkylhydroxylamino de formule 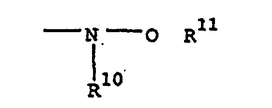 dans laquelle R¹⁰ et R¹¹ représentent indépendamment chacun un atome d'hydrogène ou un groupe alkyle en C₁₋₄ ou alcanoyle en C₁₋₅,
R⁶ représente un atome d'hydrogène ou un groupe méthyle,
R⁷, R⁸ et R^{8'} représentent indépendamment chacun un groupe alkyle en C₁₋₄, un groupe halogénoalkyle en C₁₋₄ ou alcanoyle en C₁₋₄, et
R⁹ représente un groupe méthyle ou éthyle,
ou un sel d'addition d'acide d'un tel composé.

2. Composé selon la revendication 1, dans lequel le composé est un sel d'addition d'acide.

3. Composé de formule 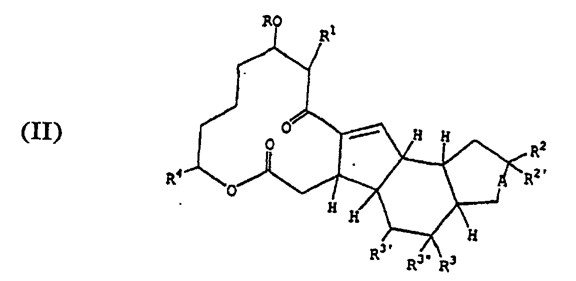 dans laquelle R représente un groupe 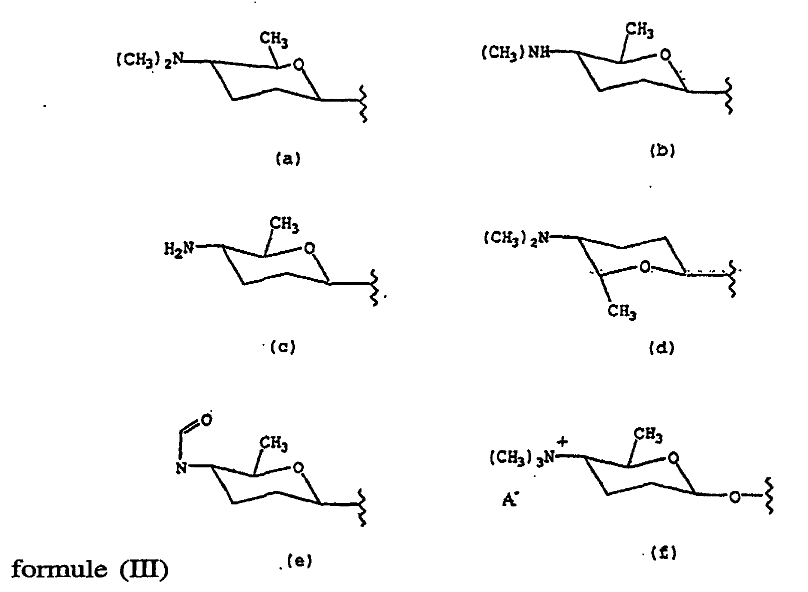 ou R¹ et R³ représentent indépendamment l'un de l'autre un atome d'hydrogène ou un groupe méthyle, R⁴ représente un groupe méthyle ou éthyle, et la signification des autres symboles correspond aux combinaisons suivantes : 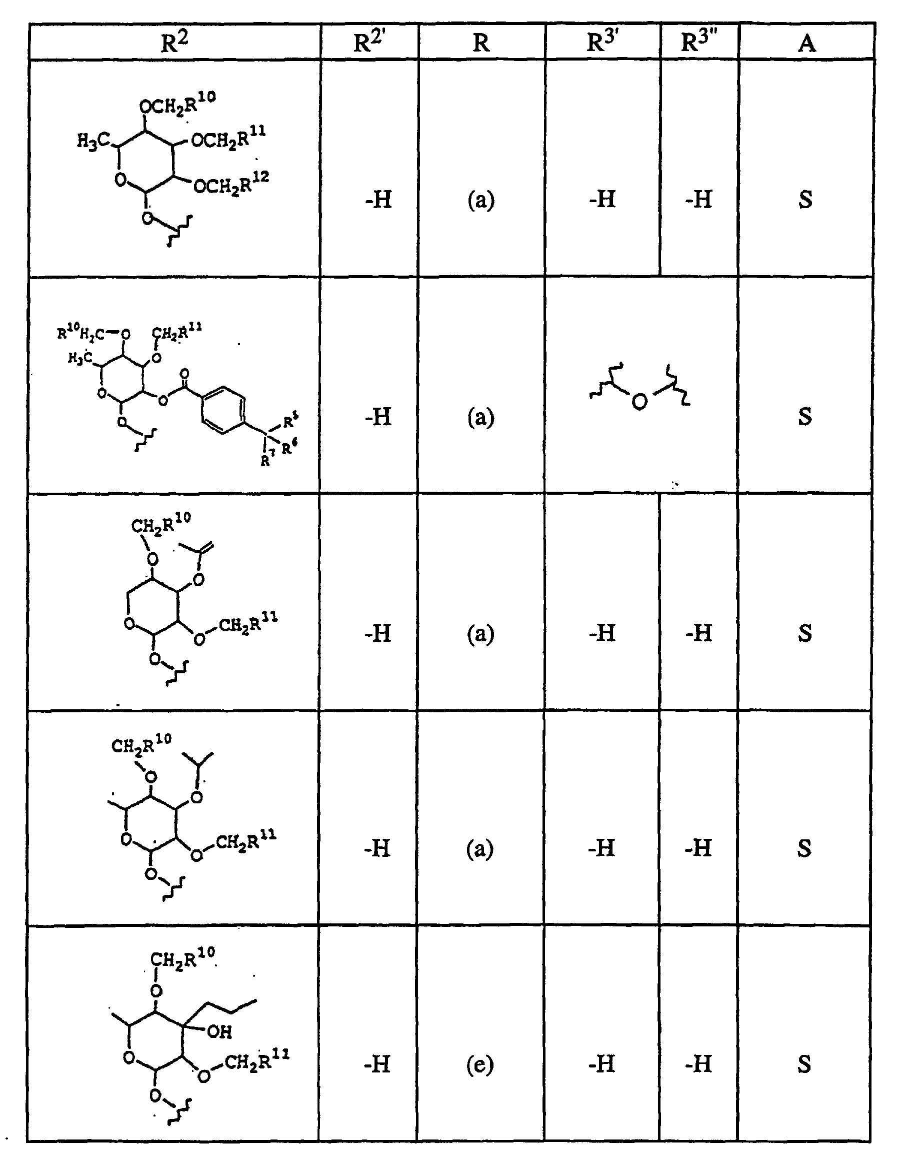 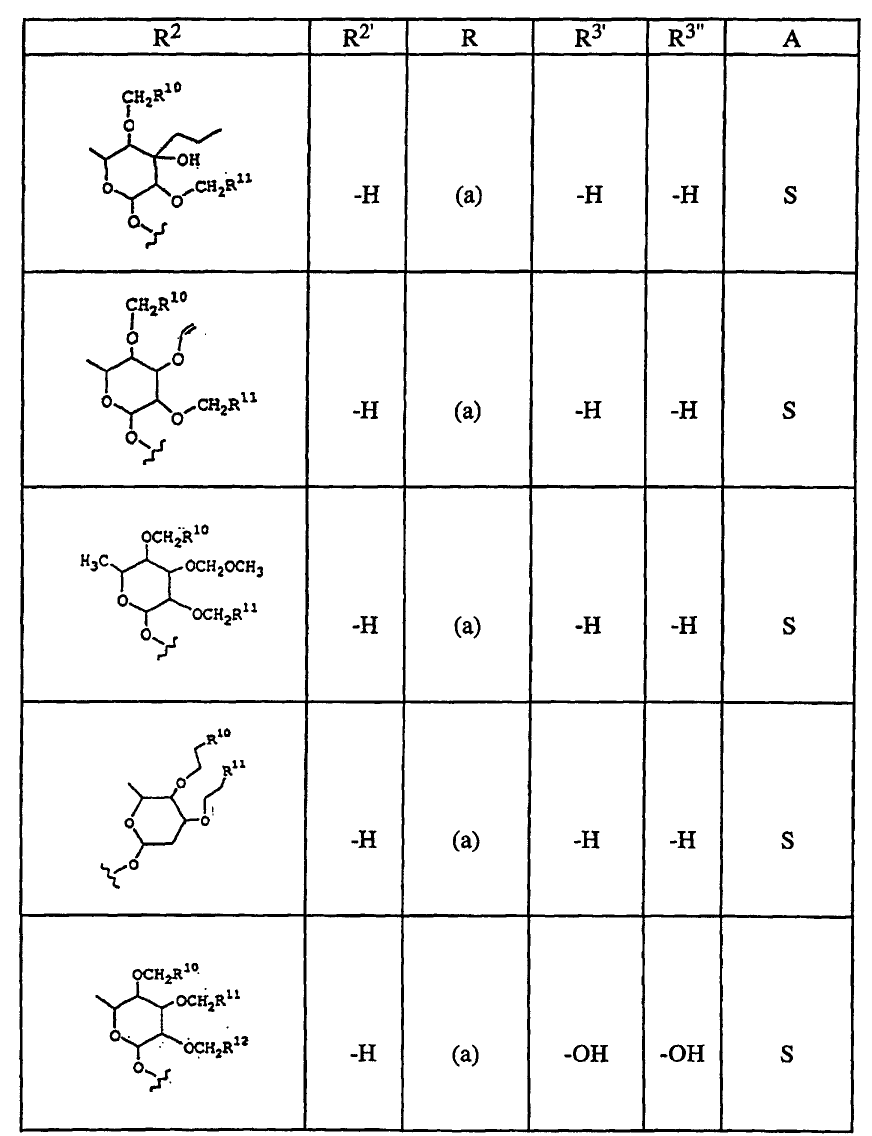 où R⁵, R⁶, R⁷, R⁸ et R⁹ représentent chacun indépendamment un atome Cl, F, I ou Br,
R¹⁰, R¹¹ R¹², R¹³ et R¹⁴ représentent chacun indépendamment un atome d'hydrogène ou un groupe alkyle en C₁₋₄, halogénoalkyle en C₁₋₄, où halogéno signifie un atome de brome, de chlore, de fluor ou d'iode, A⁻ = Cl⁻, Br⁻ ou I⁻,
S = simple liaison.

4. Composé selon la revendication 3 qui est un sel d'addition d'acide.

5. Composé de formule (VI) ou sel d'addition d'acide d'un tel composé : 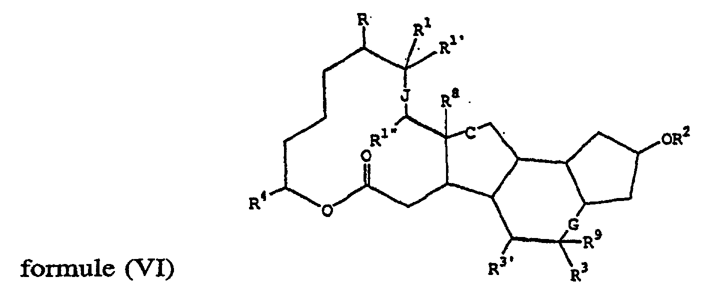 dans laquelle R a la signification indiquée dans le tableau ci-dessous, représente un atome d'hydrogène ou un groupe 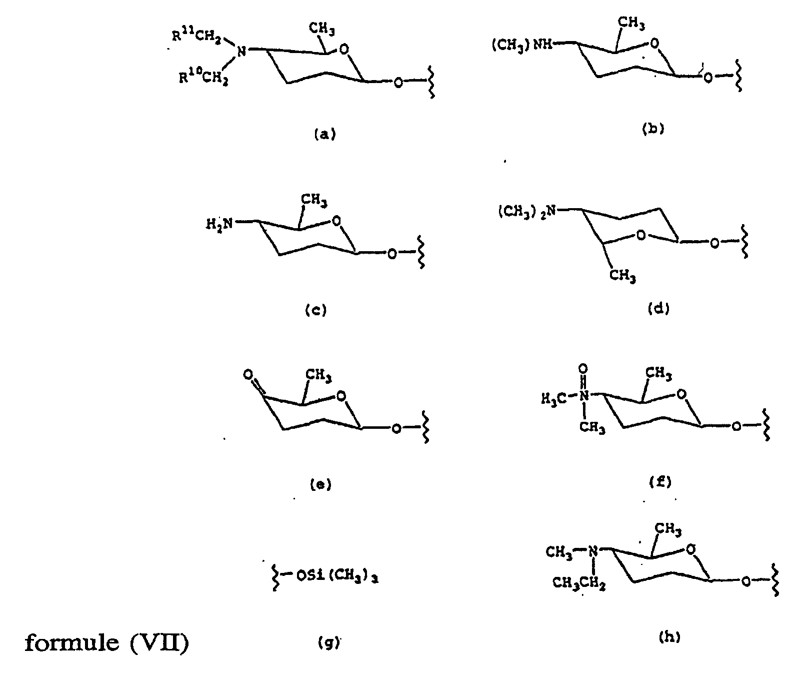 R² a la signification indiquée dans le tableau ci-dessous, représente un atome d'hydrogène ou un groupe 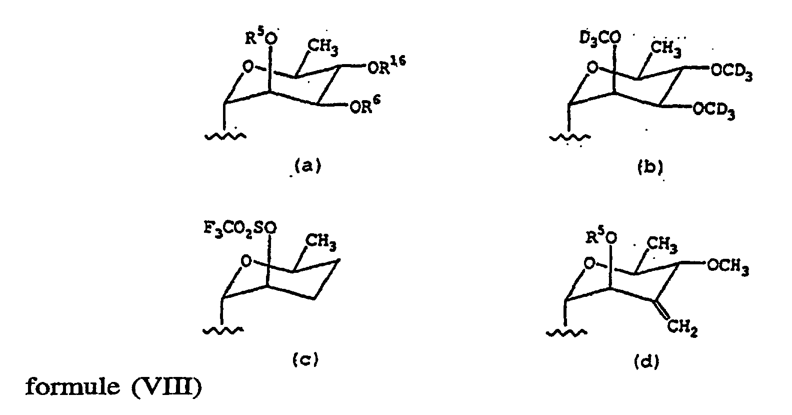 où R⁵, R⁶ et R¹⁶ représentent chacun indépendamment un groupe méthyle, éthyle, propyle ou butyle, R¹ représente un atome d'hydrogène ou un groupe méthyle, R⁴ représente un groupe méthyle ou éthyle, et la signification des autres symboles est celle correspondant aux combinaisons suivantes : 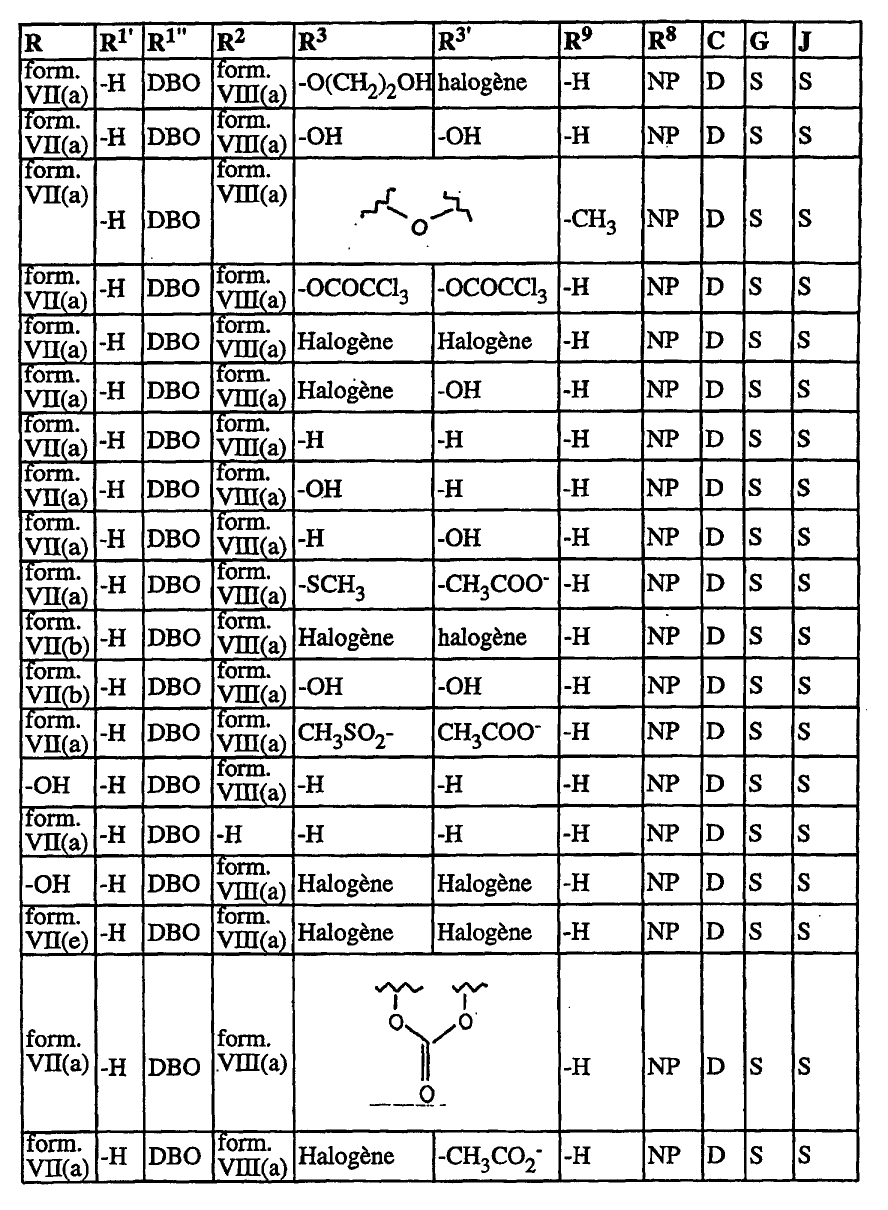 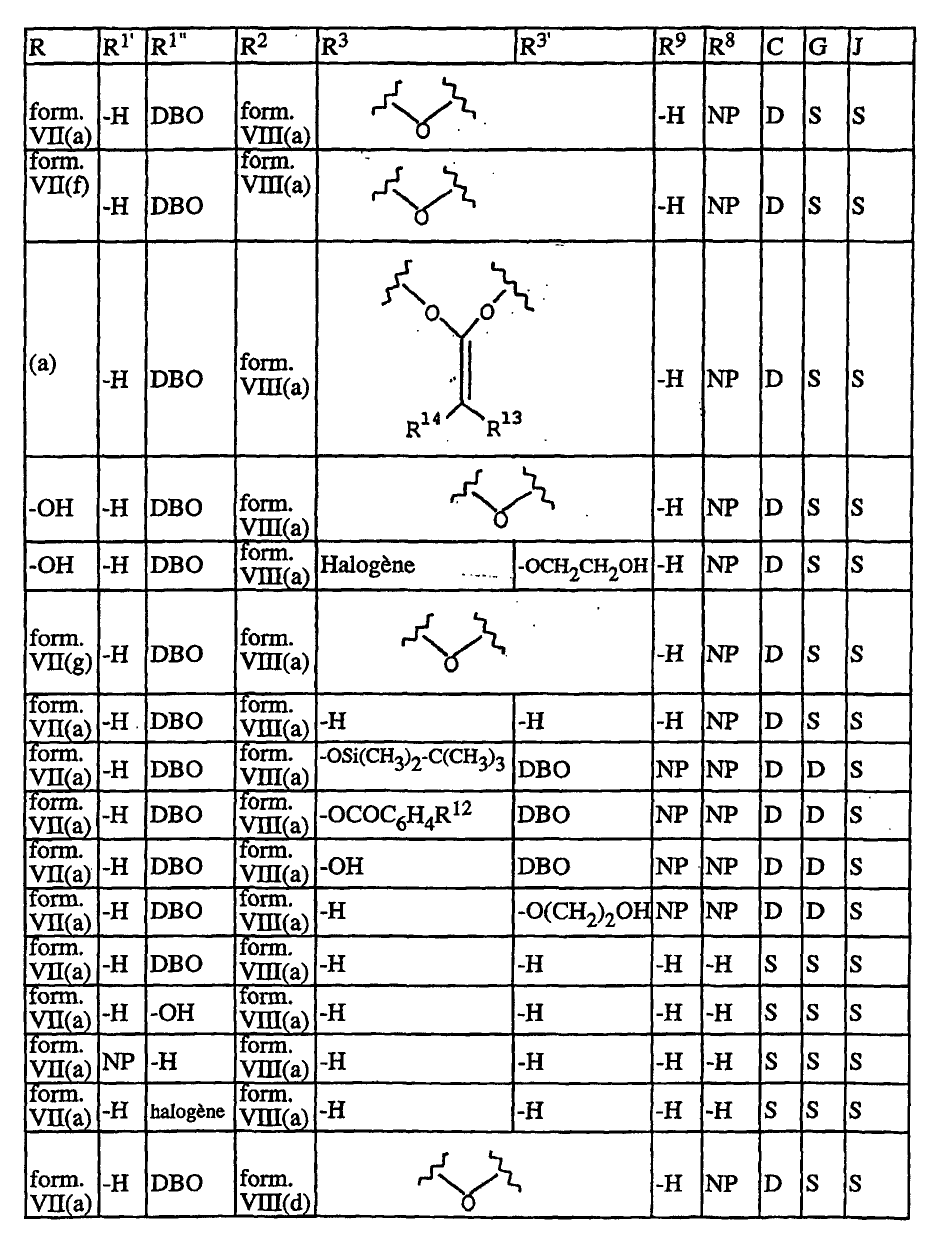 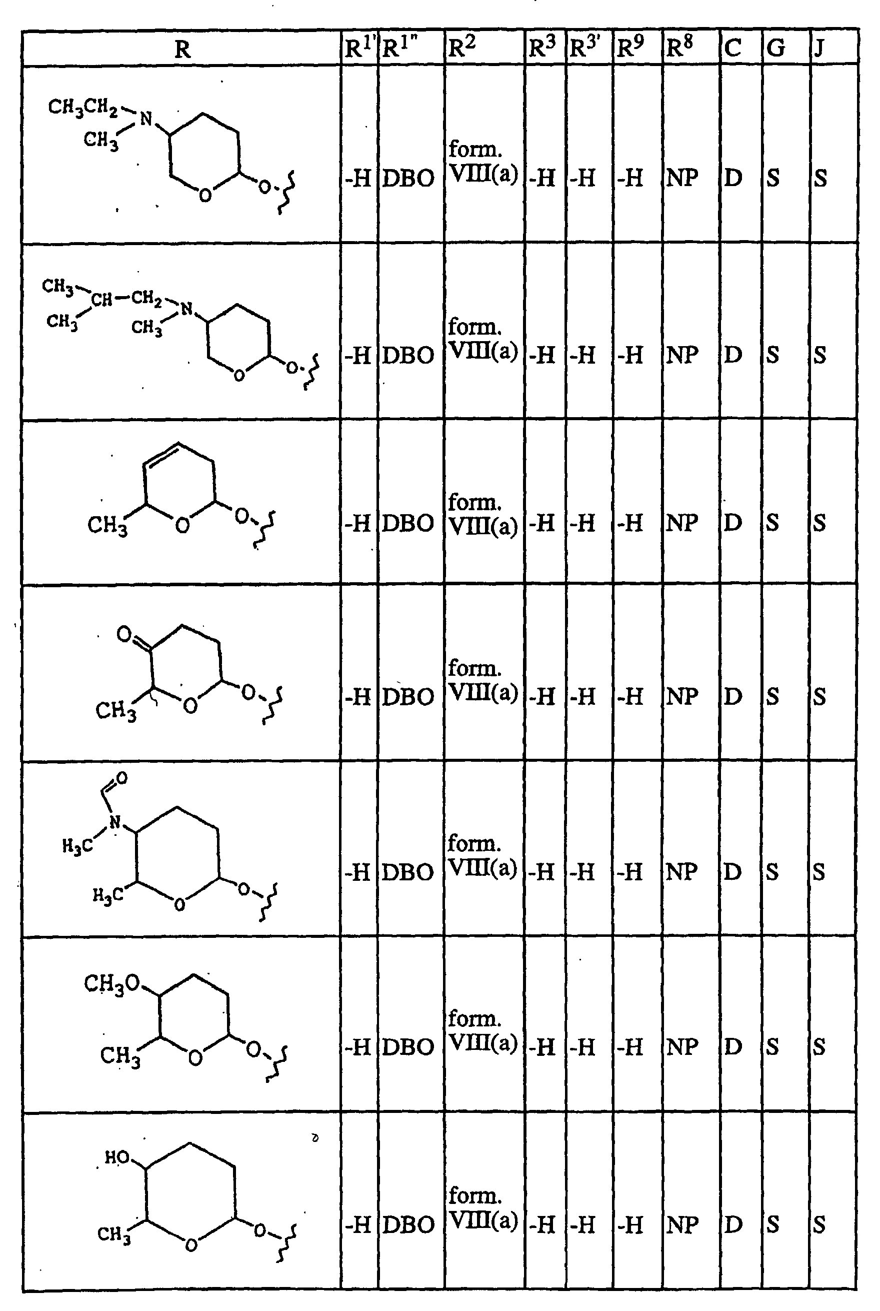 où
DBO représente un oxygène lié par une double liaison
NP signifie "non présent",
R¹⁰, R¹¹ et R¹² représentent indépendamment chacun un atome d'hydrogène, un groupe alkyle en C₁₋₄ ou halogénoalkyle en C₁₋₄, halogéno englobant les atomes F, Cl, I et Br,
A⁻ = I⁻ ou Br⁻,
R¹³, R¹⁴ et R¹⁵ représentent indépendamment un atome F, Cl, I ou Br, et Halogéne signifie F, Cl, I ou Br.

6. Composé selon la revendication 5, dans lequel le composé est un sel d'addition d'acide.

7. Composé selon la revendication 5 de formule 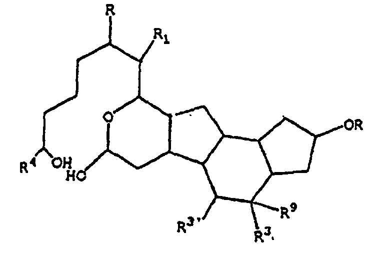 dans laquelle
R représente un groupe de formule VII(a) 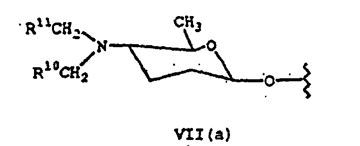 où R¹⁰ et R¹¹ représentent indépendamment chacun un atome d'hydrogène, un groupe alkyle en C₁₋₄ ou halogénoalkyle en C₁₋₄, où halogéno englobe F, Cl, I et Br,
R¹ représente un atome d'hydrogène ou un groupe méthyle,
R² représente un groupe de formule VIII(a) 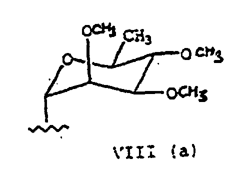 R³ et R^{3'} représentent chacun un atome d'hydrogène ou un groupe méthyle, R⁴ représente un groupe méthyle ou éthyle, et R⁹ représente un atome d'hydrogène ou un groupe CH₃.

8. Composition insecticide, miticide ou ectoparasiticide comprenant, en tant que principe actif, un composé selon l'une quelconque des revendications 1, 3 ou 5.

9. Composé choisi dans le groupe formé par le dérivé 5,6-dihydrogéné de la 9-*O*-(2,3,4-tri-*O*-éthyl-α-L-rhamnosyl)-spinosyne A 9-Psa ;
la (5*S*,6*R*)-époxy-2'-*O*-(p-trifluorométhyl)benzoylspinosyne Q,
la 5,6-dihydro-3'-*O*-éthyl spinosyne J,
la 5,6-dihydro-3'-*O*-n-propylspinosyne J,
la 5,6-dihydro-3'-*O*-n-butylspinosyne J,
l'hémiglutarate de 5,6-dihydro-3'-O-n-propylspinosyne J
la 5,6-dihydro-2'-*O*-éthylspinosyne H,
la 5,6-dihydro-2'-*O*-n-propylspinosyne H,
la (5*R*,6*S*)-5,6-époxy-3'-*O*-n-propylspinosyne L ;
la (5*S*,6*R*)-5,6-époxy-3'-*O*-n-propylspinosyne L,
la (5*R*,6*S*)-3'-désoxy-5,6-époxy-3'-méthylénespinosyne J,
la (5*S*,6*R*)-3'-désoxy-5,6-époxy-3'-méthylènespinosyne J,
la 5,6-dihydro-3'-*O*-n-propylspinosyne J,
la 5,6-dihydro-3'-*O*-iso-propylspinosyne J,
la 5,6-dihydro-3'-épi-N-formyl-3'-propylspinosyne J,
la 5,6-dihydro-3'-épi-3'-propylspinosyne J,
la 5,6-dihydro-3'-*O*-vinylspinosyne J,
la (14*S*)-5,6,13,14-tétrahydro-3'-O-n-propylspinosyne J,
la 5,6-dihydro-3'-méthoxyméthylspinosyne J,
4"-N-déméthyl-4"-N-(2-fluoroéthyl)-5,6-dihydro-3'-O-propylspinosyne J,
la 4"-N-déméthyl-5,6-dihydro-3'-*O*-propylspinosyne J,
la 5,6-dihydro-4"-N-(2-méthyl-1-propyl)spinosyne B,
la 3",4"-déshydro-4"-désamino-5,6-dihydrospinosyne A,
la 5,6-dihydro-N-formylspinosyne B,
la 4"-désamino-5,6-dihydro-4"-cétospinosyne C,
la 4"-désamino-5,6-dihydro-4"-(*S*)-hydroxyspinosyne C,
la 4"-désamino-5,6-dihydro-4"(*S*)-méthoxyspinosyne C,
la (14*S*)-5,6,13,14-tétrahydrospinosyne A,
la (14*R*)-5,6,13,14-tétrahydrospinosyne A,
la (14*S*)-5,6,13,14-tétrahydro-3'-désoxyspinosyne J,
la (5*R*,6*S*)-5-(2-hydroxyéthoxy)-6-bromospinosyne A 17-Psa,
la (5*S*,6*R*)-5,6-époxy-17-*O*-triméthylsilylspinosyne A 17-Psa,
la (5*S*,6*R*)-5,6-époxyspinosyne A 17 Psa,
la (5*R*,6*S*)-5-(2-hydroxyéthoxy)-6-bromospinosyne A,
la (5*S*,6*R*)-5,6-dihydroxy-5,6-dihydrospinosyne A,
le carbonate de (5*S*,6*R*)-spinosyne A,
la (5*S*,6*R*)-5-acétoxy-6-bromospinosyne A,
la (5*R*,6*S*)-5-acétoxy-6-bromo-dihydrospinosyne A,
la 5,6-dibromospinosyne A 17-Psa,
la 5,6-dihydrospinosyne A,
la (5*S*,6*R*)-5,6-époxyspinosyne A,
la (5*R*,6*S*)-époxyspinosyne A,
la (5*S*,6*R*)-époxy-spinosyne D (5*R*,6*S*)-époxyspinosyne D,
la (5*S*,6*R*)-époxyspinosyne A N-oxydée,
la (5*R*,6*S*)-époxyspinosyne A N-oxydée,
la (5*S*,6*R*)-époxyspinosyne D N-oxydée,
la (5*R*,6*S*)-époxyspinosyne D N-oxydée,
la (5*R*,6*S*)-5,6-dibromo-4"-cétospinosyne A,
la (5*R*,6*S*)-5,6-dibromospinosyne B,
la 5,6-cis-dihydroxy-5,6-dihydrospinosyne B,
le dichlorocéténe-acétal de (5*S*,6*R*)-5,6-dihydrospinosyne A,
la (5*S*,6*R*)-5,6-bis(trichloroacétoxy)spinosyne A,
la (5*R*,6*S*)-5,6-dibromospinosyne A,
la (5*S*)-5,6-dihydro-5-hydroxyspinosyne A,
la (6*S*)-5,6-dihydro-6-hydroxyspinosyne A,
la (6*R*)-5,6-dihydro-6-hydroxyspinosyne A,
la (5*R*,6*R*)-5-acétoxy-6-thiométhoxyspinosyne A,
la (5*R*,6*R*)-5-acétoxy-6-méthylsulfonylspinosyne A,
la (5*R*,6*R*)-6-bromo-5-hydroxyspinosyne J,
la 5,6-dihydrospinosyne A 9-Psa,
la 5,6-dihydrospinosyne A 17 Psa,
la 5,6-dihydrospinosyne J,
la 5,6-dihydrospinosyne H,
l'hémiglutarate de la 5,6-dihydrospinosyne A,
la 5,6-dihydrospinosyne B,
la (5*S*,6*R*)-époxyspinosyne Q,
la (5*S*,6*R*)-5,6-dihydro-5,6-dihydroxyspinosyne A,
la (5*R*,6*S*)-5,6-dihydro-5,6-dihydroxyspinosyne A,
la 5,6-dihydrospinosyne D,
la 9-(2H-5-*R*-acétoxy-5,6-dihydro-6-S-méthyl-2-α-pyranyl)-spinosyne A 9-pseudoaglycone,
la 5,6-dihydro-9-(2-désoxy-3-*O*,4-*O*-diéthyl-1-α-rhamnosyl)-spinosyne A 9-pseudoaglycone, et
la 5,6-dihydro-9-(2-désoxy-3-*O*,4-*O*-di-n-propyl-1-α-rhamnosyl)spinosyne A 9-pseudoaglycone.
